# EUROPEAN PATENT APPLICATION

(11) **EP 2 767 298 A2**
(43) Date of publication of application: **20.08.2014**
(21) Application number: 14166779.0
(22) Date of filing: 23.11.2011
(51) Int. Cl.: A61M 5/14

(54) **Therapeutic methods and compositions for solid delivery**

(30) Priority: 23.11.2010 US 416686 P; 24.11.2010 US 417157 P; 18.03.2011 US 201161454115 P; 14.04.2011 US 201161475594 P; 15.04.2011 US 201161475858 P; 06.05.2011 US 201161483437 P
(62) Divisional of application: 11843742.5
(71) Applicant: Presage Biosciences, Inc., Seattle, WA 98109 (US)
(72) Inventor: The designation of the inventor has not yet been filed
(74) Representative: Wilkinson, Marc George

(57) **Abstract**

An administration device comprising an array of needles, one or more fluid agents, and at least one hydrogel is described. The device can simultaneously deliver a plurality of fluid agents along respective axes into a tissue. The use of hydrogel leads to constrained delivery of the fluid agents. The constrained delivery of an agent is also achieved by depositing a drug implant into a tissue. The effect of an agent on the tissue can be evaluated thereafter. In addition, the invention is directed to treating muscle diseases by delivering a therapeutic agent in vivo, and the use of reporter tissues for candidate drug evaluation, detecting and characterizing resistance.

## Description

### RELATED APPLICATION INFORMATION

This application claims the benefit of priority under 35 U.S.C. section 119(e) to US Provisional Application 61/416,686, filedNovember 23, 2010; US Provisional Application 61/417,157, filed November 24, 2010; US Provisional Application 61/454,115, filed March 18, 2011; US Provisional Application 61/483,437, filed May 6, 2011; US Provisional Application 61/475,858, filed April 15, 2011; and US Provisional Application 61/475,594, filed April 14, 2011; the contents of which are incorporated by reference in their entirety.

### TECHNICAL FIELD

In general, the disclosed embodiments relate to devices and methods for the introduction and subsequent evaluation of therapeutic agents to biological tissue, and in particular to the simultaneous introduction of a plurality of agents to the tissue *in vivo.*

### BACKGROUND OF THE INVENTION

Numerous cancer-related agents are under preclinical and clinical trials and evaluations at any particular time; however, most of them will fail to advance. In fact, it is estimated that more than 90% of cancer-related therapeutics will fail phase I or II clinical trial evaluation. The failure rate in phase III trials is almost 50%, and the cost of new drug development from discovery through phase III trials is between $0.8 billion and $1.7 billion and can take between eight and ten years.

In addition, many subjects fail to respond even to standard drugs that have been shown to be efficacious. For reasons that are not currently well understood or easily evaluated, some individual subjects do not respond to standard drug therapy. One significant challenge in the field of oncology is to exclude drug selection for individual subjects having cell autonomous resistance to a candidate drug to reduce the risk of unnecessary side effects without concomitant benefit. A related problem is that excessive systemic concentrations are required for many oncology drug candidates in efforts to achieve a desired concentration at a tumor site, an issue compounded by poor drug penetration in many under-vascularized tumors (Tunggal et al., 1999 Clin. Canc. Res. 5:1583).

Furthermore, hyperactive oncogenic signaling pathways drive cancer initiation, progression, and maintenance. In some cases, cancer cells can become reliant on the hyper-activated signal, such that pharmacologic inhibition of this signal will result in tumor cell death. This reliance prompts cancer cells to rewire signaling, develop mutations that confer drug resistance, and eventually thrive in the presence of targeted inhibitors. As a result, targeted agents show limited clinical efficacy. There is a need in the art for an effective strategy to combat tumor plasticity, targeting whole pathways rather than single oncogenic proteins. Furthermore, validation of the multiple targets that are identified in whole-pathway analysis requires the ability to screen multiple candidate therapeutic agents in parallel. Relatively rapid and inexpensive in vitro screening methods have resulted in high failure rates of candidate drugs in clinical trials, due to the inability of in vitro models to recapitulate signaling in the in vivo context of a tissue or tumor. Therefore, there is a need for in vivo screening models that efficiently assess the efficacy of various candidate agents against various components of signaling pathways activated in cancer.

Duchenne Muscular Dystrophy (DMD), the most frequent and severe form of muscular dystrophy, could also benefit from efficient drug screening and delivery. Duchenne Muscular Dystrophy (DMD) is caused by the lack of dystrophin, a component of a multi-protein complex linking the cytoskeleton of the muscle fiber to the extracellular matrix, the dystrophin-associated glycoprotein complex (DAG). In the absence of dystrophin, the complex is functionally impaired, and this results in degeneration of striated, cardiac and smooth muscle fibers and gradual replacement of muscles by fat and connective tissue. The clinical course of DMD is severe and progressive: affected individuals can be diagnosed at birth based on high serum levels of muscle enzymes, they exhibit muscle weakness by age 5, next lose independent ambulation, and eventually succumb to respiratory failure or cardiomyopathy in their late teens or early twenties. There is a need in the art for treatment of muscle diseases and disorders such as muscular dystrophies and sarcopenia. Furthermore, systemic administrations, e.g. intravascular, of some otherwise promising agents, are inefficient and subject to unwanted systemic side effects. Therefore, therapeutic methods of local delivery are advantageous.

Clearly there is a need in the art for improved devices and methods for testing and delivering candidate agents, and for identifying agents having increased likelihood of benefitting individual subjects. The present invention addresses these and similar needs, and offers other related advantages.

### SUMMARY OF THE INVENTION

It is an aspect of the present invention to provide an administration device comprising: (a) an array of needles positioned for delivery of a plurality of fluid agents along parallel axes within a tissue; and (b) one or more fluid agents and a hydrogel, wherein the agents are formulated to disperse through the tissue to a lesser degree than the same fluid agents lacking the hydrogel upon delivery to the tissue. In certain further embodiments, the administration device comprises an actuator associated with the array of needles, and the fluid agents are dispensed via the actuator. The actuator may comprise a plunger or a pump.

The administration device may comprise 1, 2, 3, 4, 5, 6, 7, 8, 8, 10, 15, 20, 30, 50, 100, or more needles. In certain embodiments, the array of needles comprises about 1 to about 1,000 needles. In certain other embodiments, the array of needles comprises about 2 to about 1,000 needles. In certain other embodiments, the array of needles comprises about 5 to about 1,000 needles.

In some embodiments, the fluid agents may comprise at least one indicator particle. In some further embodiments, the indicator particle is selected from the group consisting of a metallic particle, a fluorescent dye, a quantum dot, a quantum barcode, a radiographic contrast agent, a magnetic resonance imaging contrast agent, a positive control, and a negative control. In some still further embodiments, the indicator particle comprises a dye. The dye may be fluorescent. In some other embodiments, the fluid agents comprise an anti-cancer agent, an anti-inflammatory agent, an anti-infective agent, a regenerative agent, a relaxing agent, an apoptosis-inhibiting agent, an apoptosis-inducing agent, an anti-coagulatory agent, a dermatological agent, a growth-stimulating agent, a vasodilating agent, a vasorestricting agent, an analgesic agent, an anti-allergic agent, or a combination thereof. In some further embodiments, the fluid agents comprise an anti-cancer agent.

According to certain embodiments, the tissue is from an animal. In certain other embodiments, the tissue is from a human. In some embodiments, the tissue is, or is suspected of being, cancerous, inflamed, infected, atrophied, numb, in seizure, or coagulated. In a further embodiment, the tissue is, or is suspected of being, cancerous. In a still further embodiment, the tissue is a tumor.

Certain embodiments contemplate the use of a hydrogel to control the delivery of fluid agents. In some embodiments, said hydrogel comprises collagen. In some embodiments, said hydrogel comprises polyethylene glycol (PEG). The PEG may have a molecular weight of at least 500. In a further embodiment, the PEG has a molecular weight of 4,000-8,000. In another further embodiment, the PEG has a molecular weight of 8,000-45,000. In some embodiments, said hydrogel comprises poly(lactic-co-glycolic acid). In certain another embodiments, said hydrogel comprise polycaprolactone. The hydrogel may comprise a binary mixture of at least one polymer with two different molecular weight ranges. In some embodiments, the hydrogel is PEG. In a further embodiment, the two different molecular ranges comprise 500-2,000 and 4,000-8,000. In another further embodiment, the two different molecular ranges comprise 4,000-8,000 and 10,000-45,000. In yet another further embodiment, the two different molecular ranges comprise 2,000-4,000 and 8,000-20,000. The hydrogel may have a tensile strength of at least 20 gf/cm² in the dry state. In a further embodiment, the hydrogel has a tensile strength of 20-120 gf/cm² in the dry state. In some embodiments, the hydrogel may have a tensile strength of at least 5 gf/cm² in the hydrate state. In a further embodiment, the hydrogel has a tensile strength of 5-15 gf/cm² in the hydrate state.

In some embodiments, the fluid agents and the hydrogel are delivered along parallel axes within the tissue. The diffusion rates of the fluid agents may be controlled by the pore size of the hydrogel. In some embodiments, the pore size is a range between about 50 nm and 500 µm.

In some embodiments, the administration device further comprises one or more reservoirs each in fluid communication with a respective one of said needles. The administration device may have two or more, five or more, or ten or more reservoirs each in fluid communication with a respective one of said needles. Each reservoir may comprise a same or a different fluid agent. In one embodiment, none of said reservoirs comprises the same fluid agent as the agent in any other reservoirs. In another embodiment, at least two of said reservoirs comprise the same fluid agent. In a further embodiment, the concentrations of the same fluid agent in different reservoirs are different. In yet another embodiment, at least one of said reservoirs comprises at least two fluid agents.

The needle may be porous needle. In some embodiments, at least one of said one or more needles comprises a plurality of ports along its length. In a further embodiment, the distribution density of the plurality of ports is inversely related to the distance of the respective port from the tip-end of the needle. In another further embodiment, the size of each of the plurality of ports is inversely related to the distance of the respective port from the tip-end of the needle. On the other hand, the needles may not be permeable to the fluid agents. The needles are neither made of porous material nor have pores along their length. In some embodiments, the administration device may further comprise one or more porous tubes. The porous tube may comprise a fluid agent and a hydrogel. The pore size of the porous tube may control the diffusion rate of the fluid agent.

It is another aspect of the present invention to provide a method of delivering a candidate agent into a tissue of an animal, the method comprising: (a) depositing at least one pharmaceutical composition comprising a hydrogel into the tissue via an administration device comprising a needle; and (b) dispensing the pharmaceutical composition into the tissue, wherein the pharmaceutical composition comprises one or more candidate agents and wherein the pharmaceutical composition disperses through the tissue to a lesser degree than does the same pharmaceutical composition lacking the hydrogel. It is yet another aspect of the present invention to provide a method of delivering a candidate agent into a tissue of an animal, the method comprising: (a) loading an administration device comprising a needle with one or more drug implants comprising one or more candidate agents; and (b) inserting said one or more drug implants into the tissue using said administration device. The administration device may comprise 1, 2, 3, 4, 5, 6, 7, 8, 9, 10, 15, 20, 30, 40, 50, 100, or more needles. In certain further embodiments, the administration device comprises an actuator. The actuator may be a plunger or a pump. The administration device may comprise at least one reservoir. In some embodiments, the administration device comprises 2 or more reservoirs each in communication with a respective one of said needle. In other embodiments, the administration device comprises 5 or more reservoirs each in communication with a respective one of said needle. In yet other embodiments, the administration device comprises 10 or more reservoirs each in communication with a respective one of said needle. The candidate agent in each reservoir may be the same or different. In some embodiments, none of said reservoirs comprises the same candidate agent as the agent in any other reservoirs. In some other embodiments, at least two of said reservoirs comprise the same candidate agent. In a further embodiment, the concentrations of the same fluid agent in different reservoirs are different. In yet some other embodiments, at least one of said reservoirs comprise two or more candidate agents. The number of candidate agents inserted into the tissue may be 1, 2, 3, 4, 5, 6, 7, 8, 9, 10, 15, 20, 50, 100, or more. In some embodiments, the candidate agents are dispensed in an approximately column-shaped region coaxial with respect to the axis of delivery. In some other embodiments, the candidate agents are dispensed along parallel axes within the tissue. In some other embodiments, the candidate agents are delivered at or below systematically detectable concentration. The animal may be a human, a mouse, a rat, or a dog

Certain embodiments contemplate the use of indicator particles. In some embodiments, the candidate agents comprise at least one indicator particle. In some further embodiments, the indicator particle is selected from the group consisting of a metallic particle, a fluorescent dye, a quantum dot, a quantum barcode, a radiographic contrast agent, a magnetic resonance imaging contrast agent, a positive control, and a negative control. In another further embodiment, the indicator particle is a fluorescent dye. In still a further embodiment, the fluorescent dye may be imaged within the tissue, thereby monitoring the disbursement of the candidate agent. The candidate agents in various embodiments may comprise an anti-cancer agent, an anti-inflammatory agent, an anti-infective agent, a regenerative agent, a relaxing agent, an apoptosis-inhibiting agent, an apoptosis-inducing agent, an anti-coagulatory agent, a dermatological agent, a growth-stimulating agent, a vasodilating agent, a vasorestricting agent, an analgesic agent, an anti-allergic agent, a gene modulating agent, an RNAi molecule, or a combination thereof. In a further embodiment, the candidate agents comprise an anti-cancer agent. The tissue may be from a human or a mouse. In some embodiments, the tissue is, or is suspected of being, cancerous. In a further embodiment, the tissue is a tumor. In still a further embodiment, the tumor is selected from a benign tumor and a malignant tumor.

Certain embodiments contemplate the use of a hydrogel to control the delivery of candidate agents. In some embodiments, said hydrogel comprises collagen. In some embodiments, said hydrogel comprises polyethylene glycol (PEG). The PEG may have a molecular weight of at least 500. In a further embodiment, the PEG has a molecular weight of 4,000-8,000. In another further embodiment, the PEG has a molecular weight of 8,000-45,000. In some embodiments, said hydrogel comprises poly(lactic-co-glycolic acid). In some another embodiments, said hydrogel comprise polycaprolactone. In some embodiments, one or more candidate agents and a hydrogel are loaded into a porous tube and then inserted into the tissue. In some embodiments, the needles comprise a porous needle. In other embodiments, the needles are not permeable to the pharmacetucical composition and the candidate agent. The hydrogel may comprise a binary mixture of at least one polymer with two different molecular weight ranges. In some embodiment, the hydrogel is PEG. In a further embodiment, the two different molecular ranges comprise 500-2,000 and 4,000-8,000. In another further embodiment, the two different molecular ranges comprise 2,000-4,000 and 8,000-20,000. In yet another further embodiment, the two different molecular ranges comprise 4,000-8,000 and 10,000-45,000. The hydrogel may have a tensile strength of at least 20 gf/cm² in the dry state. In a further embodiment, the hydrogel has a tensile strength of 20-120 gf/cm² in the dry state. In some embodiments, the hydrogel may have a tensile strength of at least 5 gf/cm² in the hydrate state. In a further embodiment, the hydrogel has a tensile strength of 5-15 gf/cm² in the hydrate state.

In some embodiments, the fluid agents and the hydrogel are delivered along parallel axes within the tissue. The diffusion rates of the fluid agents may be controlled by the pore size of the hydrogel. In some embodiments, the pore size is a range between about 50 nm and 500 µm.

Certain further embodiments contemplate the use of a dye for the detection of activation and deactivation of a biological function. In one embodiment, the pharmaceutical composition comprises a dye. The dye allows for the detection of activation and deactivation of a biological function. The biological function is a pathway, an activity of an enzyme, an expression of a gene, drug sensitivity, drug resistance, transcription of the gene, translation of an RNA molecule associated with the gene, or peptide synthesis. In some embodiments, the biological function is associated with cancer, degenerative disease, inflammation, metabolism, apoptosis, or an immune response. In a further embodiment, the biological function is associated with cancer. In still a further embodiment, the biological function is an apoptotic pathway, and said dye reports apoptosis. The detection may be performed by measuring fluorescence. In some embodiments, the dye can be imaged, thereby allowing detecting the activation or deactivation of the biological function. In a further embodiment, the potential therapeutic value of the therapeutic agents can be predicted based on the results of the imaging. In another embodiment, the activation or deactivation of the biological function can be quantified based on the imaging.

In some embodiments, the drug implants comprises an inert biodegradable binder that permits sustained or timed release of a drug within the tissue. The drug implants comprise at least one candidate agent in solid form. In some embodiments, the candidate agent is released over a period of time between one minute and six weeks. Each of the one or more drug implants may have a diameter of less than about 0.1, 0.2, 0.3, 0.4, 0.5, 0.6, 0.7, 0.8, 0.9, or 1 mm. Each of the needles may be configured to receive 1, 2, 3, 4, 5, 6, 7, 8, 9, 10, 20 or more of said drug implants. Multiple drug implants, such as 2, 3, 4, 5, 6, 7, 8, 9, 10 or more, may be inserted into the tissue.

Certain embodiments contemplate evaluating the effect of said one or more candidate agents on the tissue. Certain embodiments further comprise one of (i) selecting at least one of said agents based on said evaluation; (ii) deselecting at least one of said agents based on said evaluation; and (iii) prioritizing at least two of said agents based on said evaluating. In some embodiments, the effect of one or more fluid agents on the tissue may be evaluated in a method in which the one or more candidate agents have been pre-delivered to the tissue. In some embodiments, the evaluation comprises analysis at least one pre-excised portion of the tissue after introducing said one or more candidate agents. In certain other embodiments, the evaluation comprises analysis of at least one pre-excised portion of the tissue after introducing said one or more candidate agents. In certain further embodiments, the excising or pre-excising may be performed between one day and six weeks after introducing said one or more candidate agents. In some embodiments, the evaluation comprises imaging the tissue. The imaging comprises radiographic imaging, magnetic resonance imaging, positron emission tomogoraphy, and biophotonic imaging. In certain embodiments, the imaging occurs before, during, or after introduction of said one or more candidate agents. In certain embodiments, the evaluation comprises detecting an altered physiological state.

In one aspect, the present invention provides for a method for screening candidate agents using a reporter tissue, comprising the steps of: a) producing a reporter cell line that generates a detectable signal upon modulation of a pathway; b) constructing a reporter tissue from said reporter cell line; and c) assessing the efficacy of a plurality of candidate agents on said reporter tissue by delivering said plurality of candidate agents to said reporter tissue using a needle array, and detecting said detectable signal. In some embodiments, the reporter tissue comprises a xenografted tumor. The modulation can comprise activation, or it can comprise inhibition. In some embodiments, each of the plurality of candidate agents comprises an agent that is selected from the group consisting of a gene therapy agent, a chemotherapy agent, a small molecule, an antibody, a protein, a small interfering RNA, a microRNA, an antisense RNA, a ribozyme, a detectable label, a therapeutic protein, a therapeutic cell, a cytokine, an antibody-drug conjugate, an antibody, a polypeptide, and a peptidomimetic. In some further embodiments, the candidate agents comprise an anti-cancer agent. Each of the plurality of candidate agents may be within a hydrogel. The reporter cell line may comprise a gene trap exhibiting luciferase-based activity. In addition, the reporter cell line may comprise a reporter gene associated with an endogenous promoter. The detectable signal may comprise fluorescence. In some embodiments, the fluorescence is generated by a fluorescent protein which is selected from the group consisting of GFP, BFP, CFP, YFP, EGFP, EYFP, Venus, Citrine, phiYFP, copGFP CGFP, ECFP, Cerulean, CyPet, T-Sapphire, Emerald, YPet, AcGFP1, AmCyan, AsRed2, dsRed, dsRed2, dsRed-Express, EBFP, HcRed, ZsGreen, ZsYellow, J-Red, TurboGFP, Kusabira Orange, Midoriishi Cyan, mOrange, DsRed-monomer, mStrawberry, mRFP1, tdTomato, mCherry, mPlum, and mRaspberry. In a further embodiment, said fluorescent protein is GFP. In addition, the detectable signal may comprise the detectable product from the reaction of an enzyme. In some other embodiments, the enzyme is selected from the group consisting of: peroxidase, alkaline phosphatase, galactosidase, luciferase, and lactamase. In a further embodiment, the enzyme is luciferase. In some embodiments, the assessing comprises imaging said reporter tissue following injection to observe said detectable signal. In some embodiments, the method further comprises the step of excising and optionally sectioning the reporter tissue prior to the imaging. In some other embodiments, the method further comprises the step of pre-excising and optionally sectioning the reporter tissue prior to the imaging.

In another aspect, the present invention provides for a method of determining a response to an agent within a solid tissue, comprising the steps of: a) obtaining a solid tissue derived from a reporter cell line that generates a detectable signal upon modulation of a signaling pathway; b) delivering a plurality of therapeutic agents to said solid tissue; and c) detecting said detectable signal to determine a response to one or more of said therapeutic agents. In some embodiments, the method further comprises the step of producing a reporter cell line from the reporter cell line of step (a) that generates a second detectable signal upon modulation of said signaling pathway. The first detectable signal may indicate activation of the signaling pathway, and the second detectable signal may indicate inhibition of the signaling pathway. In some embodiments, the method further comprises the step of isolating cells that previously generated said second detectable signal in response to said one or more therapeutic agents, but now generates said first detectable signal in response to said one or more therapeutic agents. In some embodiments, the method further comprises the step of isolating cells that previously generated said detectable signal in response to said one or more therapeutic agents, but no longer generate said detectable signal. In some embodiments, the method further comprises the step of sequencing at least one nucleic acid from said isolated cells to determine the genetic basis of the change in response to one or more of said therapeutic agents. The at least one nucleic acid may be a genomic DNA or a messenger RNA. The modulation can comprise activation or inhibition. The obtaining can comprise engineering a solid tissue, which can comprise a tumor or a xenografted tumor. The detectable signal can comprise a molecule selected from the group consisting of a chromophore, a fluorophore, a fluorescent protein, a phosphorescent dye, a tandem dye, a particle, a hapten, a radioisotope, and the chemiluminescent substrate of an enzyme, including luciferase.

In yet another aspect, the present invention provides for a method for determining the genetic basis of an in vivo response to a drug, comprising the steps of: a) delivering a drug to a reporter tissue derived from a reporter cell line that generates a detectable signal upon modulation of a pathway; b)isolating a population of cells within said reporter tissue that exhibit said detectable signal in response to said drug; and c) sequencing at least one nucleic acid of said population of cells to determine the genetic basis of the drug response. The sequencing can comprise high-throughput sequencing. The method can further comprise the step of whole-genome amplification, or the step of subtractive hybridization of genomic DNA from a population of cells that did not exhibit the detectable signal in response to the drug.

In yet another aspect, the present invention provides for a method for determining resistance to a cancer treatment, comprising the steps: a) administering a plurality of cancer treatments to a reporter tissue that generates a detectable signal upon modulation of a pathway; b) isolating a population of cells within said reporter tissue that exhibit said detectable signal in response to one or more of said plurality of cancer treatments; and c) sequencing at least one nucleic acid from the population of cells, thereby determining resistance to the selected cancer treatment. The administering can comprise injecting with a needle array. The modulation can comprise activation or inhibition. The isolating can comprise cell sorting. The sequencing can comprise high-throughput sequencing.

In a further aspect, the present invention provides for a method of producing a reporter cell line, comprising the steps of: a) obtaining a cell line comprising an active signaling pathway; b) randomly integrating a reporter to a plurality of locations within the genomes of said cell line using a gene trap vector; c) isolating a population of cells that express the reporter in response to an inhibitor of said signaling pathway; and d) producing a reporter cell line from the selected cells. The method can further comprise the step after step c of performing a second round of selection to isolate cells that additionally express the reporter in response to a second inhibitor of said signaling pathway. The method can further comprise the step after step b of removing cells that constitutively express the reporter. In some embodiments, the cell line is a cancer cell line. In some embodiments, the signaling pathway is selected from the group consisting of the Akt pathway, PI3K pathway, MEK pathway, mTOR pathway, EGF pathway, or Ras pathway. In some embodiments, the inhibitor is selected from the group consisting of Gefitinib, MK-2206, and PD325901. The reporter may be a luciferase-based reporter.

In another aspect, the present invention provides for a method for determining resistance to a cancer treatment, comprising the steps of: a) administering a plurality of cancer treatments to a reporter tissue that generates a detectable signal upon modulation of a pathway; b) isolating a population of cells within said reporter tissue that exhibit said detectable signal in response to one or more of said plurality of cancer treatments; and c) assessing the number of copies of genes, thereby determining resistance to the selected cancer treatment.

In another aspect, the present disclosure involves treating muscle diseases. In one embodiment, there is provided a method of local delivery of a therapy for the treatment of a muscle disease or disorder of a subject, comprising administrating of a therapeutic agent in vivo simultaneously to a tissue of said subject using a needle array device comprising a plurality of needles. The therapy may comprise a gene replacement therapy, a microRNA therapy, a RNAi therapy, an antibody therapy, and an aptamer therapy.The muscle disease or disorder may be selected from the group consisting of: Duchenne muscular dystrophy (DMD), Becker muscular dystrophy, Limb Girdle muscular dystrophy, facioscapulhumeral muscular dystrophy, congenital muscular dystrophy, oculopharyngeal muscular dystrophy, distal muscular dystrophy, Emery-Dreifuss muscular dystrophy, muscle atrophy, X-linked spinal-bulbar muscular atrophy (SBMA), cachexia, malnutrition, tuberculosis, leprosy, diabetes, renal disease, chronic obstructive pulmonary disease (COPD), cancer, end stage renal failure, burns, diabetes, congestive heart failure, sarcopenia, emphysema, osteomalacia, or cardiomyopathy. The needles may comprise a porous needle or a needle impermeable to the agent. In some embodiments, the needles are configured for delivery by intramuscular injection. In some embodiments, the gene or gene replacement therapeutic agent is delivered into greater than 1%, 2%, 5%, 10%, 20%, 30%, 40%, 50%, 60%, 70%, 80%, or 90% of large muscle fibers of a single limb. The tissue may be brain, liver, lung, kidney, prostate, ovary, spleen, lymph, thyroid, pancreas, heart, muscle, intestine, larynx, esophagus or stomach tissue. In a further embodiment, the muscle is skeletal muscle, smooth muscle or cardiac muscle. In some embodiments, the gene replacement therapeutic agent comprises an adenovirus, a herpes simplex virus (HSV), a baculovirus, an adeno-associated virus (AAV), a recombinant adeno-associated virus (rAAV), a retrovirus, or a lentivirus.

In another aspect, there is provided a method of delivering a selected gene to a muscle tissue of a subject in vivo, said method comprising: (a) providing a virus which comprises a viral vector, said viral vector comprising said selected gene operably linked to control elements capable of directing the in vivo transcription and translation of said selected gene; and (b) introducing said virus into said muscle tissue in vivo using a needle array device comprising a plurality of needles. In some embodiment, the subject may be a nonhuman primate, mouse, dog, rat, rabbit, pig, sheep, horse, bovine, goat, gerbil, hamster, guinea pig or human. In certain embodiments, the gene may comprise a dystrophin. In certain further embodiments, the gene may comprise a dystrophin minigene encoding a protein or the complement of the dystrophin minigene, wherein the protein comprises: (a) a N-terminal domain of a dystrophin protein or modified N-terminal domain of the dystrophin protein; (b) five rod repeats of the dystrophin protein; (c) an HI domain of a dystrophin gene and an H4 domain of the dystrophin protein; and (d) a cysteine-rich domain of the dystrophin protein, wherein said nucleotide sequence has fewer than 5,000 nucleotides. In some embodiments, encodes a myostatin inhibitor. In some further embodiments, the myostatin inhibitor is myostatin propeptide, follistatin, other follistatin-like proteins, FLRG or GASP-1. In some embodiments, the gene is operably linked to one or more control elements. In some further embodiments, each of said one or more control elements is selected from the group consisting of: AAV inverted terminal repeat, retrovirus long terminal repeat, cytomegalovirus (CMV) immediate early promoter, CMV enhancer, β-actin promoter, α-actin promoter, myosin promoter, muscle-specific creatine kinase (MCK) promoter, and MCK enhancer. In some embodiments, wherein the AAV is selected from the group consisting of: AAV-1, AAV-2, AAV-3, AAV-4, AAV-5, AAV-6, AAV-7, AAV-8, AAV-9, AAV-10 and AAV-11. In a further embodiment, the AAV is AAV-6. In some embodiments, the gene replacement therapeutic agent comprises a stem cell. In a further embodiment, the stem cell is an embryonic stem cell, adult stem cell, or induced pluripotent stem cell. In some embodiments, the needles comprise at least one porous needle. In some embodiments, the needles may comprise at least two porous needles.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 illustrates a method of administering a candidate agent to a tumor in an animal
Figure 2 is a schematic diagram of a needle array assembly for injecting biological tissue with therapeutic agents according to various embodiments.
Figure 3 is a diagrammatic view of a delivery assembly according to an embodiment.
Figure 4 shows a diagram of a needle array, according to an embodiment.
Figure 5 shows elements of a delivery assembly according to another embodiment.
Figure 6 shows diagrammatically a portion of a tumor illustrating principles of the invention.
Figure 7 is a diagram of a data processing system according to an embodiment.
Figure 8 illustrates a slice of lymphoma tumor that was administered doxorubicin via a porous needle.
Figure 9 illustrates microscopy of spatially-restricted cell-kill at multiple tumor depths.
Figure 10 illustrates fluorescent microscopy of spatially-restricted injections of four different amounts of a fluorescent dye into a tumor.
Figure 11 illustrates *ex vivo* response to hedgehog pathway antagonism in a human medulloblastoma sample.
Figure 12 illustrates tumor kill following spatially-restricted injection
Figure 13 illustrates survival of mice injected with vehicle or Shh antagonist.
Figure 14 illustrates fluorescent imaging of a mouse tumor injected with doxorubicin and control.
Figure 15 illustrates spatially-restricted lentivirus expression in a tumor.
Figure 16 illustrates KIF11 shRNA injection in a tumor.
Figure 17 illustrates cell death in response to shRNA injection in a tumor.
Figure 18 illustrates a scheme of a method of using reporter cell lines to evaluate efficacy of candidate therapeutic agents.
Figure 19 illustrates a scheme of a method of using reporter cell lines to determine the genetic basis of resistance to a therapy.
Figure 20 illustrates a selection strategy for generating a reporter cell line.
Figure 21 depicts a porous needle array of the disclosure.
Figure 22 shows localized delivery of dye, drug, and lentivirus.

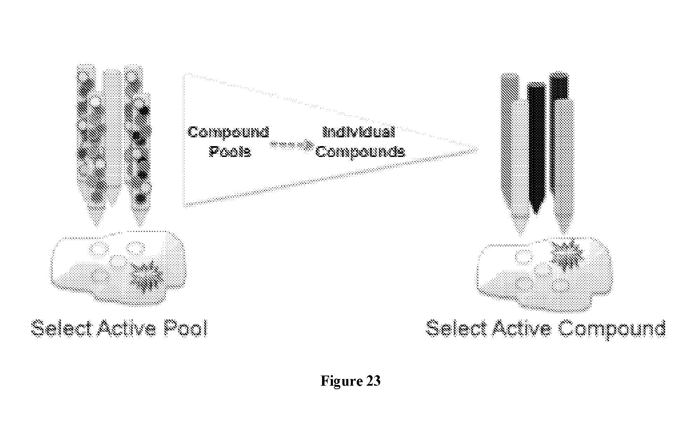

Figure 23 shows a pooling strategy for screening compounds using a porous needle array.
Figure 24 shows porous needle mediated injection of GSK3 inhibitors induces b-catenin driven luciferase activity in tumors.

### DETAILED DESCRIPTION OF THE INVENTION

### General Overview

The present invention provides devices and methods for the spatially constrained delivery of fluid agents to solid tissues. The disclosure features the use of hydrogels in conjunction with a needle array for delivery of a fluid agent by diffusion through pores of the hydrogel, allowing for control of delivery by varying such parameters as pore size. The control of delivery parameters such as localization and rate of delivery provides many advantages in therapeutic and diagnostic applications.

In some preferred embodiments, compositions comprising a hydrogel and one or more fluid agents are inserted along parallel axes within a solid tissue through the use of an array of precisely positioned delivery needles, coupled to an actuator module such as a plunger or a pump, allowing for controlled insertion of multiple compositions each containing a distinct fluid agent along parallel axes within a tissue. In some cases, passive delivery by diffusion through pores in the hydrogel matrix occurs following insertion.

In some embodiments, the administration device further comprises one or more reservoirs each in fluid communication with a respective one of said needles. The administration device may have two or more, five or more, or ten or more reservoirs each in fluid communication with a respective one of said needles. Each reservoir may comprise a same or a different fluid agent. In one embodiment, none of said reservoirs comprises the same fluid agent as the agent in any other reservoirs. In another embodiment, at least two of said reservoirs comprise the same fluid agent. In a further embodiment, the concentrations of the same fluid agent in different reservoirs are different. In yet another embodiment, at least one of said reservoirs comprises at least two fluid agents.

In some preferred embodiments, one or more hydrogel compositions are inserted in a target tissue of an organism through the use of a delivery device, such as a needle. In some cases, more than 1, 2, 3, 4, 5, 6, 10, 20, 50, 100, or 1,000 needles are arranged in an array, such that the needles deliver a plurality of compositions to a plurality of parallel regions within the tissue. Upon insertion, two or more hydrogels may occupy parallel columns in the tissue, with these columns determined by the size, shape, and configuration of the needles. In some preferred cases, solid delivery from these tubes is constrained to a column within the tissue by pore size and diffusion rate. In some preferred embodiments, delivery occurs through pores of a porous needle. One or more porous needles may be configured in an array for parallel delivery. In some other preferred embodiments, the fluid agent with or without hydrogel are loaded in a porous tube and then inserted into the tissue. The delivery of the fluid agent is controlled by the pore size of the porous tube.

Spatial restriction allows for parallel screening of multiple candidate therapeutic agents in a tissue, and controls for tissue heterogeneity by introducing each of the candidate therapeutic agents across an axis in the tissue. In certain embodiments the selected region of tissue is a portion of a solid tissue in a subject, and in certain further embodiments the subject is one of a preclinical model or a human subject. In certain other embodiments, the method comprises excising at least the portion of the tissue after the introducing. In certain other embodiments, the method comprises pre-excising at least the portion of the tissue after the introducing. Certain further embodiments comprise at least one of imaging the tissue prior to the excising, imaging the tissue concurrently with the excising, and imaging the tissue after the excising. In certain other embodiments the excising comprises excising at least the portion of the tissue at a time that is a selected period of time after introducing one or more fluid agents. The selected period of time may be a range of between about 2 and 96 hours. In certain embodiments the selected period of time is a period exceeding one week. Following excision, spatially constrained delivery of multiple candidate agents may allow for ex vivo analysis of the relative efficacies of the agents.

Some embodiments as disclosed herein relate to a method for constrained delivery of a fluid-phase agent to a solid tissue. Such selective delivery obviates the need for excessive systemic concentrations of therapeutic or candidate agents in order to achieve therapeutically effective concentrations in the desired solid tissue, thereby avoiding clinically detrimental toxicities to uninvolved tissues and also avoiding undesirable side-effects. The fluid agent can be delivered at or below systemically detectable concentration to achieve a desirable effect.

In some embodiments, the present method is directed to testing and delivering cancer agents, where multiple candidate therapeutic agents are delivered along parallel axes of a tumor. Such methods permit efficient pre-clinical and clinical studies of candidate oncology medicines, and facilitate identification of therapeutics having a high likelihood of benefitting individual subjects. The disclosure provides for methods useful in evaluating treatment for cancer and permits early exclusion from a screening program or a therapeutic regimen of candidate drugs to which disease cells can be resistant.

In some embodiments, the effect of one or more candidate agents on the tumor may be evaluated in a method in which the one or more fluid agents have been pre-delivered to the tumor. In some embodiments, the evaluation comprises analysis of at least one pre-excised portion of the tissue after introducing said one or more candidate agents. In certain other embodiments, the evaluation comprises pre-excising at least one portion of the tumor after introducing said one or more candidate agents. In certain further embodiments, the excising or pre-excising may be performed between one day and six weeks after introducing said one or more candidate agents. In some embodiments, the evaluation comprises imaging the tumor. The imaging comprises radiographic imaging, magnetic resonance imaging, positron emission tomogoraphy, and biophotonic imaging. In certain embodiments, the imaging occurs before, during, or after introduction of said one or more candidate agents. In certain embodiments, the evaluation comprises detecting an altered physiological state. Furthermore, the present disclosure provides for the screening of candidate agents in vivo, allowing advantages over in vitro methods that do not accurately replicate the microenvironment of a solid tissue within a living organism.

The present disclosure relates to methods of performing multiplexed chemotherapy drug efficacy studies in live tumors for drugs.

Methods of the disclosure have advantages over screening in vitro cell culture models, because in vitro models do not respond to drugs in the same way as tumors grown in intact animal models of cancer or in patients. Therefore decisions to pursue development of drugs for entry into the clinic, or for therapeutic intervention of cancer in a patient, based on in vitro models are flawed. In contrast, the present disclosure provides methods for testing multiple potential anti-cancer agents simultaneously in the context of a live human tumor or in the context of an animal model of cancer.

In some embodiments, individually prepared drug implants comprising anti-cancer agents or test agents are distributed within a solid tissue using an arrayed head of introducer needles that enable parallel insertion of the drug sticks into the tumor. An injection device can allow drug implants to be inserted through the introducer needles into the tumor. A drug implant can comprise a solid rod or stick, and may preferably comprise a drug agent in solid form. In some cases, the drug implant comprises an inert biodegradable binder that permits sustained or timed release of the drug within the tissue.

In some embodiments, a drug implant has a diameter of less than about 0.1, 0.2, 0.3, 0.4, 0.5, 0.6, 0.7, 0.8, 0.9, or 1 mm. A needle array head can be configured to accommodate about 1, 2, 3, 4, 5, 6, 7, 8, 9, 10, 20, 30, 40, 50, 60, 70, 80, 100, 200, 300, 400, or more than 1,000 drug implants. A plurality of drug implants can be bundled to form a bundle of drug implants, wherein the bundle has a diameter suitable for injection using a needle.

In some embodiments, an injection device comprises a plunger configured to push one or more drug implants through needles into a solid tissue. In some embodiments, a solid tissue comprises a tumor.

Following insertion into a tumor, a drug agent can be released from one or more drug implants over a period of one minute to six weeks to allow discrete portions of the tumor to be exposed to each drug individually in a spatially confined manner.

Since drugs are inserted using methods of the present disclosure along parallel axes down the depth or z-axis of the tumor, sections of the tumor can be made cross-wise (perpendicular, or orthogonal to) the insertion path of the drug sticks to enable analysis of tumor response in the area proximal to each inserted drug, thereby allowing multiplexed analysis of drug efficacy in the context of a living tumor.

In some embodiments of the present disclosure, one or more drug implants are inserted into a tissue through use of a carrier device such as a needle. In some preferred embodiments, a plurality of drug implants is inserted along parallel axes of a tissue through the use of a needle array, such as that described in PCT/US2008/073212, which is hereby incorporated by reference in its entirety.

In some cases, a plurality of needles is attached to a plurality of actuators coupled to a plurality of drug implants or bundles of drug implants within a plurality of reservoirs, such that depressing the plunger causes ejection of the drug implants, or injection of the drug implants into a tissue. In certain further embodiments the plungers of the plurality of plungers are operatively coupled together at respective second ends so as to be simultaneously depressable.

An implant can be partially- or fully-submerged in the tissue. Deposition of implants into the tissue can be facilitated by inserting the implant into the tissue via a needle. The implant can be further deposited into the tissue by depression of a plunger associated with the needle.

Once an implant has been deposited into the tissue, the implant can dissolve and diffuse into the tissue. The rate of diffusion can be influenced by the volume of the implant, or it can be influenced by the presence of an inert binder that serves to reduce the rate of dissolution of the implant within the tissue. The drug agent can diffuse into the tissue over a period of about a minute to about a month; about an hour to about six weeks; about 12 hours to about 72 hours; about 24 hours to about 48 hours; about one week to two weeks; about two weeks to three weeks; or about three weeks to four weeks.

The present disclosure provides for methods of generating in vivo tissue models comprising reporter cells that emit a detectable signal in response to specific modulation of a signaling pathway. Several methods, for example, gene trap technology or using reporter genes that are associated with an endogenous promoter, could be used to generate reporter cells for constructing tissue models, Both strategies are advantageous in that they report on native cell pathways, due to the integration of reporter genes into endogenous genomic sites. The use of reporter genes associated with an endogenous promoter could be generated by introduction of a vector that expresses a reporter sequence of interest (luciferase, GFP etc.) under control of a known promoter or transcription factor response element. For instance, the forkhead response element (also known as the insulin response element) has been used to track the activation state of the AKT pathway. Importantly, inhibition of AKT activates transcription of genes under control of this promoter. Reporter cells could be generated in cell types harboring inactivation mutations of phosphatase and tensin homolog gene (PTEN) thus resulting in high AKT pathway status, and low background luciferase signal. Although this approach would require identification and construction of distinct pathway specific vectors for each cancer pathway of interest, the use of ectopically expressed promoters represents a low risk, viable alternative to the aforementioned gene trap approach.

In some embodiments of the method, a reporter is integrated into a gene in a cell line within an oncogenic pathway, such that the reporter is activated upon inhibition of the oncogenic pathway. A cell comprising a reporter integrated into a genomic site is termed a reporter cell line. A reporter tissue refers to a solid tissue comprising reporter cells. A reporter tissue can be generated from a reporter cell line, for example by introducing cells from the reporter cell line into an animal, such as a mouse, and allowing the cells to develop into a solid tumor. Cells of a reporter tissue (e.g. a tumor) which is derived from a reporter cell line can produce a detectable signal upon oncogenic pathway inhibition, providing a useful in vivo or ex vivo platform for screening candidate methods of treating cancer.

In some embodiments, a reporter tissue is employed in conjunction with a needle array for multiplexed analysis of candidate therapeutic agents. A needle array can be used, for example, to deliver a plurality of therapeutic agents, or it can be used to administer a plurality of cancer treatments. Devices and methods for delivery using a needle array are described in PCT/US2008/073212, which is hereby incorporated by reference in its entirety. A needle array can contain more than 2, 3, 4, 5, 6, 7, 8, 9, 10, 20, 30, more than 100, or more than 1,000 needles. These needles can be configured to deliver a common agent, or one or more needles can be configured to deliver different agents. The needles can be coupled to a common actuator, such as a plunger or pump, thereby allowing simultaneous injection from all the needles upon activation of the actuator. In some embodiments, the needles are configured to deliver their respective contents along parallel axes within a tissue. This delivery can occur in a spatially constrained fashion, wherein delivery of a plurality of agents by a plurality of needles is restricted for each needle to a column within a tissue. In some embodiments, spatially constrained delivery is achieved through the use of one or more porous tubes, wherein a candidate agent is delivered to a tissue by diffusion through pores of the tubes. In some embodiments, the porous tube comprises a porous polymer such as polysulfone. In some embodiments, a candidate agent comprises a polymer matrix such as a hydrogel, wherein the rate and extent of diffusion of the candidate agent are reduced by the presence of the polymer matrix. Cells within the reporter tissue of the disclosure can respond to the delivery of a candidate agent by emitting a detectable signal to indicate inhibition of an oncogenic pathway by a given agent. Spatially constrained delivery of a plurality of agents can allow for discernment of the effect of different agents. Furthermore, the delivery of an agent along a column within a tissue can control for heterogeneous cell behavior in different regions of the tissue.

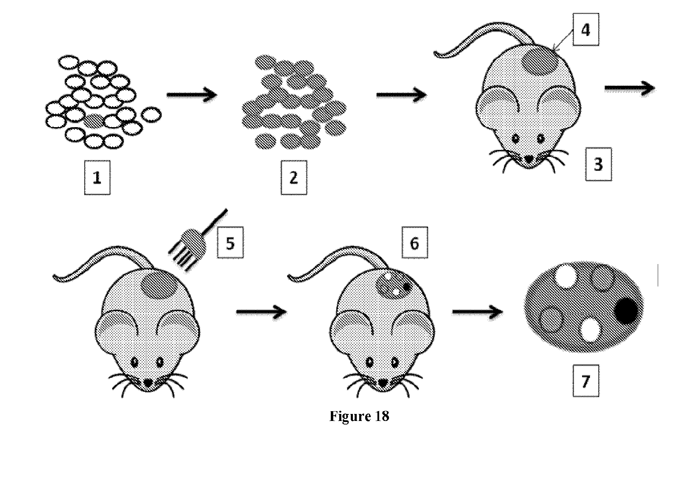

Figure 18 provides a schematic illustration of a method of the disclosure, in which a reporter cell line 2 is produced by selection from a pool of cells 1. Cells can be selected based on activation of a reporter in response to a known pathway modulator (e.g., a drug that inhibits an oncogenic signaling pathway). The reporter cell line 2 can be used to generate a xenografted mouse 3, with a tumor 4 derived from the reporter cell line. The xenografted tumor 4 can serve as a platform for the screening of candidate therapeutic agents useful in treating cancer. In some embodiments, a needle array 5 is used to deliver a plurality of candidate therapeutic agents along parallel axes within the xenograft tumor. Delivery using a needle array can be spatially restricted such that the regions of delivery 6 are distinct from one another. The tumor can be excised and the excised tumor 7 is optionally sectioned and imaged to evaluate the relative efficacies of the various candidate therapeutic agents.

In some embodiments, a reporter tissue is used in determining the basis of resistance to a method of treatment. A reporter can be introduced to a gene under control of a known oncogenic pathway-specific response element, thereby generating a cell line that produces a detectable signal indicating active pathway status. A gene trap technique can then be employed to select for cells that produce a detectable signal upon pathway inhibition. A reporter cell line can produces distinct signals (e.g. emissions at different wavelengths) for pathway activation versus inhibition. Cells from such a line can be xenografted to an animal model such as a mouse to produce a reporter reporter tissue that differentially reports activated and inhibited status for an oncogenic pathway. In some embodiments, a reporter tissue that differentially reports activated ("on") and inhibited ("off") status for an oncogenic pathway is employed to screen candidate therapeutic agents, and can also be used to indicate development of resistance to a previously successful therapeutic agent, wherein the development of resistance is indicated by a switch in signal from pathway "off" status to pathway "on" status during continuous administration of the therapeutic agent. Cells that develop resistance to treatment can be isolated and further characterized to determine the genetic basis of the resistance.

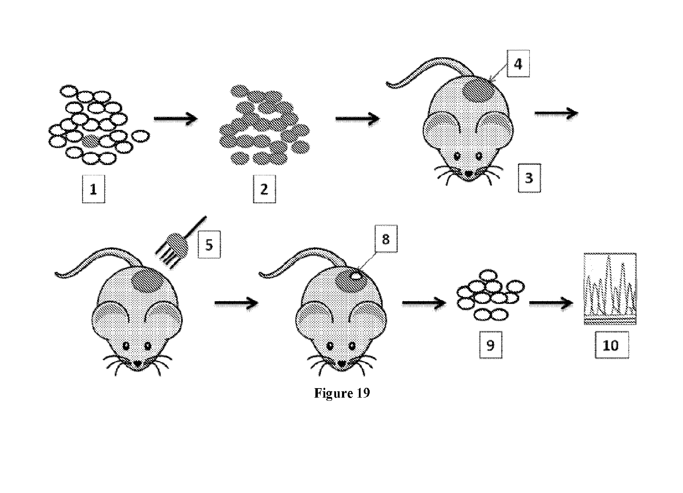

Figure 19 illustrates a scheme for a method of the disclosure, wherein a reporter cell line can be used to determine the basis of resistance to a method of cancer treatment. In some embodiments, a reporter cell line 2 is generated from a pool of cells 1, and this cell line can be used to produce a mouse 3 with a xenografted tumor 4 derived from the reporter cell line 2. In some embodiments, a reporter cell line generates a reporter signal upon inhibition of an oncogenic pathway. A reporter cell line can generate two distinguishable signals (e.g. fluorescence at two distinguishable wavelengths), to indicate activated versus inhibited status of a pathway. In some embodiments, a reporter cell line generates a reporter signal upon activation of an oncogenic pathway. A needle array 5 can be used in conjunction with reporter tissue 4 to determine compounds that lead to pathway activation or inhibition. A needle array is useful for delivering a plurality of agents in parallel regions of a tissue, and allows for control for heterogeneity of a tissue by delivering along an axis within the tissue. Once the cells of a reporter tissue derived from a reporter cell line are known to exhibit a response to an agent, the reporter tissue or other reporter tissues similarly derived from the reporter cell line can be used to identify one or more cells 8 that cease to respond to the agent. Such cells can be isolated and clonally amplified 9, and the genetic basis of the resistance to the agent can be determined by obtaining a genomic sequence profile 10 of those cells.

In some embodiments, the genetic basis of a response to a therapeutic agent is determined by sequencing genomic DNA from cells that exhibit a detectable signal upon administration of the therapeutic agent. Sequencing genomic DNA can comprise high-throughput sequencing. Methods of high-throughput sequencing are known in the art and are described in further detail herein. In some embodiments, whole-genome amplification is performed on the genomic DNA prior to sequencing.

In some embodiments, a reporter cell is used to generate a reporter tissue useful in determining the response of a tissue to various fluid agents. A reporter tissue comprises a plurality of reporter cells. A plurality of fluid agents can be delivered to a reporter tissue using a needle array. The reporter tissue can exhibit features of a disease or disorder, or it can be normal. The response can take the form of activation of a signaling pathway, inhibition of a signaling pathway, or another altered physiologic state. In some embodiments, the method is useful for determining side effects of a drug. An altered physiologic state can be any detectable parameter that directly relates to a condition, process, pathway, dynamic structure, state or other activity in a solid tissue (and in some embodiments, in a solid tumor) including in a region or a biological sample that permits detection of an altered (e.g., measurably changed in a statistically significant manner relative to an appropriate control) structure or function in a biological sample from a subject or biological source. The methods of the present invention thus pertain in part to such correlation where an indicator of altered physiologic state can be, for example, a cellular or biochemical activity, including as further non-limiting examples, cell viability, cell proliferation, apoptosis, cellular resistance to anti-growth signals, cell motility, cellular expression or elaboration of connective tissue-degrading enzymes, cellular recruitment of angiogenesis, or other criteria as provided herein.

Altered physiologic state can further refer to any condition or function where any structure or activity that is directly or indirectly related to a solid tissue function has been changed in a statistically significant manner relative to a control or standard, and can have its origin in direct or indirect interactions between a solid tissue constituent and an introduced agent, or in structural or functional changes that occur as the result of interactions between intermediates that can be formed as the result of such interactions, including metabolites, catabolites, substrates, precursors, cofactors and the like. Additionally, altered physiologic states include, but are not limited to, altered signal transduction, respiratory, metabolic, genetic, biosynthetic or other biochemical or biophysical activity in some or all cells or tissues of a subject or biological source, in some embodiments in some or all cells of a solid tissue, and in some embodiments in some or all cells of a tumor such as a solid tumor in a solid tissue. As non-limiting examples, altered biological signal transduction, cell viability, cell proliferation, apoptosis, cellular resistance to anti-growth signals, cell motility, cellular expression or elaboration of connective tissue-degrading enzymes, cellular recruitment of angiogenesis, or other criteria including induction of apoptotic pathways and formation of atypical chemical and biochemical crosslinked species within a cell, whether by enzymatic or non-enzymatic mechanisms, can all be regarded as indicative of altered physiologic state.

The present disclosure involves methods of local delivery using a needle array device for treating diseases. Exemplary devices are described in PCT/US2008/073212, which is hereby incorporated by reference in its entirety.

Duchenne Muscular Dystrophy (DMD) in humans is a fatal, X-linked, recessive muscle disease caused by lack of dystrophin due to mutations in the dystrophin gene. DMD affects both skeletal and cardiac muscles characterized by progressive muscle wasting leading to rapid decline in the ability to move, and eventually to complete paralysis and death.

The present disclosure involves gene replacement therapeutic agents, a term that refers to genes or gene products that promote tissue function, e.g., muscle function. A gene replacement therapeutic agent can promote tissue function by restoring function in a subject with a genetic disorder that results in loss of tissue function, or it can enhance tissue function in a healthy subject. In some embodiments, an effector of tissue function enhances tissue function that has been lost through the process of aging.

Some embodiments of the present disclosure provide for methods of treating a muscle disease or disorder comprising simultaneous administration of a gene replacement therapy to adjacent regions within an affected tissue of a subject.

In other embodiments, the present disclosure provides for methods of local delivery of a gene replacement therapy to a subject comprising a) providing a therapeutic agent comprising a gene replacement therapeutic agent and b) locally delivering said therapeutic agent to a tissue of said subject using a needle array device comprising a plurality of needles.

The present disclosure also provides for methods of delivering a selected gene to a muscle tissue in vivo, said method comprising: (a) providing a recombinant adeno-associated virus (AAV) virion which comprises an AAV vector, said AAV vector comprising said selected gene operably linked to control elements capable of directing the in vivo transcription and translation of said selected gene; and (b) introducing said recombinant AAV virion into said muscle tissue in vivo using a needle array device comprising a plurality of needles.

In some embodiments, the needles are configured for delivery by intramuscular injection.

In some embodiments, large scale delivery of therapeutic AAV vectors into muscles of an entire functional muscle group in the limb is achieved in a subject, e.g. cxmd dogs, using a porous needle delivery device. Expression of dystrophin protein in animals with muscular dystrophy can be achieved using the AAV6 virus of the disclosure. A gene replacement therapeutic agent delivery can enable a physician or advanced practice nurse to introduce AAV6 carrying the gene for dystrophin into greater than 1%, 2%, 5%, 10%, 20%, 30%, 40%, 50%, 60%, 70%, 80%, or 90% of all large muscle fibers of a single limb or one side of the trunk. A small team of medical practitioners, each working on a different body region, can allow complete gene therapy to be delivered within a single sedation period.

### Target Tissues

In some embodiments, the present disclosure exemplifies a system for screening candidate therapeutic agents in a solid tissue. Solid tissues are well known to the medical arts and may include any cohesive, spatially discrete non-fluid defined anatomic compartment that is substantially the product of multicellular, intercellular, tissue and/or organ architecture, such as a three-dimensionally defined compartment that may comprise or derive its structural integrity from associated connective tissue and may be separated from other body areas by a thin membrane (e.g., meningeal membrane, pericardial membrane, pleural membrane, mucosal membrane, basement membrane, omentum, organ-encapsulating membrane, or the like). Non-limiting exemplary solid tissues may include brain, liver, lung, kidney, prostate, ovary, spleen, lymph node (including tonsil), thyroid, pancreas, heart, skeletal muscle, intestine, larynx, esophagus and stomach. Anatomical locations, morphological properties, histological characterization, and invasive and/or non-invasive access to these and other solid tissues are all well known to those familiar with the relevant arts. In some embodiments, the tissue is normal. In some embodiments, the tissue is, or is suspected of being, cancerous, inflamed, infected, atrophied, numb, in seizure, or coagulated. In some embodiments, the tissue is, or is suspected of being, cancerous. In some embodiments, the tissue is cancerous.

In a preferred embodiment, the present method is directed to cancer, and the target tissue comprises a tumor, which may be benign or malignant, and comprises at least one cancer cell selected from the group consisting of a prostate cancer cell, a breast cancer cell, a colon cancer cell, a lung cancer cell, a brain cancer cell, and an ovarian cancer cell. In certain embodiments the tumor comprises a cancer selected from adenoma, adenocarcinoma, squamous cell carcinoma, basal cell carcinoma, small cell carcinoma, large cell undifferentiated carcinoma, chondrosarcoma and fibrosarcoma. Art-accepted clinical diagnostic criteria have been established for these and other cancer types, such as those promulgated by the U.S. National Cancer Institute (Bethesda, MD, USA) or as described in DeVita, Hellman, and Rosenberg's Cancer: Principles and Practice of Oncology (2008, Lippincott, Williams and Wilkins, Philadelphia/ Ovid, New York); Pizzo and Poplack, Principles and Practice of 25 Pediatric Oncology (Fourth edition, 2001, Lippincott, Williams and Wilkins, Philadelphia/Ovid, New York); and Vogelstein and Kinzler, The Genetic Basis of Human Cancer (Second edition, 2002, McGraw Hill Professional, New York). Other non-limiting examples of typing and characterization of particular cancers are described, e.g., in Ignatiadis et al. (2008 PathobioL 75:104); Curr. Drug Discov. Technol. 5:9); and Auman et al. (2008 Drug Metab. Rev. 40:303). In certain embodiments the selected region of tissue is a portion of a tumor in a subject, and in certain further embodiments the subject is one of a preclinical model and a human patient.

Certain preferred embodiments contemplate a subject or biological source that is a human subject such as a patient that has been diagnosed as having or being at risk for developing or acquiring cancer according to art-accepted clinical diagnostic criteria, such as those of the U.S. National Cancer Institute (Bethesda, MD, USA) or as described in DeVita, Hellman, and Rosenberg's Cancer: Principles and Practice of Oncology (2008, Lippincott, Williams and Wilkins, Philadelphia/ Ovid, New York); Pizzo and Poplack, Principles and Practice of Pediatric Oncology (Fourth edition, 2001, Lippincott, Williams and Wilkins, Philadelphia/ Ovid, New York); and Vogelstein and Kinzler, The Genetic Basis of Human Cancer (Second edition, 2002, McGraw Hill Professional, New York); certain embodiments contemplate a human subject that is known to be free of a risk for having, developing or acquiring cancer by such criteria.

Certain other embodiments contemplate a non-human subject or biological source, for example a non-human primate such as a macaque, chimpanzee, gorilla, vervet, orangutan, baboon or other non-human primate, including such non-human subjects that may be known to the art as preclinical models, including preclinical models for solid tumors and/or other cancers. Certain other embodiments contemplate a non-human subject that is a mammal, for example, a mouse, rat, rabbit, pig, sheep, horse, bovine, goat, gerbil, hamster, guinea pig or other mammal; many such mammals may be subjects that are known to the art as preclinical models for certain diseases or disorders, including solid tumors and/or other cancers (e.g., Talmadge et al., 2007 Am. J. Pathol. 170:793; Kerbel, 2003 Canc. Biol. Therap. 2(4 Suppl 1):S134; Man et al., 2007 Canc. Met. Rev. 26:737; Cespedes et al., 2006 Clin. TransL Oncol. 8:318). The range of embodiments is not intended to be so limited, however, such that there are also contemplated other embodiments in which the subject or biological source may be a non-mammalian vertebrate, for example, another higher vertebrate, or an avian, amphibian or reptilian species, or another subject or biological source. A transgenic animal is a non-human animal in which one or more of the cells of the animal includes a nucleic acid that is non-endogenous (i.e., heterologous) and is present as an extrachromosomal element in a portion of its cell or stably integrated into its germ line DNA (i.e., in the genomic sequence of most or all of its cells). In certain embodiments of the present invention, the tissue of a transgenic animal may be targeted. In some embodiments, the solid tissue is a xenograft produced by introducing one or more cells of one organism (e.g. cultured human cancer cells) into a nonhuman model organism.

The method of the invention is suitable for administering therapeutic agents to a variety of solid tissues; thus the method has medical and veterinary uses. In some embodiments, the animal is a pet, a companion, a guardian, a working animal, a breeding animal, a service animal, a racing animal, a farm animal, a herded animal, or a laboratory animal.

In some embodiments, the subject is a preclinical animal model. In some preferred embodiments, the subject is one of a mouse model or rat model. A preclinical model may be an animal model that is the recipient of a xenograft or xenotransplantation, terms that are used interchangeably to refer to the transplantation of living cells, tissues or organs from one species to another. In some preferred cases, the preclinical model is the recipient of one or more cancer cells that develops into a tumor. The recipient preclinical model may be an immunocompromised animal, such as a SCID mouse or nude mouse. An athymic nude mouse is a laboratory mouse from a strain with a genetic mutation that causes a deteriorated or absent thymus, resulting in an inhibited immune system due to a greatly reduced number of T cells. An immunocompromised state in a preclinical model may be the result of genetic abnormalities, or it may be the result of drug treatments to suppress immune system function. Immunosuppressive drugs or immunosuppressive agents are drugs that inhibit or prevent activity of the immune system. Non-limiting examples of immunosuppressive drugs include glucocorticoids; cytostatics; alkylating agents; antimetabolites including folic acid analogues, such as methotrexate, and purine analogues such as azathioprine and mercaptopurine; azathioprine and mercaptopurine; cytotoxic antibiotics, including dactinomycin, anthracyclines, mitomycin C, bleomycin, and mithramycin; polyclonal and monoclonal antibodies targeting elements of the immune system; and drugs acting on immunophilins, including cyclosporin, tacrolimus, voclosporin and other calcineurin inhibitors, and sirolimus; interferons, opioids, TNF-binding proteins, mycophenolate, and fingolimod.

In some embodiments, the solid tissue is soft tissue. Non-limiting examples of soft tissue include muscle, adipose, skin, tendons, ligaments, blood, and nervous tissue. Biological samples can be provided by obtaining a blood sample, biopsy specimen, tissue explant, organ culture, biological fluid or any other tissue or cell preparation from a subject or a biological source.

The subject or biological source can be a human or non-human animal, a transgenic or cloned or tissue-engineered (including through the use of stem cells) organism, a primary cell culture or culture adapted cell line including but not limited to genetically engineered cell lines that can contain chromosomally integrated or episomal recombinant nucleic acid sequences, immortalized or immortalizable cell lines, somatic cell hybrid cell lines, differentiated or differentiatable cell lines, transformed cell lines and the like. In some embodiments of the invention, the subject or biological source can be suspected of having or being at risk for having a malignant condition, and in some embodiments of the invention the subject or biological source can be known to be free of a risk or presence of such disease.

### Fluid Agents

In certain embodiments the fluid agent comprises an agent that is selected from (a) a gene therapy agent; (b) a chemotherapy agent; (c) a small molecule; (d) an antibody; (e) a protein; (f) one of a small interfering RNA and an encoding polynucleotide therefor; (g) one of an antisense RNA and an encoding polynucleotide therefor, (h) one of a ribozyme and an encoding polynucleotide therefor; (i) a detectable label; (j) one of a therapeutic protein, polypeptide, and a peptidomimetic; and (k) a microRNA (miRNA). In certain further embodiments the detectable label is selected from a radiolabel, a radio-opaque label, a fluorescent label, a colorimetric label, a dye, an enzymatic label, a GCMS tag, avidin, and biotin. In certain embodiments the agent is selected from (i) a gene therapy agent that comprises at least one operably linked promoter, (ii) a small interfering RNA-encoding polynucleotide that comprises at least one operably linked promoter; (iii) an antisense RNA encoding polynucleotide that comprises at least one operably linked promoter; and (iv) a ribozyme-encoding polynucleotide that comprises at least one operably linked promoter. In certain further embodiments the operably linked promoter is selected from a constitutive promoter and a regulatable promoter. In certain still further embodiments the regulatable promoter is selected from an inducible promoter, a tightly regulated promoter and a tissue-specific promoter.

Candidate agent or candidate compound refers to any fluid or molecule in an aqueous solution, mixture, or colloid that may be delivered to a target tissue. When used to refer to agents delivered from needles, the term fluid is to be read broadly to read on any substance capable of flowing through such a needle, including liquids, gases, colloids, suspended solids, etc.

Selection of candidate oncology agents is understood and determinable by one skilled in the relevant arts (see, *e.g.,* Berkowet al., eds., The Merck Manual, 16th edition, Merck and Co., Rahway; N.J., 1992; Goodman et al., eds., Goodman and Gilman's The Pharmacological Basis of Therapeutics, 10th edition, Pergamon Press, Inc., Elmsford, N.Y., (2001); De Vita, Hellman , and Rosenberg's Cancer: Principles and Practice of Oncology (2008, Lippincott, Williams and Wilkins, Philadelphia/ Ovid, New York); Pizzo and Poplack, Principles and Practice of Pediatric Oncology (Fourth edition, 2001, Lippincott, Williams and Wilkins, Philadelphia/ Ovid, New York); Avery's Drug Treatment: Principles and Practice of Clinical Pharmacology and Therapeutics, 3rd edition, ADIS Press, LTD., Williams and Wilkins, Baltimore, MD. (1987), Ebadi, Pharmacology, Little, Brown and Co., Boston, (1985); Osolci al., eds., Remington's Pharmaceutical Sciences, 18th edition, Mack Publishing Co., Easton, PA (1990); Katzung, Basin and Clinical Pharmacology, Appleton and Lange, Norwalk, CT (1992)). Candidate agents can be selected from resources that disclose listings of investigational therapeutics, for instance, the National Institutes of Health (Bethesda, MD) which maintains a database of ongoing and planned clinical trials at its "ClinicalTrials.gov" website.

Candidate agents for use in screening methods and in methods of rating candidate agents for development into therapeutic agents such as a therapeutic agent for treating a solid tumor can be provided as "libraries" or collections of compounds, compositions or molecules. Such molecules typically include compounds known in the art as "small molecules" and having molecular weights less than 10⁵ daltons, less than 10⁴ daltons, or less than 10³ daltons.

For example, a plurality of members of a library of test compounds can be introduced as candidate agents to a region of a solid tumor of known tumor type in each one or a plurality of subjects having a tumor of the known tumor type, by distributing each of the candidate agents to a plurality of positions along an axis within the region in each subject, and after a selected period of time *(e.g.,* a range of time, a minimum time period or a specific time period) the region of solid tumor in which the candidate agents have been introduced can be imaged or removed from each subject, and each region compared by detecting an effect (if any) of each agent on the respective position within the region, for instance, by determining whether an altered physiologic state is present as provided herein, relative to positions in the region that are treated with control agents as provided herein, which would either produce no effect (negative control) or a readily detectable effect (positive control).

Candidate agents further can be provided as members of a combinatorial library, which can include synthetic agents prepared according to a plurality of predetermined chemical reactions performed in a plurality of reaction vessels. For example, various starting compounds can be prepared employing one or more of solid-phase synthesis, recorded random mix methodologies and recorded reaction split techniques that permit a given constituent to traceably undergo a plurality of permutations and/or combinations of reaction conditions. The resulting products comprise a library that can be screened followed by iterative selection and synthesis procedures, such as a synthetic combinatorial library of peptides (see *e.g.*, PCT/US91/08694, PCT/US91/04666, which are hereby incorporated by reference in their entireties) or other compositions that can include small molecules as provided herein (see *e.g.,* PCT/US94/08542, EP 0774464, U.S. 5,798,035, U.S. 5,789,172, U.S. 5,751,629, which are hereby incorporated by reference in their entireties). Those having ordinary skill in the art will appreciate that a diverse assortment of such libraries can be prepared according to established procedures, and tested for their influence on an indicator of altered mitochondrial function, according to the present disclosure.

Other candidate agents can be proteins (including therapeutic proteins), peptides, peptidomimetics, polypeptides, and gene therapy agents (e.g., plasmids, viral vectors, artificial chromosomes and the like containing therapeutic genes or polynucleotides encoding therapeutic products, including coding sequences for small interfering RNA (siRNA), ribozymes and antisense RNA) which in certain further embodiments can comprise an operably linked promoter such as a constitutive promoter or a regulatable promoter, such as an inducible promoter (*e.g*., IPTG-inducible), a tightly regulated promoter *(e.g.,* a promoter that permits little or no detectable transcription in the absence of its cognate inducer or derepressor) or a tissue-specific promoter. Methodologies for preparing, testing and using these and related agents are known in the art. See, *e.g.,* Ausubel (Ed.), Current Protocols in Molecular Biology (2007 John Wiley & Sons, NY); Rosenzweig and Nabel (Eds), Current Protocols in Human Genetics (esp. Ch. 13 therein, "Delivery Systems for Gene Therapy", 2008 John Wiley & Sons, NY); Abell, Advances in Amino Acid Mimetics and Peptidomimetics, 1997 Elsevier, NY.

Other candidate agents can be antibodies, including naturally occurring, immunologically elicited, chimeric, humanized, recombinant, and other engineered antigen-specific immunoglobulins and artificially generated antigen-binding fragments and derivatives thereof, such as single-chain antibodies, minibodies, Fab fragments, antibody/drug conjugates, bi-specific antibodies and the like. See, *e.g.,* Coligan et al. (Eds.), Current Protocols in Immunology (2007 John Wiley & Sons, NY); Harlow and Lane, Antibodies: A Laboratory Manual (1988 Cold Spring Harbor Press, Cold Spring Harbor, NY); Harlow and Lane, Using Antibodies (1999 Cold Spring Harbor Press, Cold Spring Harbor, NY).

Pharmaceutically acceptable carriers for therapeutic use are well known in the pharmaceutical art, and are described, for example, in Remingtons Pharmaceutical Sciences. Mack Publishing Co. (A.R. Gennaro edit. 1985). For example, sterile saline and phosphate-buffered saline at physiological pH can be used. Preservatives, stabilizers, dyes and other ancillary agents can be provided in the pharmaceutical composition. For example, sodium benzoate, sorbic acid and esters of p-hydroxybenzoic acid can be added as preservatives. Id. at 1449. In addition, antioxidants and suspending agents can be used. Id. "Pharmaceutically acceptable salt" refers to salts of drug compounds derived from the combination of such compounds and an organic or inorganic acid (acid addition salts) or an organic or inorganic base (base addition salts). The agents, including drugs, contemplated for use herein can be used in either the free base or salt forms, with both forms being considered as being within the scope of the certain present invention embodiments.

The pharmaceutical compositions that contain one or more agents can be in any form which allows for the composition to be administered to a subject. According to some embodiments the composition will be in liquid form and the route of administration will comprise administration to a solid tissue as described herein. The term parenteral as used herein includes transcutaneous or subcutaneous injections, and intramuscular, intramedullar and intrastemal techniques.

The pharmaceutical composition is formulated so as to allow the active ingredients contained therein to be bioavailable upon administration of the composition to a subject such as a human subject. Compositions that will be administered to a subject can take the form of one or more doses or dosage units, where for example, a pre-measured fluid volume can comprise a single dosage unit, and a container of one or more compositions (e.g., drugs) in liquid form can hold a plurality of dosage units. A dose of a drug includes all or a portion of a therapeutically effective amount of a particular drug that is to be administered in a manner and over a time sufficient to attain or maintain a desired concentration range of the drug, for instance, a desired concentration range of the drug in the immediate vicinity of a delivery needle in a solid tissue, and where the absolute amount of the drug that comprises a dose will vary according to the drug, the subject, the solid tissue and other criteria with which the skilled practitioner will be familiar in view of the state of the medical and pharmaceutical and related arts. In certain embodiments at least two doses of the drug can be administered, and in certain other embodiments 3, 4, 5, 6, 7, 8, 9, 10 or more doses can be administered.

A liquid pharmaceutical composition as used herein, whether in the form of a solution, suspension or other like form, can include one or more of the following adjuvants: sterile diluents such as water for injection, saline solution, physiological saline, Ringer's solution, saline solution (e.g., normal saline, or isotonic, hypotonic or hypertonic sodium chloride), fixed oils such as synthetic mono or digylcerides which can serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. In some embodiments, physiological saline is the adjuvant. An injectable pharmaceutical composition can be sterile. It can also be desirable to include other components in the preparation, such as delivery vehicles including but not limited to aluminum salts, water-in-oil emulsions, biodegradable oil vehicles, oil-in-water emulsions, biodegradable microcapsules, hydrogels, and liposomes.

While any suitable carrier known to those of ordinary skill in the art can be employed in the pharmaceutical compositions of this invention, the type of carrier will vary depending on the mode of administration and whether a conventional sustained drug release is also desired. For parenteral administration, such as supplemental injection of drug, the carrier can comprise water, saline, alcohol, a fat, a wax or a buffer. Biodegradable microspheres (e.g., polylactic galactide) can also be employed as carriers for the pharmaceutical compositions of this invention. Suitable biodegradable microspheres are disclosed, for example, in U.S. Patent Nos. 4,897,268 and 5,075,109. In some embodiments, the microsphere be larger than approximately 25 microns, while other embodiments are not so limited and contemplate other dimensions.

Pharmaceutical compositions can also contain diluents such as buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. In some embodiments, an agent (e.g., a therapeutic drug or a candidate drug) is formulated as a lyophilizate using appropriate excipient solutions (e.g., sucrose) as diluents.

Certain embodiments contemplate direct delivery of multiple drugs, candidate drugs, imaging agents, positional markers, indicators of efficacy and appropriate control compositions to a plurality of spatially defined locations along parallel axes in a solid tissue, such as a solid tumor, followed, after a desired time interval, by excision of the treated tissue and evaluation or analysis of the tissue for effects of the treatments. Indicators of efficacy can be, for example, detectable indicator compounds, nanoparticles, nanostructures or other compositions that comprise a reporter molecule which provides a detectable signal indicating the physiological status of a cell, such as a vital dye (*e.g.,* Trypan blue), a colorimetric pH indicator, a fluorescent compound that can exhibit distinct fluorescence as a function of any of a number of cellular physiological parameters (e.g., pH, intracellular Ca²⁺ or other physiologically relevant ion concentration, mitochondrial membrane potential, plasma membrane potential, etc., *see* Haugland, The Handbook: A Guide to Fluorescent Probes and Labeling Technologies (10th Ed.) 2005, Invitrogen Corp., Carlsbad, CA), an enzyme substrate, a specific oligonucleotide probe, a reporter gene, or the like. Control compositions can be, for example, negative controls that have been previously demonstrated to cause no statistically significant alteration of physiological state, such as sham injection, saline, DMSO or other vehicle or buffer control, inactive enantiomers, scrambled peptides or nucleotides, etc.; and positive controls that have been previously demonstrated to cause a statistically significant alteration of physiological state, such as an FDA-approved therapeutic compound.

In some embodiments, the fluid agent further comprises a dye. The dye can be imaged after administration of the pharmaceutical composition to a solid tissue to observe the distribution and activity of a therapeutic agent present in the same pharmaceutical composition. In some embodiments, the dye is a fluorescent dye. In some embodiments, the dye is a radioactive dye.

In some embodiments, the fluid agent comprises a positional marker. Positional markers are known and include, as non-limiting examples, fluorescent quantum dots, India ink, metal or plastic beads, dyes, stains, tumor paint (Veiseh et al., 2007 Canc. Res. 67:6882) or other positional markers, and can be introduced at desired positions. Markers can include any subsequently locatable source of a detectable signal, which can be a visible, optical, colorimetric, dye, enzymatic, GCMS tag, avidin, biotin, radiological (including radioactive radiolabel and radio-opaque), fluorescent or other detectable signal.

A detectable marker thus comprises a unique and readily identifiable gas chromatography/mass spectrometry (GCMS) tag molecule. Numerous such GCMS tag molecules are known to the art and can be selected for use alone or in combination as detectable identifier moieties. By way of illustration and not limitation, various different combinations of one, two or more such GCMS tags can be added to individual reservoirs of the device described herein in a manner that permits the contents of each reservoir to be identified on the basis of a unique GCMS "signature", thereby permitting any sample that is subsequently recovered from an injection region to be traced back to its needle of origin for identification purposes. Examples of GCMS tags include α, α, α-trifluorotoluene, α-methylstyrene, o-anisidine, any of a number of distinct cocaine analogues or other GCMS tag compounds having readily identifiable GCMS signatures under defined conditions, for instance, as are available from SPEX CertiPrep Inc. (Metuchen, NJ) or from SigmaAldrich (St. Louis, MO), including Supelco® products described in the Supelco® 2005 gas chromatography catalog and available from SigmaAldrich.

### Porous Tubes

In some embodiments, the present method provides for the administration of a fluid agent to a tissue through the use of one or more porous tubes. The fluid agent contacts the tissue by diffusion through pores of the porous tubes. Porous tubes used in the devices and methods of the present application may be hollow, or may uniformly comprise porous material. The porous tubes are suitable for containing, storing, administering, delivering, and transporting contents. The contents can be a pharmaceutical composition comprising one or more therapeutic agents. The therapeutic agents within a single hollow and/or porous tube can be the same or can be a mixture of different types of therapeutic agents. Within a plurality of hollow and/or porous tubes, each tube can contain the same therapeutic agents as another tube, or different therapeutic agents as another tube. In some embodiments, every hollow and/or porous tube contains therapeutic agents that are unique from the therapeutic agents contained in every other tube of the plurality of tubes.

The hollow and/or porous tubes can be connected to a frame that holds the tubes and facilitates drug delivery. The hollow and/or porous tubes can be detachable from the frame. The number and spatial orientation of hollow and/or porous tubes connected to the frame can be varied based on the drug-delivery needs of a subject.

A hollow and/or porous tube is made of a tube material. The tube material is suitable for containing, storing, administering, delivering, and transporting a fluid agent. The contents can be a pharmaceutical composition comprising one or more therapeutic agents. In some embodiments, the tube material is essentially inert to acid. In some embodiments, the tube material is essentially inert to base. In some embodiments, the tube material is essentially inert to acid and base. In some embodiments, the tube material is insoluble in water. In some embodiments, the tube material is insoluble in organic solvents. In some embodiments, the tube material is essentially insoluble in organic solvents. In some embodiments, the tube material is insoluble in non-halogenated organic solvents. In some embodiments, the tube material is essentially insoluble in non-halogenated organic solvents.

The tube material is biocompatible. The tube material is essentially physiologically-inactive, and does not trigger physiological events. The tube material does not cause inflammation, immune response, infection, or any other sort of rejection within a solid tissue. In some embodiments, the tube material is biodegradable. In some embodiments, the tube material decomposes over time within a solid tissue. The tube material is thermostable, and the tubes can be sterilized in an autoclave prior to use on a subject.

The tube material is suitable for being shaped into a tube, but also suitable for retaining the tube shape upon deposition into solid tissue. The tube material is suitable for being broken, cut, sliced, disjoined, or separated in a clean way, and can be broken, cut, sliced, disjoined, or separated after deposition into a solid tissue. In some embodiments, the tube material is scissile.

In some embodiments, the tube material is polymeric. In some embodiments, the tube material is co-polymeric. In some embodiments, the tube material is a cross-linked polymer or co-polymer. Non-limiting examples of tube materials include polysulfone, polyamine, polyamide, polycarbonate, polycarbamate, polyurethane, polyester, polyether, polyolefin, polyaromatic materials. In some embodiments, the tube material is polysulfone.

The preparation of hollow tubes from polymers can be achieved by various routes. These are referred to as wet, dry or melt-forming processes. Melt-forming involves heating a polymer above its melting point and extruding it through an orifice (usually referred to as a die) which is designed to form a hollow tube. Once extruded, the melt is cooled via a quench which allows the polymer to solidify into a fine tube. In the dry-forming process, a solution of the polymer is extruded through a desired orifice and is fed into a heated column which allows for evaporation of the solvent and subsequent formation of a tube. In a wet-membrane forming process, a solution of the polymer is extruded though an orifice and quenched in a non-solvent for the polymer resulting in coagulation of the polymer to a tube. Of the above mentioned forming processes, wet-membrane forming allows one to easily produce hollow porous tubes. The particular forming process used will be dependent upon the polymer used and type of hollow tube desired.

In some embodiments, the tube material comprises a plurality of pores. The contents of the tube can diffuse from the tube into solid tissue via the pores. The rate of diffusion form the porous tube into the solid tissue can be influenced by the pore size, for example, larger pores result in a higher diffusion rate. In some embodiments, the tube material is permeable. In some embodiments, a porous tube is permeable.

The effective agent diffuses in the direction of lower chemical potential, i.e., toward the exterior surface of the device. At the exterior surface of the device, equilibrium is again established. A steady state flux of the effective agent will be established in accordance with Fick's Law of Diffusion. The rate of passage of the drug through the material by diffusion is generally dependent on the solubility of the drug therein, as well as on the thickness of a porous wall. Selection of porous tube materials may depend on the particular fluid agent to be delivered.

In producing a porous material, the size of the pores is affected by the solvent strength of a polymer. A rapid decrease in solvent strength often tends to entrap a dispersion of small droplets within the continuous polymer phase. A slow decrease in solvent strength allows for nucleation sites within the polymer matrix allowing for formation of larger pores. In such cases, the reduction in solvent strength must be rapid enough to allow for the structure of the membrane to set.

Another way to change porosity and volume of the porous network in producing a porous polymer is to change the concentration of the polymer solution. Lower concentrations have a tendency to promote larger pores and greater pore volume. However, there is a limit to how high (usually no more than 45% w/w) the polymer concentration can be in a solvent. Otherwise, the polymer will become the dispersed phase in a continuous solvent phase, thereby eliminating the porous network. Another method to achieve porous tubular membranes is to cause a rapid phase inversion of the polymer solution by cooling.

In preferred embodiments, the average pore size is about 50 nm, 100 nm, 200 nm, 300 nm, 400 nm, 500 nm, 600 nm, 700 nm, 800 nm, 900 nm, 1 µm, 2 µm, 3 µm,5 µm, 10 µm, 20 µm, 30 µm, 50 µm, 100 µm, or 500 µm. All the pores of a single tube can be about the same pore size. In some embodiments, each pore of a single tube has a pore size that is independent of the pore size of all the other pores of the tube. Within a plurality of porous tubes, all pores can have about the same pore size, or each pore can have a size that is independent of the size of all the other pores of the plurality of porous tubes.

Within a single porous tube, the pore sizes can vary by as much as 5%, 10%, 15%, 20%, 25%, 30%, 40%, 50%, 60%, 70%, 75%, 80%, 90%, 100%, 125%, 150%, 200%, 300%, 400%, 500%, 750%, or 1,000%. Within a single porous tube, the pore sizes can vary by as much as about 5%, about 10%, about 15%, about 20%, about 25%, about 30%, about 40%, about 50%, about 60%, about 70%, about 75%, about 80%, about 90%, about 100%, about 125%, about 150%, about 200%, about 300%, about 400%, about 500%, about 750%, or about 1,000%.

The pore size can influence the rate of diffusion, and the pore size can be modulated to influence the rate of diffusion. A porous tube can be generated having a pre-determined average pore size for the purpose of influencing the rate of diffusion. Different pharmaceutical compositions of therapeutic agents can diffuse form the porous tubes at varying rates, influenced in part by the physical and chemical properties of the pharmaceutical compositions, therapeutic agents, and porous tube materials. Porous tubes with varying average pore sizes can be generated and used experimentally to find a pore size that provides a desired diffusion rate for a specific pharmaceutical composition or therapeutic agent.

In a plurality of tubes, all tubes can have about the same tube length, or each tube can have a tube length that is independent of the tube length of all the other tubes of the plurality of tubes. A tube has a diameter. In a plurality of tubes, all tubes can have about the same diameter, or each tube can have a diameter that is independent of the diameter of all the other tubes of the plurality of tubes. A tube has a wall thickness. In a plurality of tubes, all tubes can have about the same wall thickness, or each tube can have a wall thickness that is independent of the wall thickness of all the other tubes of the plurality of tubes.

A porous tube has a top end and a bottom end. In some embodiments, the entire tube contains a fluid agent, such as a pharmaceutical composition or therapeutic agents. In some embodiments, the bottom end contains a fluid agent, such as a pharmaceutical composition or therapeutic agents, and the top end does not contain a fluid agent, such as a pharmaceutical composition or therapeutic agents.

The bottom end of a tube can be attached to a device suitable for assisting in the administration of the contents into a solid tissue. The bottom end of a tube can be connected, for example, to a needle, port, catheter, intravenous line, or other apparatus suitable for delivering a pharmaceutical composition into a solid tissue. In some embodiments, the apparatus (e.g., a needle) is suitable for penetrating a solid tissue.

The top end of a tube can be attached to a device suitable for assisting in the administration of the contents into a solid tissue. The top end of a tube can be connected, for example, to a plunger, pump, piston, or other apparatus suitable for providing a pressure sufficient to deliver a pharmaceutical composition into a solid tissue, or any such device described herein.

The tubes can be loaded, packed, or charged with a fluid agent. The tubes can be loaded immediately prior to use, or can be loaded, stored, and shipped. Either end of a porous tube, or both ends, may be sealed following loading with a fluid agent. In some cases, one or both ends of a porous tube may be sealed prior to loading with a fluid agent by, for example, soaking the tube for an extended period of time in the fluid agent.

The narrow diameter and shape of the tubes provides for convenient loading by capillary action. A tube, or a plurality thereof, can be dipped into a fluid pharmaceutical composition, and the pharmaceutical composition can be drawn into the tubes. In a closed environment, the application of positive pressure to the pharmaceutical composition results in loading a greater amount of the pharmaceutical composition into the tubes; thus, the amount of pharmaceutical formulation in a tube can be controlled easily and reliably.

The porosity of the tubes can provide for convenient loading by soaking the tubes in a bath of a fluid pharmaceutical composition. The pharmaceutical composition can diffuse into the tubes, for example, through the pores or via permeability of the tube material. The amount of pharmaceutical composition that diffuses into the tubes can be influenced, for example, by external pressure, pore size, permeability, tube length, bath depth, bath amount, amount of time spent in the bath, and tube material.

A pharmaceutical composition loaded into a hollow and/or porous tube comprises one or more therapeutic agents. Non-limiting examples of therapeutic agents compatible with the invention are detailed elsewhere herein and include anti-cancer agents, anti-inflammatory agents, anti-infective agents, regenerative agents, relaxing agents, apoptosis-inhibiting agents, apoptosis-inducing agents, anti-coagulatory agents, dermatological agents, growth-stimulating agents, vasodilating agents, vasorestricting agents, analgesic agents, anti-allergic agents, and any therapeutic agents described herein. In some embodiments, the therapeutic agent is an anti-cancer agent.

### Hydrogels

Hydrogels are described in US patent application 20100189794 to Luo, Lee, et al., published July 29, 2010 entitled "Nucleic Acid Hydrogel via Rolling Circle Amplification," hereby incorporated by reference in its entirety.

In some embodiments, a composition comprising a hydrogel is delivered to a tissue. The hydrogel is effective to slow the rate of diffusion or dispersion of a pharmaceutical formulation through a solid tissue. In some embodiments, a pharmaceutical composition containing the hydrogel disperses through a solid tissue to a lesser degree than does an analogous pharmaceutical composition lacking the hydrogel. In some embodiments, a pharmaceutical composition containing the hydrogel disperses through a solid tissue more slowly than does an analogous pharmaceutical composition lacking the hydrogel. The hydrogel may be one polymer or a mixture of two or more polymers. In some embodiments, the hydrogel has In some embodiments, the hydrogel comprises a binary mixture of at least one polymer with two different molecular weight ranges. In some embodiment, the hydrogel is PEG. In a further embodiment, the two different molecular ranges comprise 500-2,000 and 4,000-8,000. In another further embodiment, the two different molecular ranges comprise 2,000-4,000 and 8,000-20,000. In another further embodiment, the two different molecular ranges comprise 4,000-8,000 and 10,000-45,000. By adjusting the molecular weight and ratio of the binary system, the diffusion rate of the fluid agents can be controlled.

Another property for characterization of hydrogel is tensile strength. The hydrogel may have a tensile strength of at least 20 gf/cm² in the dry state. In a further embodiment, the hydrogel has a tensile strength of 20-120 gf/cm² in the dry state. In some embodiments, the hydrogel may have a tensile strength of at least 5 gf/cm² in the hydrate state. In a further embodiment, the hydrogel has a tensile strength of 5-15 gf/cm² in the hydrate state.

The hydrogel can be present in an amount from about 1% to about 99% of a pharmaceutical composition. In some embodiments, the hydrogel is present in an amount of about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.5%, about 6%, about 6.5%, about 7%, about 7.5%, about 8%, about 8.5%, about 9%, about 9.5%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% of a pharmaceutical composition.

The hydrogel can be porous. Exemplary porous hydrogel suitable for biological use are described in U.S. Pat. No. 4,014,335, which is incorporated herein by reference in its entirety. These materials include cross-linked polyvinyl alcohol, polyolefins or polyvinyl chmorides or cross-linked gelatins; regenerated, insoluble, non-erodible cellulose, acylated cellulose, esterified celluloses, cellulose acetate propionate, cellulose acetate butyrate, cellulose acetate phthalate, cellulose acetate diethyl-aminoacetate; polyurethanes, polycarbonates, and microporous polymers formed by co-precipitation of a polycation and a polyanion modified insoluble collagen.

In some embodiments, the hydrogel comprises collagen. Non-limiting examples of sources of collagen include Engelbreth-Holm-Swarm murine sarcoma basement membrane, bovine achilles tendon, bovine nasal septum, bovine tracheal cartilage, calf skin, chicken sternal cartilage, human lung , human placenta, kangaroo tail, mouse sternum, and rat tail tendon. In some cases, collagen may comprise more than 0.1%, 0.2%, 0.5%, 0.7%, 1%, 1.2%, 1.4%, 1.6%, 1.8%, 2%, or 5% of the hydrogel. In some cases, recombinant collagen may be used. Several sources of collagen are described in US Patent Application No. US20070254041. Collagen material that is insoluble in water can be used, and can be derived from natural tissue sources (e.g. xenogenic, allogenic, or autogenic relative to the recipient human or other patient) or recombinantly prepared. Collagens can be subclassified into several different types depending upon their amino acid sequence, carbohydrate content and the presence or absence of disulfide crosslinks. Types I and III collagen are two of the most common subtypes of collagen. Type I collagen is present in skin, tendon and bone, whereas Type III collagen is found primarily in skin. The collagen used in compositions of the invention can be obtained from skin, bone, tendon, or cartilage and purified by methods well known in the art and industry. Alternatively, the collagen can be purchased from commercial sources. Type I bovine collagen is preferred for use in the invention.

The collagen can be atelopeptide collagen and/or telopeptide collagen. Still further, either or both of non-fibrillar and fibrillar collagen can be used. Non-fibrillar collagen is collagen that has been solubilized and has not been reconstituted into its native fibrillar form.

Suitable collagen products are available commercially, including for example from Kensey Nash Corporation (Exton, Pa.), which manufactures a fibrous collagen known as semed F, from bovine hides. Collagen materials derived from bovine hide are also manufactured by Integra Life Science Holding Corporation (Plainsboro, N.J.). Naturally-derived or recombinant human collagen materials are also suitable for use in the invention. Ilustratively, recombinant human collagen products are available from Fibrogen, Inc. (San Francisco, Calif.). The solid particulate collagen incorporated into the inventive compositions can be in the form of intact or reconstituted fibers, or randomly-shaped particles, for example.

Collagen can be dissolved in water to form an aqueous solution at room temperature, but undergoes polymerization to form a gel at 37 degrees. Miyata notes in US Pat. No. 4,164,559 that the chemistry, molecular structure and biochemical properties of collagen have been well established (Annual Review of Biophysics and Bioengineering, Vol. 3, pp. 231-253, 1974). Collagen is a major protein of connective tissue such as cornea, skin, etc., and can be solubilized and purified by the treatment with protelolytic enzymes (other than collagenase) such as pepsin. Solubilized collagen is telopeptides-poor, relatively inexpensive, not antigenic and useful as a biomedical material. Enzyme solubilized native collagen is soluble in acidic pH but polymerizes to form a gel at physiologic pH and at 37 degrees.

In other embodiments, the hydrogel comprises polyethylene glycol (PEG), in various formulations known in the art. Non-limiting examples of polymers that may be present in PEG hydrogels include polylactic acid (PLA), poly(lactic-co-glycolic acid) (PLGA), and polycaprolactone (PCL). Some examples of these copolymers include PLA-PEG-PLA, PLGA-PEG-PLA, and mPEG-b+PCL(1200)-b-PEG(6000)-b-PCL(1200) copolymer. The PEG may have a molecular weight of at least 500. In a further embodiment, the PEG has a molecular weight of 4,000-8,000. In another further embodiment, the PEG has a molecular weight of 8,000-45,000. PLGA is a copolymer which is used in a host of Food and Drug Administration (FDA) approved therapeutic devices, owing to its biodegradability and biocompatibility. PLGA is synthesized by means of random ring-opening co-polymerization of two different monomers, the cyclic dimers (1,4-dioxane-2,5-diones) of glycolic acid and lactic acid. Depending on the ratio of lactide to glycolide used for the polymerization, different forms of PLGA can be obtained: these are usually identified in regard to the monomers' ratio used (e.g. PLGA 75:25 identifies a copolymer whose composition is 75% lactic acid and 25% glycolic acid). All PLGAs are amorphous rather than crystalline and show a glass transition temperature in the range of 40-60 degrees. There is very minimal systemic toxicity associated with using PLGA for drug delivery or biomaterial applications. PLA is a biodegradable, thermoplastic, aliphatic polyester derived from renewable resources, such as corn starch, tapioca products, or sugarcanes. PCL is a biodegradable polyester with a low melting point of around 60°C and a glass transition temperature of about -60°C. PCL is prepared by ring opening polymerization of e-caprolactone using a catalyst such as stannous octanoate. PCL is an FDA-approved material that is used in the human body, and undergoes slow degradation upon implantation.

### Drug Implants

In some embodiments, the present disclosure relates to methods of performing multiplexed chemotherapy drug efficacy studies using drug implants in live tumors. Methods of the disclosure have advantages over screening in vitro cell culture models, because in vitro models do not respond to drugs in the same way as tumors grown in intact animal models of cancer or in patients. Therefore decisions to pursue development of drugs for entry into the clinic, or for therapeutic intervention of cancer in a patient, based on in vitro models are flawed. In contrast, the present disclosure provides methods for testing multiple potential anti-cancer agents simultaneously in the context of a live human tumor or in the context of an animal model of cancer.

In some embodiments, individually prepared drug implants comprising anti-cancer agents or test agents are distributed within a solid tissue using an arrayed head of introducer needles that enable parallel insertion of the drug sticks into the tumor. An injection device can allow drug implants to be inserted through the introducer needles into the tumor. A drug implant can comprise a solid rod or stick, and may preferably comprise a drug agent in solid form. In some cases, the drug implant comprises an inert biodegradable binder that permits sustained or timed release of the drug within the tissue.

In some embodiments, a drug implant has a diameter of less than about 0.1, 0.2, 0.3, 0.4, 0.5, 0.6, 0.7, 0.8, 0.9, or 1 mm. A needle array head can be configured to accommodate about 1, 2, 3, 4, 5, 6, 7, 8, 9, 10, 20, 30, 40, 50, 60, 70, 80, 100, 200, 300, 400, or more than 1,000 drug implants. A plurality of drug implants can be bundled to form a bundle of drug implants, wherein the bundle has a diameter suitable for injection using a needle.

In some embodiments, an injection device comprises a plunger configured to push one or more drug implants through needles into a solid tissue. In some embodiments, a solid tissue comprises a tumor.

Following insertion into a tumor, a drug agent can be released from one or more drug implants over a period of one minute to six weeks to allow discrete portions of the tumor to be exposed to each drug individually in a spatially confined manner.

Since drugs are inserted using methods of the present disclosure along parallel axes down the depth or z-axis of the tumor, sections of the tumor can be made cross-wise (perpendicular, or orthogonal to) the insertion path of the drug sticks to enable analysis of tumor response in the area proximal to each inserted drug, thereby allowing multiplexed analysis of drug efficacy in the context of a living tumor. Alternatively, the drug can be inserted parallel to the body plane.

In some embodiments of the present disclosure, one or more drug implants are inserted into a tissue through use of a carrier device such as a needle. In some preferred embodiments, a plurality of drug implants is inserted along parallel axes of a tissue through the use of a needle array of the disclosure.

In some cases, a plurality of needles is attached to a plurality of actuators coupled to a plurality of drug implants or bundles of drug implants within a plurality of reservoirs, such that depressing the plunger causes ejection of the drug implants, or injection of the drug implants into a tissue. In certain further embodiments the plungers of the plurality of plungers are operatively coupled together at respective second ends so as to be simultaneously depressable.

An implant can be partially- or fully-submerged in the tissue. Deposition of implants into the tissue can be facilitated by inserting the implant into the tissue via a needle. The implant can be further deposited into the tissue by depression of a plunger associated with the needle.

Once an implant has been deposited into the tissue, the implant can dissolve and diffuse into the tissue. The rate of diffusion can be influenced by the volume of the implant, or it can be influenced by the presence of an inert binder that serves to reduce the rate of dissolution of the implant within the tissue. The drug agent can diffuse into the tissue over a period of about a minute to about a month; about an hour to about six weeks; about 12 hours to about 72 hours; about 24 hours to about 48 hours; about one week to two weeks; about two weeks to three weeks; or about three weeks to four weeks.

### Delivery Devices and Methods

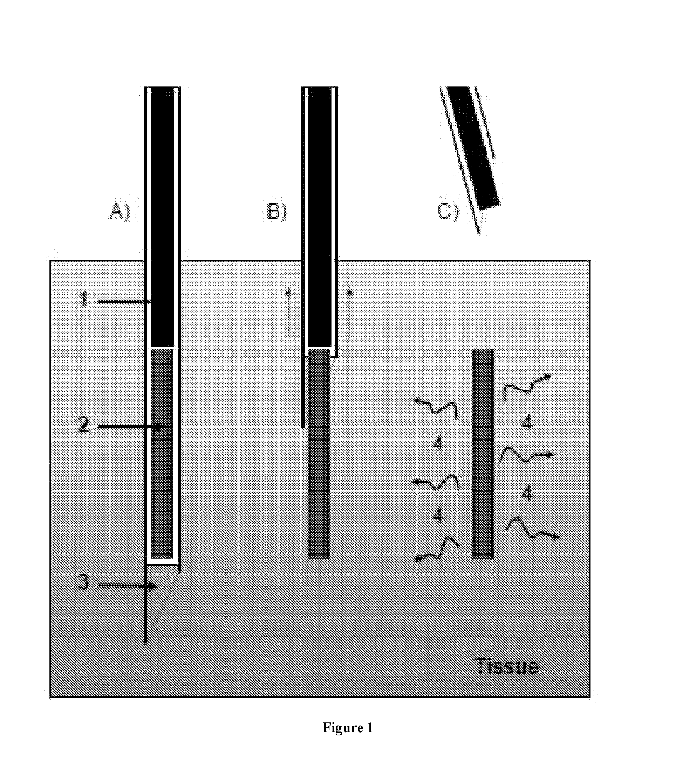

Figure 1 illustrates delivery of a candidate agent to a tissue through use of a needle and a drug implant. A needle 3 is inserted into a tissue, and a plunger 1 is depressed to inject a drug implant 2 into the tissue. Following insertion into the tissue, the candidate agent 4 is delivered to the tissue such as by diffusion.

In some embodiments, one or more porous tubes are inserted into a tissue through use of a carrier device such as a needle. In some preferred embodiments, a plurality of porous tubes is inserted along parallel axes of a tissue through the use of a needle array, such as that described in PCT/US2008/073212, which is hereby incorporated by reference in its entirety.

In some cases, a plurality of needles is attached to a plurality of actuators coupled to a plurality of porous tubes or bundles of porous tubes within a plurality of reservoirs, such that depressing the plunger causes ejection of the porous tubes, or injection of the porous tubes into a tissue. In certain further embodiments the plungers of the plurality of plungers are operatively coupled together at respective second ends so as to be simultaneously depressable. Certain still further embodiments comprise a plunger driver configured to depress all of the plurality of plungers at a selectively variable rate. In other embodiments each of the plurality of actuators comprises one of a plurality of fluid transmission lines having first and second ends, a first end of each of the plurality of fluid transmission lines being coupled to a respective one of the plurality of reservoirs. In other embodiments the device comprises a fluid pressure source, and each of the plurality of actuators comprises a fluid coupling between the fluid pressure source and a respective one of the plurality of reservoirs. In further embodiments the fluid pressure source comprises at least one of a compressor, a vacuum accumulator, a peristaltic pump, a master cylinder, a microfluidic pump, and a valve.

In another embodiment, each of the plurality of needles comprises a plurality of ports distributed along its length. A fluid agent within a porous tube may come into diffusional communication with a solid tissue via these ports, allowing delivery of the fluid agent to the tissue for the duration of needle insertion. This application may be useful for controlling the duration of passive delivery. In some cases, one end of a porous tube may be open, and configured such that application of pressure to this end determines the rate of delivery of fluid agent from the porous tube.

Ports may be variously configured along a needle. In certain embodiments a size of each of the plurality of ports is inversely related to a distance of the respective port from a tip-end of the needle. In certain other embodiments a distribution density of the plurality of ports is inversely related to a distance of the respective port from a tip-end of the needle. In certain other embodiments the plurality of ports is distributed in a spiral pattern along the length of the needle. In certain other embodiments the plurality of ports is arranged in pairs of ports on opposite sides of the needle, with each pair of ports rotated 90 degrees with respect to adjacent pairs of ports along the length of the needle.

A tube can be partially- or fully-submerged in the tissue. A tube may be hollow, or may uniformly comprise porous material. Deposition of tubes into the tissue can be facilitated by inserting the tube into the tissue via a needle. The tube can be further deposited into the tissue by depression of a plunger associated with the needle.

After deposition of the tube into the tissue, the tube can be broken, cut, sliced, disjoined, or separated to remove the top end of the tube from the bottom end of the tube. The bottom end remains deposited in the tissue, whereas the top end is removed from the tissue. In some embodiments, the bottom end of the tube contains a therapeutic agent and the top end of the tube does not contain a therapeutic agent. In some embodiments, both the bottom end and the top end of the tube contain a therapeutic agent.

Once a tube has been deposited into the tissue, the contents of the tube (for example, a pharmaceutical composition or a therapeutic agent) can diffuse form the tube into the tissue. The rate of diffusion can be influenced by the porosity of the tube. The contents can diffuse through the tube material into the tissue over a period of about a minute to about a month; about an hour to about a week; about 12 hours to about 72 hours; or about 24 hours to about 48 hours.

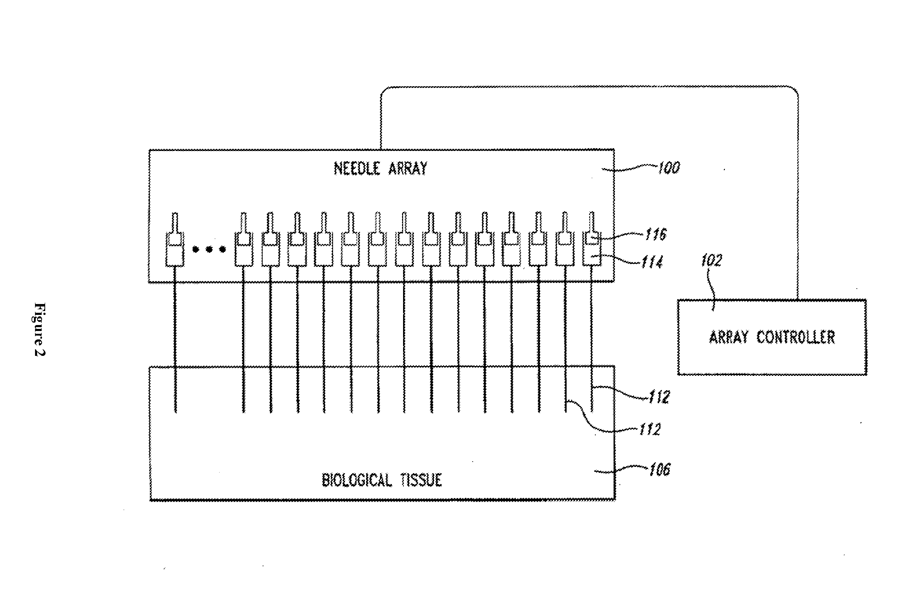

Referring to Figure 2, a needle array assembly 100 is shown, including a plurality of needles 112, a plurality of reservoirs containing porous tubes 114, a plurality of delivery actuators such as, in the present example, plungers 116, and a controller 102. Each of the plurality of needles 112 is fixed in position relative to the others of the plurality of needles, and the plungers are likewise operatively coupled so as to be fixed in position and simultaneously actuable. Each of the plurality of needles 112 is in fluid communication with a respective one of the plurality of reservoirs 114, and each of the plurality of plungers includes a first end positioned in a respective one of the plurality of reservoirs 114. The controller 102 is operatively coupled to second ends of each of the plurality of plungers 116. The controller is configured to control actuation of the plungers within the reservoir with respect to speed, distance, and direction of movement.

Each of the porous tubes within a reservoir 114 can be charged with a different agent, or some or all of the porous tubes can be charged with a common agent. Movement of the plurality of plungers 116 in a second direction creates a positive pressure, or overpressure, in the respective reservoirs 114, forcing the contents of the reservoirs out via the respective needles 112.

In this configuration, a relatively small amount of a plurality of therapeutic agents can be simultaneously inserted directly to a region of solid tissue 106 for evaluation and analysis. Following insertion, a fluid agent within a porous tube is released to the surrounding tissue by passive diffusion. In some embodiments, the amount of a therapeutic agent delivered to the tissue is less than 1 µL per needle. The evaluation of the tissue 106 and the efficacy of the different therapeutic agents delivered thereto can be used, for example, to screen potential therapeutic agents for subsequent clinical trials or to make subject-specific treatment decisions based on the relative efficacy of the therapeutic agents in the tissue 106.

According to various embodiments, any number of needles can be used. For example, as few as one, two, or three needles can be used, and according to some embodiments, more than one thousand needles can be used.

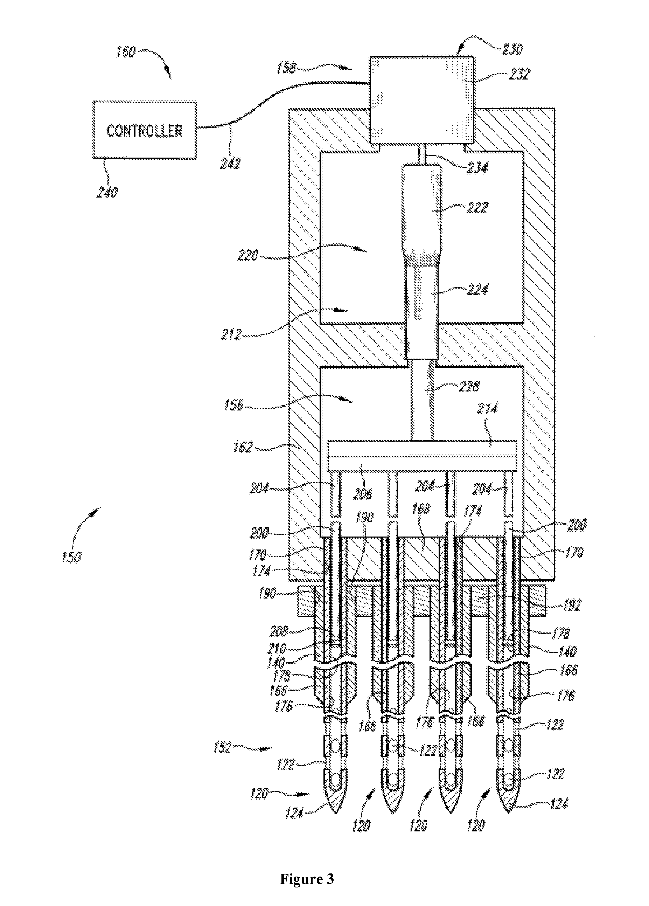

Figure 3 is a diagrammatic view of a delivery assembly 150 according to another embodiment. The delivery assembly 150 includes a needle array 152, an inserted assembly 190, an actuator assembly 156, a driver assembly 158, a control assembly 240, and a frame 162. The frame 162 provides a substantially rigid structure to which other elements of the assembly 150 are coupled.

The needle array 152 comprises a plurality of needle cylinders 166 and a needle block 168. In the embodiment shown, the needle block 168 is integral with the frame 162. Each of the plurality of needle cylinders 166 is coupled, at a first end 170, in a respective needle aperture 174 extending in the needle block 168, and comprises a lumen 176, having, in the illustrated embodiment, a nominal diameter of 0.15 mm, extending substantially the entire length of the needle cylinder 166. Each needle cylinder 166 includes a reservoir 178 in a region toward the first end 170, a needle 120 in a region toward a second end, and a tip-end 124 at the second end of the needle cylinder 166. In the embodiment shown, the tip-end 124 is tapered to a point.

Each delivery needle 120 is defined by a plurality of ports 122 distributed along its length. The length of each of the plurality of needle cylinders 166 and of the respective needles 120 varies according to the embodiment. In one embodiment, each needle cylinder 166 is longer than 15 cm, while according to other embodiments the needle cylinders are each longer than 10 cm, between 5 cm and 10 cm, and preferably greater than 2 cm, respectively. Likewise, according to various embodiments, each of the plurality of delivery needles 120, defined by the portion of the respective needle cylinder 166 along which the ports 122 are spaced, is longer than 0.1 cm, longer than 2 cm, longer than 4 cm, and longer than 8 cm.

The inserter assembly 190 comprises a plurality of inserter needles 140 coupled to an inserter block 192 in respective inserter apertures 190 extending therein in a configuration that corresponds to the arrangement of the needle cylinders 166 in the needle block 168, such that each of the plurality of needle cylinders 166 can be positioned within a respective one of the plurality of inserter needles 140 as shown in Figure 3. The inserter assembly 190 is axially slidable over the needle cylinders 166 between a first position, in which only the tip-ends 124 of each of the needle cylinders 166 extend from respective ones of the plurality of inserter needles 140, to a second position, in which the second ends of each of the needle cylinders 166 extends from the respective inserter needle 140 a distance sufficient to clear all of the ports 122 of the respective delivery needle 120.

According to an embodiment, a spacer is provided, configured to be positioned between the inserter block 192 and the needle block 168, sized such that when the inserter block and the needle block are both engaged with the spacer, the inserter block is maintained in the first position. Removal of the spacer permits movement of the inserter block 192 and the needle block 168 relative to each other, to permit placement of the inserter block into the second position, relative to the needle block.

The actuator assembly 156 comprises a plurality of plungers 200 coupled at respective first ends 204 to a plunger block 206 in a configuration that corresponds to the arrangement of the needle cylinders 166 and the inserter needles 140 such that a second end 208 of each of the plurality of plungers 200 can be positioned within the reservoir 178 of a respective one of the plurality of the needle cylinders 166 as shown. An O-ring 210 is provided at the second end 208 of each of the plurality of plungers 200 to sealingly engage the wall of the respective lumen 176. The actuator assembly 156 also comprises an actuator 212 coupled to an actuator block 214, which in turn is rigidly coupled to the plunger block 206. In the embodiment shown, the actuator 212 comprises a micrometer device 220 having a thimble 222, a barrel 224, and a spindle 228 such as are well known in the art. The barrel 224 is rigidly coupled to the frame 162 while the spindle 228 is rotatably coupled to the actuator block 214 so as to control translational movement of the actuator block relative to the frame 162. The micrometer device is calibrated in 0.01 mm increments, with a spindle travel of 0.5 mm per rotation of the thimble 222 and a maximum stroke of 15 mm. Thus, each complete rotation of the thimble moves each of the plurality of plungers 0.5 mm within the lumen 178 of the respective needle cylinder 166 and displaces about 0.0001 cm³ of volume, or 0.1 nL per revolution. Thus, given a maximum stroke of 15 mm, the maximum dispensing capacity of each of the plurality of needles 120 is about 3 nL.

The driver assembly 158 comprises a stepper motor 230 such as is well known in the art, and that includes a motor casing 232, a motor shaft 234 coupled to a rotor of the motor 230, and other elements such as are well known in the art. The motor casing 232 is rigidly coupled to the frame 162, and the motor shaft 234 is slidably coupled to the thimble 222 of the micrometer device while being rotationally locked therewith, such as via a spline coupling, for example. Accordingly, rotational force from the motor shaft 234 is transmitted to the thimble 222, while axial movement of the thimble is not limited by the motor shaft. Such couplings are well known in the mechanical arts. The stepper motor 230 of the illustrated embodiment is configured to divide each rotation into 125 steps. Thus, each incremental rotational step of the motor 230 rotates the thimble about 3°, displacing a volume of about 0.8 pL per reservoir 178.

The controller assembly 160 includes a controller 240 and a control cable 242 that extends from the controller to the stepper motor 230.

Signals for controlling direction, speed, and degree of rotation of the motor shaft 234 are transmitted from the controller 240 to the stepper motor 230 via the control cable 242 in a manner that is well known in the field to which such motors belong. According to an embodiment, the controller is programmable. A user can program the controller to control a speed of delivery from the delivery needles 120 by selecting the speed of rotation, and in some cases a volume of porous tube delivered by selecting the number of partial and complete rotations of the rotor. According to another embodiment, the controller is manually operated, such that a user controls a rate and direction of rotation of the motor 230 in real time. According to a third embodiment, the driver and controller assemblies are omitted, and a user controls delivery by manually rotating the thimble 222 of the actuator assembly 212.

Charging the reservoirs 178 can be accomplished in a number of ways. For example, a charging vessel can be provided that includes a plurality of cups or compartments in an arrangement that corresponds to the arrangement of the needle cylinders 166. The user first places a selected fluidic agent or combination of agents within a porous tube material in each of the cups. The delivery assembly 150 of Figure 3 is positioned with the needle cylinders pointing downward as shown in the drawing, and the spindle 228 of the actuator 212 fully extended. The frame 162 is lowered until the needles 120 are fully immersed in the contents of the respective cups. The motor 230 is then controlled to rotate in the reverse direction, drawing the spindle 228 inward and pulling the plungers 200 upward. This in turn creates a negative pressure in the reservoirs 178 relative to ambient, drawing the contents into the needle cylinders 166 via the needle ports 122. When the reservoirs are sufficiently charged, rotation of the rotor is halted and the needle array 152 is withdrawn from the charging vessel.

In order to deliver the charge, according to one embodiment, each of the needle cylinders 166 of the needle array 152 is positioned in a respective one of the inserter needles 140 of the inserter assembly 190 so that the tip-ends 178 of the needles 120 protrude from the inserter needles 140. The delivery assembly 190 is then positioned in axial alignment with a target tissue region of a subject and translated axially so that the tip-ends of the needles 120 penetrate the subject's skin. Axial translation of the delivery assembly 190 continues until the tip-ends 124 of the needle cylinders 166 have penetrated to within a selected distance of the target tissue region. The inserter assembly 190 is then held in position while the frame 162 and the elements coupled thereto continue to move axially, such that the needles 120 extend into the target tissue region.

When the needles 120 are correctly positioned, movement of the delivery assembly 190 is halted and the frame 162 is held in position relative to the subject. The stepper motor 230 is then controlled to rotate the thimble 222 in the forward direction so as to cause the spindle 228 to extend, driving the plungers 200 into the needle cylinders 166 and creating an overpressure in the respective reservoirs 178, thereby forcing contents from the reservoirs to the target tissue region via the ports 122 of the delivery needles 120.

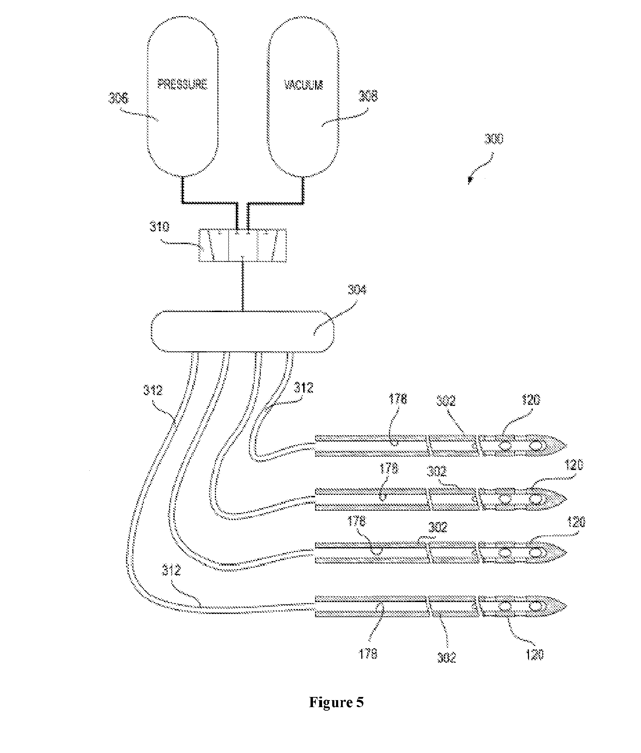

Delivery can be performed in a few seconds, or it can be extended over minutes or hours under a relatively low overpressure to promote complete absorption of the reservoir contents into the surrounding tissue. According to the embodiment described with reference to Figure 5, the stepper motor 230 can be controlled to rotate the rotor fast enough to depress the plungers 200 the full 15 mm in less than one second, or slow enough that a single rotation can take many hours.

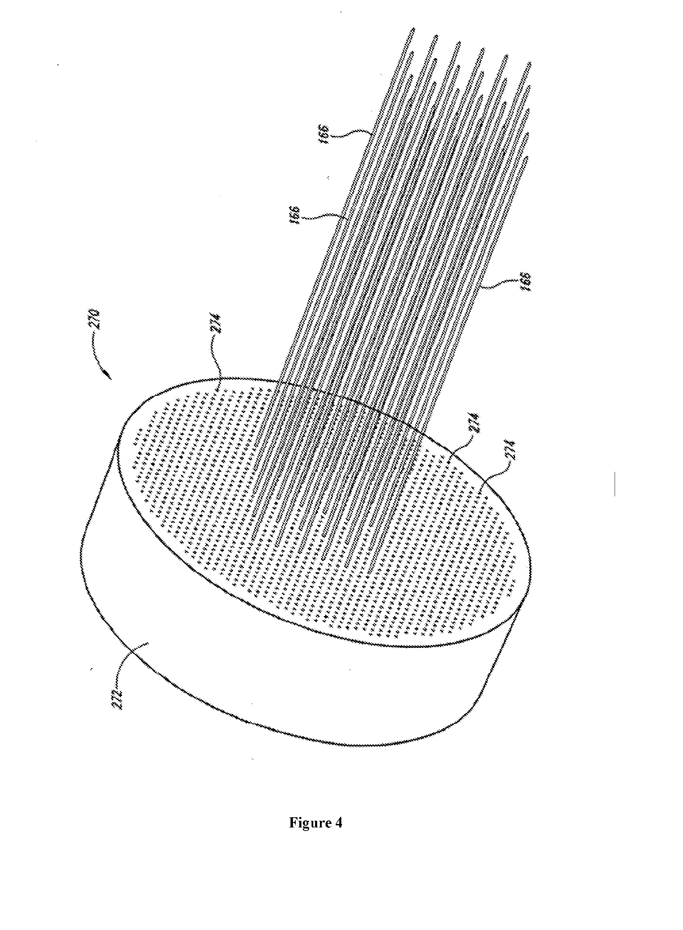

Turning now to Figure 4, elements of a delivery assembly 270 are shown according to another embodiment. A needle block 272 includes a large plurality of needle apertures 274 extending therethrough, arranged in a closely spaced array. Needle cylinders 166 are provided separately, in various assortments of lengths and numbers, sizes, and spacings of ports.

In use, a user selects a number of needles to be used for a particular procedure, and selects the particular needle cylinders 166, placing each in a respective one of the plurality of apertures 274 of the needle block, in an arrangement that is selected for the particular procedure. The user can require only a small number of needles; such as one to five, for example, or can require hundreds or thousands of needles. Furthermore, the needle cylinders 166 can be of varying lengths and configurations. The needles may be pre-loaded with fluid agents within porous tubes. The user selects the arrangement of the needle cylinders 166 in the needle block 272, and their respective lengths and configurations, at least in part according to factors such as the size, shape, and position of a target tissue region in a subject's body, the desired distribution density of fluid in the target tissue region, the permeability of the target tissue, etc.

The delivery actuators of previous embodiments have been described as plungers. However, any suitable actuator can be used to control an amount of therapeutic agent delivered from the reservoirs into the needle. For example, fluid pressure such as by compressed air or pressurized liquid can be used to control an amount of therapeutic agent delivered to a region of biological tissue via the porous tubes and needles.

Referring now to Figure 5, a delivery assembly 300 is shown, according to another embodiment. The delivery assembly 300 includes a plurality of needle cylinders 302 comprising respective reservoirs 178 and needles 120. Fluid couplings 312 place the needle cylinders 302 in fluid communication with a manifold 304. A fluid pressure source 306 and a fluid vacuum source 308 can each be placed in fluid communication with the manifold 304 by operation of a valve 310.

According to the embodiment of Figure 5, the needle cylinders 302 are not fixed with respect to each other, but can be individually emplaced, in a target tissue region, for example. The reservoirs may be charged by placing the delivery needles 120 in a selected fluid, *e.g.,* a therapeutic agent or respective therapeutic agent, and the fluid vacuum source is placed in fluid communication with the manifold, drawing a negative pressure into the reservoirs and drawing the agent into the needles. The user then positions the needles 120 in the target tissue region. When they are all in place, the manifold 304 is pressurized, forcing fluid from the reservoirs of each of the needle cylinders 166 via the ports 122 of the respective delivery needles. While Figure 5 shows a simple fluid circuit, it will be understood that in practice such a circuit could include any of valves, pressure regulator, peristaltic pump, microfluidic pump, vacuum accumulator, compressor, controller, etc., all of which are well known in the art, and within the abilities of one of ordinary skill to select and configure for a given application.

### Screening Candidate Agents

A drug-delivery device of the invention is useful for methods of administering therapeutic or candidate therapeutic agents to a subject by depositing one or more porous compositions packed with one or more therapeutic agents in the a tissue of the subject. Spatially constrained delivery of a plurality of candidate therapeutic agents permits parallel evaluation of agents for effect on a solid tissue such as a tumor.

A fluid agent within a porous composition may contain a dye, useful in monitoring the response to a therapeutic agent. A dye may be a position marker, or it may be chosen to report, for example, by fluorescence, the activation or inactivation of a biological function upon imaging. This process allows an experimenter to make a direct assessment of the affect of a therapeutic agent on a physiological system of interest.

Detection of an effect of a therapeutic or candidate therapeutic agent can be performed by assessing an alteration in a biological function of a cell or tissue. In some embodiments, the biological function is a pathway, an activity of an enzyme, an expression of a gene, transcription of the gene, translation of an RNA molecule associated with the gene, or peptide synthesis. In some embodiments, the biological function is associated with cancer, degenerative disease, inflammation, metabolism, apoptosis, or an immune response. In some embodiments, the biological function is associated with cancer. In some embodiments, the pathway is a cancer pathway. In some embodiments, the enzyme is associated with cancer. In some embodiments, the gene is associated with cancer. In some embodiments, the pathway is an apoptotic pathway, and the dye reports apoptosis.

In some embodiments, the gene is ABL1, ABL2, ACSL3, AF15Q14, AF1Q, AF3p21, AF5q31, AKAP9, AKT1, AKT2, ALK, ALO17, APC, ARHGEF12, ARHH, ARNT, ASPSCR1, ASXL1, ATF1, ATIC, ATM, BCL10, BCL11A, BCL11B, BCL2, BCL3, BCL5, BCL6, BCL7A, BCL9, BCR, BHD, BIRC3, BLM, BMPR1A, BRAF, BRCA1, BRCA2, BRD3, BRD4, BRIP1, BTG1, BUB1B, C12orf9, C15orf21, CANT1, CARD11, CARS, CBFA2T1, CBFA2T3, CBFB, CBL, CBLB, CBLC, CCND1, CCND2, CCND3, CD74, CD79A, CD79B, CDH1, CDH11, CDK4, CDK6, CDKN2A- p14ARF, CDKN2A -p16(INK4a), CDKN2C, CDX2, CEBPA, CEP1, CHCHD7, CHEK2, CHIC2, CHN1, CIC, CLTC, CLTCL1, CMKOR1, COL1A1, COPEB, COX6C, CREB1, CREB3L2, CREBBP, CRLF2, CRTC3, CTNNB1, CYLD, D10S170, DDB2, DDIT3, DDX10, DDX5, DDX6, DEK, DICER1, DUX4, EGFR, EIF4A2, ELF4, ELK4, ELKS, ELL, ELN, EML4, EP300, EPS15, ERBB2, ERCC2, ERCC3, ERCC4, ERCC5, ERG, ETV1, ETV4, ETV5, ETV6, EVI1, EWSR1, EXT1, EXT2, EZH2, FACL6, FANCA, FANCC, FANCD2, FANCE, FANCF, FANCG, FBXW7, FCGR2B, FEV, FGFR1, FGFR1OP, FGFR2, FGFR3, FH, FIP1L1, FLI1, FLT3, FNBP1, FOXL2, FOXO1A, FOXO3A, FOXP1, FSTL3, FUS, FVT1, GAS7, GALA1, GATA2, GATA3, GMPS, GNAQ, GNAS, GOLGA5, GOPC, GPC3, GPHN, GRAF, HCMOGT-1, HEAB, HEI10, HERPUD1, HIP1, HIST1H4I, HLF, HLXB9, HMGA1, HMGA2, HNRNPA2B1, HOOK3, HOXA11, HOXA13, HOXA9, HOXC11, HOXC13, HOXD11, HOXD13, HRAS, HRPT2, HSPCA, HSPCB, IDH1, IDH2, IGH@, IGK@, IGL@, IKZF1, IL2, IL21R, IL6ST, IRF4, IRTA1, ITK, JAK1, JAK2, JAK3, JAZF1, JUN, KDM5A, KDM5C, KDM6A, KDR, KIAA1549, KIT, KLK2, KRAS, KTN1, LAF4, LASP1, LCK, LCP1, LCX, LHFP, LIFR, LMO1, LMO2, LPP, LYL1, MADH4, MAF, MAFB, MALT1, MAML2, MAP2K4, MDM2, MDM4, MDS1, MDS2, MECT1, MEN1, MET, MHC2TA, MITF, MKL1, MLF1, MLH1, MLL, MLLT1, MLLT10, MLLT2, MLLT3, MLLT4, MLLT6, MLLT7, MN1, MPL, MSF, MSH2, MSH6, MSI2, MSN, MTCP1, MUC1, MUTYH, MYB, MYC, MYCL1, MYCN, MYH11, MYH9, MYST4, NACA, NBS1, NCOA1, NCOA2, NCOA4, NF1, NF2, NFIB, NFKB2, NIN, NONO, NOTCH1, NOTCH2, NPM1, NR4A3, NRAS, NSD1, NTRK1, NTRK3, NUMA1, NUP214, NUP98, NUT, OLIG2, OMD, P2RY8, PAFAH1B2, PALB2, PAX3, PAX5, PAX7, PAX8, PBX1, PCM1, PCSK7, PDE4DIP, PDGFB, PDGFRA, PDGFRB, PER1, PHOX2B, PICALM, PIK3CA, PIK3R1, PIM1, PLAG1, PML, PMS1, PMS2, PMX1, PNUTL1, POU2AF1, POU5F1, PPARG, PRCC, PRDM16, PRF1, PRKAR1A, PRO1073, PSIP2, PTCH, PTEN, PTPN11, RAB5EP, RAD51L1, RAF1, RANBP17, RAP1GDS1, RARA, RB1, RBM15, RECQL4, REL, RET, ROS1, RPL22, RPN1, RUNX1, RUNXBP2, SBDS, SDH5, SDHB, SDHC, SDHD, SEPT6, SET, SETD2, SFPQ, SFRS3, SH3GL1, SIL, SLC45A3, SMARCA4, SMARCB1, SMO, SOCS1, SRGAP3, SS18, SS18E1, SSH3BP1, SSX1, SSX2, SSX4, STK11, STL, SUFU, SUZ12, SYK, TAF15, TAL1, TAL2, TCEA1, TCF1, TCF12, TCF3, TCL1A, TCL6, TET2, TFE3, TFEB, TFG, TFPT, TFRC, THRAP3, TIF1, TLX1, TLX3 TMPRSS2, TNFAIP3, TNFRSF17, TNFRSF6, TOP1, TP53, TPM3, TPM4, TPR, TRA@, TRB@, TRD@, TRIM27, TRIM33, TRIP11, TSC1, TSC2, TSHR, TTL, USP6, VHL, WAS, WHSC1, WHSC1L1, WRN, WT1, WTX, XPA, XPC, ZNF145, ZNF198, ZNF278, ZNF331, ZNF384, ZNF521, ZNF9, mTOR, MEK, PI3K, HIF, IGF1R, GLS1, or ZNFN1A1. In some embodiments, the pathway is associated with any of the aforementioned genes. In some embodiments, the peptide of the peptide synthesis is a gene product of any of the aforementioned genes.

In some embodiments, the target tissue does not exhibit features of a disease, and a dye may be used to assess the response of an individual tissue to one or more compounds. In some cases, one or more compounds may be administered to produce an altered physiologic state within a tissue. An altered physiologic state can be any detectable parameter that directly relates to a condition, process, pathway, dynamic structure, state or other activity in a solid tissue (and in some embodiments in a solid tumor) including in a region or a biological sample that permits detection of an altered (e.g., measurably changed in a statistically significant manner relative to an appropriate control) structure or function in a biological sample from a subject or biological source. The methods of the present invention thus pertain in part to such correlation where an indicator of altered physiologic state can be, for example, a cellular or biochemical activity, including as further non-limiting examples, cell viability, cell proliferation, apoptosis, cellular resistance to anti-growth signals, cell motility, cellular expression or elaboration of connective tissue-degrading enzymes, cellular recruitment of angiogenesis, or other criteria as provided herein.

Altered physiologic state can further refer to any condition or function where any structure or activity that is directly or indirectly related to a solid tissue function has been changed in a statistically significant manner relative to a control or standard, and can have its origin in direct or indirect interactions between a solid tissue constituent and an introduced agent, or in structural or functional changes that occur as the result of interactions between intermediates that can be formed as the result of such interactions, including metabolites, catabolites, substrates, precursors, cofactors and the like. Additionally, altered physiologic state can include altered signal transduction, respiratory, metabolic, genetic, biosynthetic or other biochemical or biophysical activity in some or all cells or tissues of a subject or biological source, in some embodiments in some or all cells of a solid tissue, and in some embodiments in some or all cells of a tumor such as a solid tumor in a solid tissue. As non-limiting examples, altered biological signal transduction, cell viability, cell proliferation, apoptosis, cellular resistance to anti-growth signals, cell motility, cellular expression or elaboration of connective tissue-degrading enzymes, cellular recruitment of angiogenesis, or other criteria including induction of apoptotic pathways and formation of atypical chemical and biochemical crosslinked species within a cell, whether by enzymatic or non-enzymatic mechanisms, can all be regarded as indicative of altered physiologic state.

According to an embodiment, a solid tissue into which a plurality of therapeutic agents has been delivered is subsequently excised from the subject and evaluated. For example, in a case where the target tissue is a cancerous tumor, the plurality of agents injected therein can include some agents whose efficacy or effect on such tumors is under investigation. By injecting the various agents *in vivo* then waiting a selected period before removing the tumor, the effect of the agents on the tumor in situ can be investigated. This preserves the tumor microenvironment and distinguishes this method from current ex *vivo* or *in vitro* therapeutics evaluation methods. Over time, each agent permeates outward from its delivery axis to a greater or lesser degree, depending on factors such as, for example, the density of the surrounding tissue, the viscosity and composition of the agent, the wettability of the tissue by the respective agent, etc. Typically, the portions of the tissue into which the agents spread are approximately column-shaped regions coaxial with the respective delivery axes.

According to various embodiments, a region of tissue is left in place for some period of time before being excised. For example, a period of 48-72 hours following delivery is thought to be generally sufficient for a tumor to exhibit a detectable response. In other cases, the wait period can be hours, days, or weeks. According to some embodiments, the tissue region is imaged using known methods to precisely locate the target region of tissue prior to insertion of the needles. The region can be imaged repeatedly before and after delivery of the plurality of agents to the region of tissue.

In some embodiments, the excised tissue can be cut into a plurality of serial histological sections along parallel planes that are substantially normal (*e.g*., perpendicular or deviating from perpendicular by as much as 1, 2, 3, 4, 5, 6, 7, 8, 9, 10, 11, 12, 13, 14, 15, 20, 25, 30, 35 or more degrees) to the parallel axes, for analysis by any of a number of known histological, histochemical, immunohistological, histopathologic, microscopic (including morphometric analysis and/or three-dimensional reconstruction), cytological, biochemical, pharmacological, molecular biological, immunochemical, imaging or other analytical techniques, which techniques are known to persons skilled in the relevant art. See, *e.g.,* Bancroft and Gamble, *Theory and Practice of Histological Techniques* (6^{th} Ed.) 2007 Churchill Livingstone, Oxford, UK; Kieman, *Histological and Histochemical Methods: Theory and Practice,* 2001 Cold Spring Harbor Laboratory Press, Cold Spring Harbor, NY; and M.A. Hayat (Ed.), *Cancer Imaging - Vols. 1 and 2, 2007* Academic Press, NY, each of which is incorporated by reference herein in its entirety. Imaging can be performed before, during or after dispenser needles are inserted into the solid tissue.

According to other embodiments, a plurality of agents is delivered to a portion of tissue via respective ones of a plurality of needles of a needle array after the portion of tissue is excised.

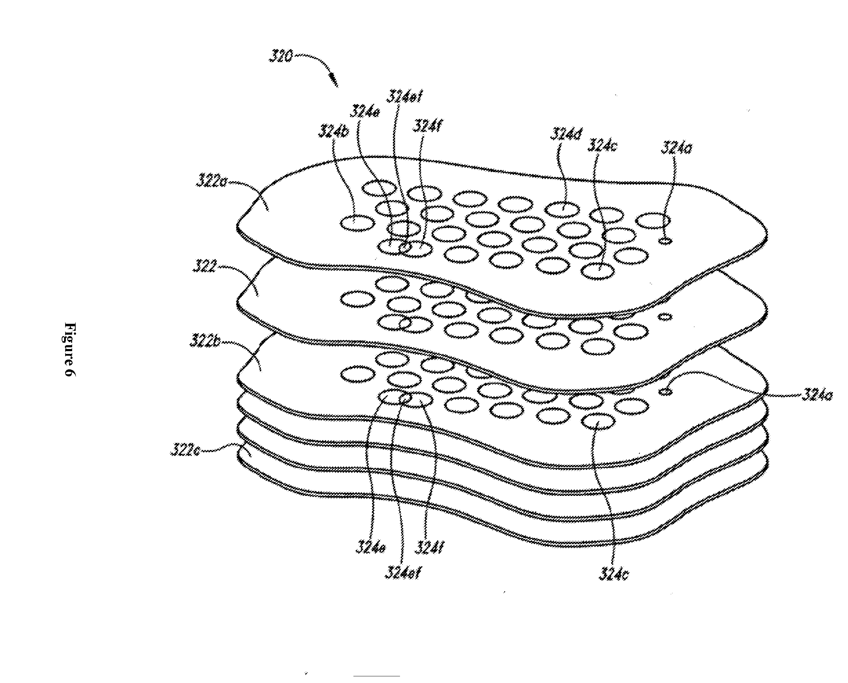

Referring now to Figure 6, a portion of a tumor 320 is shown, following an injection procedure and subsequent resection. The tumor 320 has been sectioned into a plurality of slices 322 along planes that lie substantially normal to the delivery axes. Column-shaped delivery regions 324 define the regions of permeation of the respective agents, and extend perpendicular to the planes of the sections 322

Many of the regions 324 may not be easily detectable to a user, so generally at least two readily detectable position markers 324a, 324b are among the agents injected, at widely separated locations. The user can then overlay a template on which the locations of each of the delivery axes is marked, aligning the indicated marker positions of the template with the detectable position markers 324a, 324b of a given section 322, thereby locating the remaining delivery regions 324. The position markers 324a, 324b can be any composition that is detectable by a user. Various exemplary position markers are described in detail elsewhere in this disclosure. According to an embodiment, the position markers are selected to resist permeation and diffusion into the surrounding tissue and to remain concentrated in a narrow column, as shown for example at 324a, so as to be detectable for an extended period after the injection procedure, and to provide an accurate guide for positioning the template.

In addition to position markers, control agents can also be among the agents injected. For example, a negative control can comprise a substance used as a vehicle in others of the agents, and a positive control can comprise a compound of most or all of the agents delivered individually at other delivery axes.

Following sectioning of the tumor 320, a user conducts selected assays on delivery regions 324 of various sections 322 of the tumor 320, as described in more detail later. One benefit of the devices and methods disclosed herein is that, in addition to evaluating the efficacy of a given agent on the tumor, the efficacy of agents at various delivery regions 324 can be evaluated and compared. Additionally, the effect of a given agent on various parts of the tumor can be evaluated, both vertically and horizontally. By comparing the effect of an agent in a delivery region 324c at section 322a, for example, with its effect in the same region 324c at sections 322b and 322c, the effect of that agent on different tissue compositions that can occur vertically can be differentiated. Similarly, the same agent can be delivered at several delivery axes in the array, *e.g.*, 324c and 324d, and the relative effects at those locations in a given section 322 can then be compared, providing horizontal differentiation. As is well known in the art, biological tissue is rarely homogeneous over even relatively small distances. A given agent might have substantially no effect on some tissue structures of a tumor, but might, on the other hand, be extremely effective on others. Such differential effects can be detected and evaluated as described above.

Another valuable aspect that can be evaluated is the effect of multiple agents in regions where they interact within the tissue. Delivery regions 324e and 324f are spaced more closely together than the others, resulting in the respective agents interacting in a region 324ef where the respective delivery regions overlap.

According to certain presently contemplated embodiments, the efficacy of a candidate agent can be identified by detecting an altered physiologic state as provided herein, including by assessing any of a number of biological parameters characteristic of a cancer cell such as those reviewed by Hanahan and Weinberg (2000 *Cell* 100:57) and in the references cited therein. Therein are disclosed methodologies for determining the effect of a candidate agent on one or more traits exhibited by cancer cells, and detectable by any of a variety of techniques known to the art for determining one or more of (i) an ability to evade apoptosis, (ii) acquisition of self-sufficiency in growth signals, (iii) insensitivity to growth-inhibitory signals, (iv) acquisition of tissue invasive and metastatic phenotype, (v) unlimited replicative potential, and (vi) sustained angiogenesis. Persons skilled in the art are familiar with multiple approaches for detecting the presence of these alterations of physiologic state, which can be adapted to a particular excised tumor system. See, *e.g.,* Bonificano et al. (Eds.) Current Protocols in Cell Biology, 2007 John Wiley & Sons, NY; Ausubel et al. (Eds.) Current Protocols in Molecular Biology, 2007 John Wiley & Sons, NY; Coligan et al. (Eds.), Current Protocols in Immunology, 2007 John Wiley & Sons, NY; Robinson et al. (Eds), Current Protocols in Cytometry, 2007 John Wiley & Sons, NY. Non-limiting examples of parameters that can be assayed to identify an altered physiologic state include assays of cell viability, cell division, apoptosis, necrosis, cell surface marker expression, cellular activation state, cellular elaboration of extracellular matrix (ECM) components or of ECM-degrading enzymes, morphometric analysis, extension or retraction of cellular processes, cytoskeletal reorganization, altered gene expression, e.g., *by in situ* hybridization of immunohistochemistry *(e.g.,* Shibata et al, 2002 J. Anat. 200:309) intracellular phosphoprotein localization *(e.g.,* Gavet et al., 1998 J Cell Sci 111:3333), and the like.

As described herein, determination of levels of at least one indicator of altered physiologic state can also be used to stratify a subject population for eligibility to participate in a clinical trial. These and related embodiments are contemplated as usefully providing advantages associated with evaluation of candidate therapeutic compounds at an earlier stage of development than is currently the case. For instance, it is not currently standard clinical trial practice to establish biomarker parameters (which can be the basis for exclusion of subjects) prior to Phase II studies, whereas the embodiments described herein can provide useful results even in the absence of established biomarker criteria, for example, at Phase II. Accordingly it is envisioned that through the practice of certain presently disclosed embodiments, relevant information on the properties of a candidate agent can be obtained earlier in a solid tumor oncology drug development program than has previously been the case, including in a manner which can time-efficiently and cost-effectively permit elimination from a clinical trial of subjects for whom no response or benefit can be expected based on a nonresponder result for a particular candidate agent.

For example, stratification of a subject population according to levels of at least one indicator of altered physiologic state, determined as described herein, can provide a useful marker with which to correlate the efficacy of any candidate therapeutic agent being used in cancer subjects, and/or to classify subjects as responders, nonresponders or possible responders.

In some embodiments, the method is useful in drug screening and drug discovery, such as in preclinical animal models to identify and functionally characterize potential new therapeutics. For instance, a plurality of siRNAs can be administered intratumorally and their relative abilities to knock down expression of a desired target gene can be compared. Other similar embodiments can find uses in clinical contexts, for example, to "deselect", or eliminate from consideration, known therapeutic agents that have no effect in a particular tumor, thereby advantageously advancing the therapeutic management of a subject by avoiding the loss of time and the undesirable side-effects that can be associated with administering an ineffectual treatment regimen.

Some embodiments include those in which the solid tissue comprises a tumor, wherein agent delivery can be made to, and/or sample retrieval can be made from, the solid tumor. It will be appreciated by persons familiar with the art from the disclosure herein that in the course of practicing certain embodiments described herein, a selected region of a tumor can comprise the site into which the needles of the presently described devices are inserted, introduced or otherwise contacted with the tumor. The region can be selected on any number of bases, including based on imaging that can be conducted before, during or after a step of needle insertion, introduction or contacting, or based on imaging conducted before, during or after excising the solid tissue from a subject, or based on other criteria including but not limited to anatomic location, accessibility in the course of a surgical procedure, degree of vascularization or other criteria.

### Generation of Reporter Cell Lines

The present disclosure provides for a method of generating a reporter cell line wherein a cell emits a detectable signal indicating modulation of a signaling pathway. In some embodiments, a reporter cell line comprises a cancer cell line and an integrated reporter that indicates modulation of an oncogenic pathway. In some embodiments, a reporter cell line is generated using a gene trap exhibiting luciferase-based activity. The reporter can report inhibition of an oncogenic pathway. In some embodiments, the present disclosure involves the use of reporter cell lines and reporter tissues to provide a detectable signal in response to modulation of a signaling pathway. A selection process can be employed to produce gene trap-derived luciferase-based activity state reporters. The resulting reporter cells generate a robust positive signal (e.g. emission of electromagnetic radiation) in response to inhibition of specific oncogenic pathways. It is a well-established observation that the activation status of cancer pathways is reflected as transcriptional output, as specific sets of genes are upregulated and downregulated in oncogene active tumors compared to normal tissues. Gene traps as described herein reflect native tumor biology by introducing reporters into endogenous genes where natural promoters, enhancers, insulators and micro-RNAs regulate expression. When combined with appropriate drug selection strategies, gene trap vectors enable rapid isolation of tumor cells that express the trapped reporter following integration into the most responsive endogenous genes for each pathway inhibitor of interest. Thus one vector is adaptable to any tumor cell type harboring any activated oncogenic pathway of interest.

In some embodiments, a detectable signal comprises a fluorescent protein, non-limiting examples of which include GFP, BFP, CFP, YFP, EGFP, EYFP, Venus, Citrine, phiYFP, copGFP CGFP, ECFP, Cerulean, CyPet, T-Sapphire, Emerald, YPet, AcGFP1, AmCyan, AsRed2, dsRed, dsRed2, dsRed-Express, EBFP, HcRed, ZsGreen, ZsYellow, J-Red, TurboGFP, Kusabira Orange, Midoriishi Cyan, mOrange, DsRed-monomer, mStrawberry, mRFP1, tdTomato, mCherry, mPlum, and mRaspberry.

In some embodiments, a detectable signal comprises a detectable product of an enzyme reaction. Non-limiting examples of enzymes for which reaction products are detectable by methods known in the art include peroxidase, alkaline phosphatase, galactosidase, luciferase, and lactamase.

In some embodiments, a reporter cell line comprises a cancer cell line. Non-limiting examples of cancer cell lines include cell lines derived from prostate cancer, breast cancer, colon cancer, lung cancer, brain cancer, and ovarian cancer. In certain embodiments, the cancer cell line derives from a cancer selected from adenoma, adenocarcinoma, squamous cell carcinoma, basal cell carcinoma, small cell carcinoma, large cell undifferentiated carcinoma, chondrosarcoma and fibrosarcoma. Breast, lung, and colon tumor lines known to harbor either activation mutations of the EGF receptor (e.g. HCC827 lung carcinoma), RAS (e.g. A549 lung carcinoma, HCT-116 colon carcinoma), or PI3K (HCT-116 colon carcinoma, MCF7 breast carcinoma, T47D breast carcinoma), or deactivating mutations of the tumor suppressor PTEN (MDA-MB-468 breast carcinoma, EVSA-T breast carcinoma) can be used.

A non-comprehensive list of commonly used cell lines that can be employed in the present method includes: 293-T, 3T3 cells, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepa1c1c7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A/ PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR.

A reporter can be introduced into a cell line to produce a reporter cell line. A reporter refers to a biological element that generates a detectable signal upon activation. In some embodiments, tumor cell lines are transduced with an optimized gene trap vector and subjected to a selection process to yield cancer pathway specific reporter cells. For example, a reporter cell line can detect EGF, PI3K-AKT, or RAS pathway inhibition. Some embodiments involve an apromoterless trapping cassette comprising a splice acceptor and splice donor sequence flanking genes encoding a reporter and a selectable marker (e.g. Neo). When integrated into a gene, expression of the reporter is regulated by endogenous promoters, enhancers, and insulators. Accordingly, reporter activity accurately indexes expression of the endogenous gene.

A reporter can generate a signal that is directly detectable (e.g. a fluorescent protein) or indirectly detectable (e.g., an enzyme). Detection includes, but is not limited to, radiation-counting, visual observation, measurement with a spectrophotometer, fluorescence excitation followed by visualization. Numerous detectable moieties are known by those of skill in the art and include, but are not limited to, particles, fluorophores, haptens, enzymes and their colorimetric, fluorogenic and chemiluminescent substrates and other labels that are described in RICHARD P. HAUGLAND, MOLECULAR PROBES HANDBOOK OF FLUORESCENT PROBES AND RESEARCH PRODUCTS 10th edition, CD-ROM, September 2005).

Reporter molecules include, without limitation, a chromophore, a fluorophore, a fluorescent protein, a phosphorescent dye, a tandem dye, a particle, a hapten, an enzyme and a radioisotope. Non-limiting examples of fluorescent proteins include green fluorescent protein (GFP) and the phycobiliproteins and the derivatives thereof. Enzymes and their appropriate substrates that produce chemiluminescence can also be used in reporter molecules described herein. Such enzymes include, but are not limited to, natural and recombinant forms of luciferases and aequorins (e.g. firefly luciferase, Renilla luciferase, Gaussia luciferase). In addition, the chemiluminescence-producing substrates for phosphatases, glycosidases and oxidases such as those containing stable dioxetanes, luminol, isoluminol and acridinium esters an also be used in reporter molecules described herein.

In some embodiments, Gaussia luciferase is used. The Gaussia luciferase can be engineered to be humanized, membrane-anchored, and extracellularly displayed. The use of luciferase permits real-time visualization of reporter activity in live animals in the presence of a luciferase substrate such as coelenterazine.

Cell surface expression of a reporter facilitates detection and is also useful for in vitro selection. Trapping events that occur within genes that are upregulated by pathway inhibition result in luciferase positive cells, which can be isolated based on their fluorescence using methods such as flow assisted cell sorting (FACS).

The present disclosure provides selection-based methods for generating a reporter cell line. In some embodiments, selection is performed using sub-lethal concentrations of a known pathway inhibitor. Gefitinib (Iressa), MK-2206, and PD325901, can be used to select EGF, PI3K-AKT, and Ras pathway inhibitor responsive reporters respectively.

In some embodiments, gene traps responding to off-target activity of the agent used in the selection process, unrelated to inhibition of the oncogenic pathway of interest, are removed by a counter-screening step using a second inhibitor of the oncogenic pathway of interest, structurally unrelated to the inhibitor used in the initial selection step. Cells that retain inducible expression of a reporter in response to a second inhibitor can be cloned and expanded for further analysis. In some embodiments, identification of the trapped gene is achieved by PCR-based methods known in the art.

In some embodiments, gene traps are generated in vivo using a selection strategy wherein cells are xenotransplanted to an animal model following introduction of a reporter construct. Following xenotransplantation, cancer cells can form a tumor in an animal model, and this tumor can serve as the basis for testing in vivo response of cells to pathway inhibition. Those cells that respond to one or more known pathway inhibitors in the in vivo tumor context can then be isolated (for example, by FACS) and developed for use as a reporter cell line.

Reporter cell lines can be generated to indicate both pathway "on" and "off" status. In some embodiments, a first reporter cell line is generated that produces a detectable signal in reponse to modulation of a signaling pathway, and a second reporter cell line is produced from the first reporter cell line, wherein the second reporter cell line generates a second detectable signal upon modulation of said signaling pathway. In some embodiments, the first detectable signal indicates activation of the signaling pathway, and the second detectable signal indicates inhibition of the signaling pathway.

### Tissue Models

In some embodiments, the present disclosure exemplifies a system for screening candidate therapeutic agents in a reporter tissue. A reporter tissue can be generated within an animal model using a reporter cell line. In some embodiments, the animal model is one of a mouse model or rat model. The animal model can be the recipient of a xenograft or xenotransplantation, terms that are used interchangeably to refer to the transplantation of living cells, tissues or organs from one species to another. In some embodiments, the reporter tissue is a tumor generated from xenotransplantation of one or more reporter cells to an immunocompromised animal, such as a SCID mouse or nude mouse. An athymic nude mouse is a laboratory mouse from a strain with a genetic mutation that causes a deteriorated or absent thymus, resulting in an inhibited immune system due to a greatly reduced number of T cells. An immunocompromised state in a preclinical model can be the result of genetic abnormalities, or it can be the result of drug treatments to suppress immune system function.

Tumor cell transplantation in a model can be performed at various sites (subcutaneous, intra-abdominal, mammary fat pads, intrahepatic, intrapulmonary, intracranial, and intracardiac) to simulate primary and metastatic tumor localization. Transplantation of tumor spheroids can also be performed. A xenografted tumor is obtained by transplantation of tumor into said mammal, said tumor being selected from the group consisting of a fresh surgical specimen, a tumor cell line, a cell from a solid tumor, a spheroid of cancerous cells, and a tumor piece obtained from a tumor passaged in a host animal.

Immunosuppressive drugs or immunosuppressive agents are drugs that inhibit or prevent activity of the immune system. Non-limiting examples of immunosuppressive drugs include glucocorticoids; cytostatics; alkylating agents; antimetabolites including folic acid analogues, such as methotrexate, and purine analogues such as azathioprine and mercaptopurine; azathioprine and mercaptopurine; cytotoxic antibiotics, including dactinomycin, anthracyclines, mitomycin C, bleomycin, and mithramycin; polyclonal and monoclonal antibodies targeting elements of the immune system; and drugs acting on immunophilins, including cyclosporin, tacrolimus, voclosporin and other calcineurin inhibitors, and sirolimus; interferons, opioids, TNF-binding proteins, mycophenolate, and fingolimod.

A reporter tissue can comprise a solid tissue which does or does not exhibit features of a disease or disorder. A reporter tissue can be an artificial tissue engineered on a tissue scaffold.

A reporter tissue can comprise any solid tissue. Solid tissues are well known to the medical arts and may include any cohesive, spatially discrete non-fluid defined anatomic compartment that is substantially the product of multicellular, intercellular, tissue and/or organ architecture, such as a three-dimensionally defined compartment that can comprise or derive its structural integrity from associated connective tissue and can be separated from other body areas by a thin membrane (e.g., meningeal membrane, pericardial membrane, pleural membrane, mucosal membrane, basement membrane, omentum, organ-encapsulating membrane, or the like). Non-limiting exemplary solid tissues include brain, liver, lung, kidney, prostate, ovary, spleen, lymph node (including tonsil), thyroid, pancreas, heart, skeletal muscle, intestine, larynx, esophagus and stomach. Anatomical locations, morphological properties, histological characterization, and invasive and/or non-invasive access to these and other solid tissues are all well known to those familiar with the relevant arts. In some embodiments, the tissue is, or is suspected of being, cancerous, inflamed, infected, atrophied, numb, in seizure, or coagulated. In some embodiments, the tissue is, or is suspected of being, cancerous. In some embodiments, the tissue is cancerous.

Certain embodiments contemplate a biological source of tissue that is a human subject such as a patient that has been diagnosed as having or being at risk for developing or acquiring cancer according to art-accepted clinical diagnostic criteria, such as those of the U.S. National Cancer Institute (Bethesda, MD, USA) or as described in DeVita, Hellman, and Rosenberg's Cancer: Principles and Practice of Oncology (2008, Lippincott, Williams and Wilkins, Philadelphia/ Ovid, New York); Pizzo and Poplack, Principles and Practice of Pediatric Oncology (Fourth edition, 2001, Lippincott, Williams and Wilkins, Philadelphia/ Ovid, New York); and Vogelstein and Kinzler, The Genetic Basis of Human Cancer (Second edition, 2002, McGraw Hill Professional, New York); certain embodiments contemplate a human subject that is known to be free of a risk for having, developing or acquiring cancer by such criteria.

Biological samples can be provided by obtaining a blood sample, biopsy specimen, tissue explant, organ culture, biological fluid or any other tissue or cell preparation from a subject or a biological source.

Certain other embodiments contemplate a non-human recipient for tissue transplantation, for example a non-human primate such as a macaque, chimpanzee, gorilla, vervet, orangutan, baboon or other non-human primate, including such non-human subjects that can be known to the art as preclinical models, including preclinical models for solid tumors and/or other cancers. Certain other embodiments contemplate a non-human subject that is a mammal, for example, a mouse, rat, rabbit, pig, sheep, horse, bovine, goat, gerbil, hamster, guinea pig or other mammal; many such mammals can be subjects that are known to the art as preclinical models for certain diseases or disorders, including solid tumors and/or other cancers (e.g., Talmadge et al., 2007 Am. J. Pathol. 170:793; Kerbel, 2003 Canc. Biol. Therap. 2(4 Suppl 1):S134; Man et al., 2007 Canc. Met. Rev. 26:737; Cespedes et al., 2006 Clin. TransL Oncol. 8:318). The range of embodiments is not intended to be so limited, however, such that there are also contemplated other embodiments in which the subject or biological source can be a non-mammalian vertebrate, for example, another higher vertebrate, or an avian, amphibian or reptilian species, or another subject or biological source. A transgenic animal is a non-human animal in which one or more of the cells of the animal includes a nucleic acid that is non-endogenous (i.e., heterologous) and is present as an extrachromosomal element in a portion of its cell or stably integrated into its germ line DNA (i.e., in the genomic sequence of most or all of its cells). In certain embodiments of the present invention, a transgenic animal can be targeted.

### Screening of Agents

In some embodiments, a reporter tissue is used in conjunction with a needle array device to acquire efficacy data for different candidate agents derived from in vivo models of cancer. This strategy bypasses the need to genetically engineer mice for in vivo testing. To account for tumor heterogeneity, the needle track penetrates deep into the tissue to ensure that multiple regions of the tumor are sampled. This approach guards against local false positive or negative results. Tissues can be harvested following candidate agent delivery and optionally sectioned into slices making it is possible to confirm target engagement, and assess tumor proliferation, and apoptosis using standard histology and immunohistochemistry. Successful coupling of the porous needle array with high fidelity, high sensitivity gene trap derived oncogene pathway reporters provides an in vivo validation platform.

In some embodiments, a needle array drug-delivery device is useful in administering one or more candidate therapeutic agents in parallel to a reporter tissue model. The reporter tissue model can be a xenografted tumor, derived from a reporter cell line. Spatially constrained delivery of a plurality of candidate therapeutic agents to a tumor from a needle array device can be achieved by delivering an agent within a hydrogel that reduces diffusion of the agent within a tissue.

In some embodiments, the present disclosure provides for a method for screening candidate therapeutic agents using reporter tissue, comprising the steps of a) producing a reporter cell line that generates a detectable signal upon modulation of a pathway; b) constructing a reporter tissue from said reporter cell line; and c) assessing the efficacy of a plurality of candidate therapeutic agents on said reporter tissue by delivering said plurality of candidate therapeutic agents to said reporter tissue using a needle array, and detecting said detectable signal.

In some embodiments, the present disclosure provides for a method for determining the genetic basis of an in vivo response to a drug, comprising the steps of a) producing a reporter cell line that generates a detectable signal upon modulation of a pathway; b) constructing a reporter tissue from said reporter cell line; c) delivering a drug to said reporter tissue; d) isolating a population of cells within said reporter tissue that exhibit said detectable signal in response to said drug; and e) sequencing genomic DNA of said population of cells to determine the genetic basis of the drug response.

In some embodiments, a xenografted tumor is grown from a reporter cell line until it reaches a size compatible with injection by a porous needle array device. The xenografted tumor can be grown to about 0.5, 0.6, 0.7, 0.8, 0.9, 1.0, 1.2, 1.4, 1.5, 1.6, 1.7, or 2.0 cm³. The tumor can then be injected at geometrically defined positions using a needle array. A tumor can be resected, fixed, and sectioned following a selected period of time after injection, which can be greater than 1, 2, 3, 4, 5, 10, 15, 24, 36, or 48 hours, or greater than one week.

Detection of an effect of a therapeutic or candidate therapeutic agent can be performed by assessing one or more detectable signals produced by a reporter cell line and tissue models derived from such cell lines. In some embodiments, the one or more detectable signals indicate the modulation of a biological pathway. In some embodiments, the modulation comprises activation. In some embodiments, the modulation comprises inhibition. In some embodiments, the biological pathway is a cancer-associated or oncogenic pathway.

In some embodiments, a detectable signal reports activation or inhibition of a gene, which can be a gene selected from the group consisting of ABL1, ABL2, ACSL3, AF15Q14, AF1Q, AF3p21, AF5q31, AKAP9, AKT1, AKT2, ALK, ALO17, APC, ARHGEF12, ARHH, ARNT, ASPSCR1, ASXL1, ATF1, ATIC, ATM, BCL10, BCL11A, BCL11B, BCL2, BCL3, BCL5, BCL6, BCL7A, BCL9, BCR, BHD, BIRC3, BLM, BMPR1A, BRAF, BRCA1, BRCA2, BRD3, BRD4, BRIP1, BTG1, BUB1B, C12orf9, C15orf21, CANT1, CARD11, CARS, CBFA2T1, CBFA2T3, CBFB, CBL, CBLB, CBLC, CCND1, CCND2, CCND3, CD74, CD79A, CD79B, CDH1, CDH11, CDK4, CDK6, CDKN2A- p14ARF, CDKN2A -p16(INK4a), CDKN2C, CDX2, CEBPA, CEP1, CHCHD7, CHEK2, CHIC2, CHN1, CIC, CLTC, CLTCL1, CMKOR1, COL1A1, COPEB, COX6C, CREB1, CREB3L2, CREBBP, CRLF2, CRTC3, CTNNB1, CYLD, D10S170, DDB2, DDIT3, DDX10, DDX5, DDX6, DEK, DICER1, DUX4, EGFR, EIF4A2, ELF4, ELK4, ELKS, ELL, ELN, EML4, EP300, EPS15, ERBB2, ERCC2, ERCC3, ERCC4, ERCC5, ERG, ETV1, ETV4, ETV5, ETV6, EVI1, EWSR1, EXT1, EXT2, EZH2, FACL6, FANCA, FANCC, FANCD2, FANCE, FANCF, FANCG, FBXW7, FCGR2B, FEV, FGFR1, FGFR1OP, FGFR2, FGFR3, FH, FIP1L1, FLI1, FLT3, FNBP1, FOXL2, FOXO1A, FOXO3A, FOXP1, FSTL3, FUS, FVT1, GAS7, GATA1, GATA2, GATA3, GMPS, GNAQ, GNAS, GOLGA5, GOPC, GPC3, GPHN, GRAF, HCMOGT-1, HEAB, HEI10, HERPUD1, HIP1, HIST1H4I, HLF, HLXB9, HMGA1, HMGA2, HNRNPA2B1, HOOK3, HOXA11, HOXA13, HOXA9, HOXC11, HOXC13, HOXD11, HOXD13, HRAS, HRPT2, HSPCA, HSPCB, IDH1, IDH2, IGH@, IGK@, IGL@, IKZF1, IL2, IL21R, IL6ST, IRF4, IRTA1, ITK, JAK1, JAK2, JAK3, JAZF1, JUN, KDM5A, KDM5C, KDM6A, KDR, KIAA1549, KIT, KLK2, KRAS, KTN1, LAF4, LASP1, LCK, LCP1, LCX, LHFP, LIFR, LMO1, LMO2, LPP, LYL1, MADH4, MAF, MAFB, MALT1, MAML2, MAP2K4, MDM2, MDM4, MDS1, MDS2, MECT1, MEN1, MET, MHC2TA, MITF, MKL1, MLF1, MLH1, MLL, MLLT1, MLLT10, MLLT2, MLLT3, MLLT4, MLLT6, MLLT7, MN1, MPL, MSF, MSH2, MSH6, MSI2, MSN, MTCP1, MUC1, MUTYH, MYB, MYC, MYCL1, MYCN, MYH11, MYH9, MYST4, NACA, NBS1, NCOA1, NCOA2, NCOA4, NF1, NF2, NFIB, NFKB2, NIN, NONO, NOTCH1, NOTCH2, NPM1, NR4A3, NRAS, NSD1, NTRK1, NTRK3, NUMA1, NUP214, NUP98, NUT, OLIG2, OMD, P2RY8, PAFAH1B2, PALB2, PAX3, PAX5, PAX7, PAX8, PBX1, PCM1, PCSK7, PDE4DIP, PDGFB, PDGFRA, PDGFRB, PER1, PHOX2B, PICALM, PIK3CA, PIK3R1, PIM1, PLAG1, PML, PMS1, PMS2, PMX1, PNUTL1, POU2AF1, POU5F1, PPARG, PRCC, PRDM16, PRF1, PRKAR1A, PRO1073, PSIP2, PTCH, PTEN, PTPN11, RAB5EP, RAD51L1, RAF1, RANBP17, RAP1GDS1, RARA, RB1, RBM15, RECQL4, REL, RET, ROS1, RPL22, RPN1, RUNX1, RUNXBP2, SBDS, SDH5, SDHB, SDHC, SDHD, SEPT6, SET, SETD2, SFPQ, SFRS3, SH3GL1, SIL, SLC45A3, SMARCA4, SMARCB1, SMO, SOCS1, SRGAP3, SS18, SS18L1, SSH3BP1, SSX1, SSX2, SSX4, STK11, STL, SUFU, SUZ12, SYK, TAF15, TAL1, TAL2, TCEA1, TCF1, TCF12, TCF3, TCL1A, TCL6, TET2, TFE3, TFEB, TFG, TFPT, TFRC, THRAP3, TIF1, TLX1, TLX3 TMPRSS2, TNFAIP3, TNFRSF17, TNFRSF6, TOP1, TP53, TPM3, TPM4, TPR, TRA@, TRB@, TRD@, TRIM27, TRIM33, TRIP11, TSC1, TSC2, TSHR, TTL, USP6, VHL, WAS, WHSC1, WHSC1L1, WRN, WT1, WTX, XPA, XPC, ZNF145, ZNF198, ZNF278, ZNF331, ZNF384, ZNF521, ZNF9, mTOR, MEK, PI3K, HIF, IGF1R, GLS1, or ZNFN1A1. In some embodiments, a pathway is associated with any of the aforementioned genes. In some embodiments, a detectable signal reports on a product of a gene, such as a polypeptide or protein.

According to an embodiment, a tissue model is used to evaluate efficacy of a therapeutic agent in treating cancer. In some embodiments, a solid tissue into which a plurality of therapeutic agents has been delivered is subsequently excised from the subject and evaluated. For example, in a case where the target tissue is a cancerous tumor, the plurality of agents injected therein can include some agents whose efficacy or effect on such tumors is under investigation. By injecting the various *agents in vivo* then waiting a selected period before removing the tumor, the effect of the agents on the tumor in situ can be investigated by assessing the signal emitted by reporter cells within the reporter tissue. The present disclosure provides for methods of screening within a native tumor microenvironment, distinguishing it from current *ex vivo* or *in vilro* therapeutics evaluation methods. Over time, each agent permeates outward from its delivery axis to a greater or lesser degree, depending on factors such as, for example, the density of the surrounding tissue, the viscosity and composition of the agent, the wettability of the tissue by the respective agent, etc. Typically, the portions of the tissue into which the agents spread are approximately column-shaped regions coaxial with the respective delivery axes.

According to various embodiments, a region of tissue is left in place for some period of time before being excised. For example, a period of 48-72 hours following delivery is thought to be generally sufficient for a tumor to exhibit a detectable response. The wait period can be hours, days, or weeks. According to some embodiments, the tissue region is imaged using known methods to precisely locate the target region of tissue prior to insertion of the needles. The region can be imaged repeatedly before and after delivery of the plurality of agents to the region of tissue.

In some embodiments, the excised tissue is cut into a plurality of serial histological sections along parallel planes that are substantially normal (e.g., perpendicular or deviating from perpendicular by as much as 1, 2, 3, 4, 5, 6, 7, 8, 9, 10, 11, 12, 13, 14, 15, 20, 25, 30, 35 or more degrees) to the parallel axes, for analysis by any of a number of known histological, histochemical, immunohistological, histopathologic, microscopic (including morphometric analysis and/or three-dimensional reconstruction), cytological, biochemical, pharmacological, molecular biological, immunochemical, imaging or other analytical techniques, which techniques are known to persons skilled in the relevant art. See, *e.g.,* Bancroft and Gamble, Theory and Practice of Histological Techniques (6th Ed.) 2007 Churchill Livingstone, Oxford, UK; Kieman, Histological and Histochemical Methods: Theory and Practice, 2001 Cold Spring Harbor Laboratory Press, Cold Spring Harbor, NY; and M.A. Hayat (Ed.), Cancer Imaging - Vols. 1 and 2, 2007 Academic Press, NY, each of which is incorporated by reference herein in its entirety. Imaging can be performed before, during or after dispenser needles are inserted into the solid tissue. In some embodiments, imaging comprises detection of a detectable signal generated by a reporter cell line in response to modulation of a pathway.

As described herein, determination of levels of at least one indicator of altered physiologic state can also be used to stratify a subject population for eligibility to participate in a clinical trial. These and related embodiments are contemplated as usefully providing advantages associated with evaluation of candidate therapeutic compounds at an earlier stage of development than is currently the case. For instance, it is not currently standard clinical trial practice to establish biomarker parameters (which can be the basis for exclusion of subjects) prior to Phase II studies, whereas the embodiments described herein can provide useful results even in the absence of established biomarker criteria, for example, at Phase I or II. Accordingly it is envisioned that through the practice of certain presently disclosed embodiments, relevant information on the properties of a candidate agent can be obtained earlier in a solid tumor oncology drug development program than has previously been the case, including in a manner which can time-efficiently and cost-effectively permit elimination from a clinical trial of subjects for whom no response or benefit can be expected based on a nonresponder result for a particular candidate agent.

For example, stratification of a subject population according to levels of at least one indicator of altered physiologic state, determined as described herein, can provide a useful marker with which to correlate the efficacy of any candidate therapeutic agent being used in cancer subjects, and/or to classify subjects as responders, nonresponders or possible responders.

In some embodiments, the method is useful in drug screening and drug discovery, such as in preclinical animal models to identify and functionally characterize potential new therapeutics. For instance, a plurality of siRNAs can be administered intratumorally and their relative abilities to knock down expression of a desired target gene can be compared. Other similar embodiments can find uses in clinical contexts, for example, to "deselect", or eliminate from consideration, known therapeutic agents that have no effect in a particular tumor, thereby advantageously advancing the therapeutic management of a subject by avoiding the loss of time and the undesirable side-effects that can be associated with administering an ineffectual treatment regimen.

In some embodiments, permeation or penetration refers to the area of retention of an agent in the solid tissue in the immediate vicinity of the needle from which the agent was introduced exclusive of perfusion (entry into and dispersion via any blood vessel), and can include retention of the agent in extracellular space or extracellular matrix or in association with a cell membrane or intracellularly. Permeation can be distinct from a physicochemical effect, which refers to microscopically detectable mechanical disruption of tissue that results from the needle insertion or fluid injection itself, or from non-biological mechanical or chemical tissue disruption caused by the agent (e.g., damage to cell membranes or disintegration of cell-cell junctions). Pharmacological effects include statistically significant alterations of a cell or tissue physiological state that are detectable as consequences of the molecular mechanism of action of the agent, for example, cytoskeletal reorganization, extension or withdrawal of cellular processes, or evidence of biological signal transduction as can be detected using any of a number of known cytological, biochemical, molecular biological or other read-outs. Comparison of serial sections can permit distinguishing the nature of the effect that is detected histologically.

A strategy for pooling and deconvolution of candidate therapeutic agents is depicted in Figure 6. In some embodiments, primary screens are conducted using pooled sets of reagents injected into spatially restricted tumor subportions. Pools resulting in positive reporter signal are deconvoluted and the individual components are rescreened to identify the agent responsible for pathway inhibition.

### Determining pathway modulation

In some embodiments it is contemplated that the target region in a solid tissue can be imaged using known techniques to evaluate the effects of the agents. The imaging can be by any suitable process or method, including, for example, radiographic imaging, magnetic resonance imaging, positron emission tomogoraphy, biophotonic imaging, etc. In some embodiments, the target region can be imaged repeatedly before, during, and after the delivery process. In some embodiments, the imaging occurs before, during, and/or after excision of a tissue model (derived from a reporter cell line) from a subject.

Upon imaging, the level of the reporting signal can be quantified by methods known to one of skill in the art. Observation and/or quantification of the reporting signal can be used to make informed research and health care decisions regarding the use and efficacy of a therapeutic agent. Non-limiting examples of decisions that can be made on such observations include fluid volume quality control, positional tracking, and drug biodistribution. Such experiments can be performed on a lower mammal, for example, a mouse, to provide reporting signals that can be used to make informed predictions regarding the activity of a potential therapeutic agent in a human. Animal studies of this type can be used to avoid the inherent uncertainty and inaccuracies that arise by conducting drug efficacy studies in cells in controlled environments instead of in the native environment.

Quantification of fluorescence signals can be accomplished by any method known in the art. Fluorescence signals can be compared with a standard or a control to determine up-regulation or down-regulation of a biological pathway. Such observations can be used to make predictions regarding the therapeutic value of drug candidates.

### Determining therapy resistance

In some embodiments, the present disclosure provides for a method of determining a response to an agent within a solid tissue, comprising the steps of a) producing a reporter cell line that generates a detectable signal upon modulation of a signaling pathway;b) obtaining a solid tissue derived from said reporter cell line; c) delivering a plurality of therapeutic agents to said solid tissue; and d) detecting said detectable signal to determine a response to one or more of said therapeutic agents. In some embodiments, the method further comprises the step of isolating cells that previously generated said detectable signal in response to said one or more therapeutic agents, but no longer generate said detectable signal. In some embodiments, a reporter cell line generates two detectable signals to indicate two different statuses (e.g. "on" or "off") of a signaling pathway, and the method further comprises the step of isolating cells that previously generated the second detectable signal in response to said one or more therapeutic agents, but now generates the first detectable signal in response to said one or more therapeutic agents. The isolated cells can be subjected to genomic DNA sequencing to determine the genetic basis of the change in response to one or more of the therapeutic agents.

In some embodiments, the present disclosure provides for a method for determining resistance to a cancer treatment, comprising the steps of a) producing a reporter cell line that generates a detectable signal upon modulation of a pathway; b) constructing a reporter tissue from said reporter cell line; c) administering a plurality of cancer treatments to said reporter tissue; d) isolating a population of cells within said reporter tissue that exhibit said detectable signal in response to a selected one of the plurality of cancer treatments; e) constructing a second reporter tissue from said isolated population of cells; f) administering said selected cancer treatment to the second reporter tissue; g) isolating a second population of cells that ceases to exhibit said detectable signal in response to the selected cancer treatment; and h) sequencing genomic DNA from the second population of cells, thereby determining resistance to the selected cancer treatment. In some embodiments, the present disclosure involves a method for determining resistance to cancer treatment, comprising the steps of: producing a reporter cell line that generates a detectable signal upon modulation of a pathway; constructing a reporter tissue from said reporter cell line; delivering a plurality of therapeutic agents to said reporter tissue; and isolating a population of cells within said reporter tissue that exhibit said detectable signal. This modulation can comprise activation or inhibition. The isolating can comprise cell sorting. The method can further comprise performing whole-genome sequencing on said population of cells that exhibit said detectable signal, and determining the genetic basis of resistance to one or more of said therapeutic agents.

A population of cells can be isolated using cell sorting techniques known in the art, such as FACS (Fluorescence Activated Cell Sorting). Cells can be sorted by FACS using a sorter (e.g., using a sorter available from Becton Dickinson Immunocytometry Systems, San Jose Calif.; see also U.S. Pat. Nos. 5,627,037; 5,030,002; and 5,137,809). As cells pass through the sorter, a laser beam excites the fluorescent compound while a detector counts cells that pass through and determines whether a fluorescent compound is attached to the cell by detecting fluorescence. The amount of label bound to each cell can be quantified and analyzed to characterize the sample. The sorter can also deflect the cell and separate cells bound by the binding protein from those cells not bound by the binding protein. The separated cells can be cultured and/or characterized.

### Sequencing

Prior to sequencing, a polynucleotide can be enriched by amplification. Similarly, a genome can be enriched by whole genome amplification. Examples of PCR techniques that can be used to amplify DNA regions herein include, but are not limited, to quantitative PCR, quantitative fluorescent PCR (QF-PCR), multiplex fluorescent PCR (MF-PCR), real time PCR (RT- PCR), single cell PCR, restriction fragment length polymorphism PCR (PCR-RFLP), PCR-RFLP/RT-PCR-RFLP, hot start PCR, nested PCR, in situ polonony PCR, in situ rolling circle amplification (RCA), bridge PCR, picotiter PCR and emulsion PCR. Other suitable amplification methods include, but are not limited to, the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide- primed PCR (DOP-PCR) and nucleic acid based sequence amplification (NABSA). Other amplification methods that can be used to amplify specific polymorphic loci include those described in, U.S. Pat. Nos. 5,242,794, 5,494,810, 4,988,617 and 6,582,938. While some embodiments of the invention are described in terms of polymerase chain reaction (PCR), in some embodiments, the method of amplification maybe, for example, self-sustained sequence reaction, ligase chain reaction, rapid amplification of cDNA ends, polymerase chain reaction and ligase chain reaction, Q-beta phage amplification, strand displacement amplification, or splice overlap extension polymerase chain reaction.

Prior to sequencing, a DNA sample of interest can be enriched by subtractive hybridization. Methods of subtractive hybridization are known in the art and described in U.S. Patent Nos. 5,935,788 and 5,599,672. Generally, in methods of subtractive hybridization, DNA or cDNA strands from a first DNA pool are hybridized to DNA or mRNA produced from a second DNA pool, and cDNA-mRNA hybrids or DNA duplexes are removed by enzymatic or chromatographic means. The remaining subtracted DNA can then be used in further analysis, such as sequencing.

In some embodiments, the sequencing error rate is evaluated based on the sequencing results of one or more control polynucleotides. In some embodiments, a sequencing platform is used wherein the sequencing platform is a massively parallel sequencing platform that produces at least 75 bp from a single end read. In some embodiments, the massively parallel sequencing platform produces at least 100, 150, 200, 300, 400, 500, 600, 700, 800, 900, 1000, 1100, 1200, 1300, 1400, 1500, or more than 2000 bp from a single end read.

A methodology useful in the present invention is based on massively parallel sequencing of millions of fragments using attachment of randomly fragmented genomic DNA to a planar, optically transparent surface and solid phase amplification to create a high density sequencing flow cell with millions of clusters, each containing about 1,000 copies of template per sq. cm. These templates are sequenced using four-color DNA sequencing-by-synthesis technology. See, products offered by Illumina, Inc., San Diego Calif. Also, see US 2003/0022207 to Balasubramanian, et al., published Jan. 30, 2003, entitled "Arrayed polynucleotides and their use in genome analysis." Using such methods, two unique adapters are ligated to each DNA fragment, which are then amplified using PCR.

In the process of bridge amplification, the flow cell surface is coated with single stranded oligonucleotides that correspond to the sequences of the adapters ligated during the sample preparation stage. Single-stranded, adapter-ligated fragments are bound to the surface of the flow cell exposed to reagents for polymerase-based extension. Priming occurs as the free/distal end of a ligated fragment "bridges" to a complementary oligonucleotide on the surface. Repeated denaturation and extension results in localized amplification of single molecules in millions of unique locations across the flow cell surface. A flow cell containing millions of unique clusters is then loaded into a sequencing device for automated cycles of extension and imaging. The first cycle of sequencing consists first of the incorporation of a single fluorescent nucleotide, followed by high resolution imaging of the entire flow cell. These images represent the data collected for the first base. Any signal above background identifies the physical location of a cluster, and the fluorescent emission identifies which of the four bases was incorporated at that position. This cycle is repeated, one base at a time, generating a series of images each representing a single base extension at a specific cluster. Base calls are derived with an algorithm that identifies the emission color over time.

In paired-end sequencing, a simple modification to the standard single-read DNA library preparation facilitates reading both the forward and reverse template strands of each cluster during one paired-end read. In some embodiments, the massively parallel sequencing platform produces at least 150, 200, 300, 400, 500, 600, 700, 800, 900, 1000, 1100, 1200, 1300, 1400, 1500, 2000, 3000, 5000, or more than 10,000 bp from a paired-end read.

In some embodiments, DNA polymerase is employed to image sequence information in a single DNA template as its complementary strand is synthesized. The nucleotides are inserted sequentially; only the time resolution to discriminate successive incorporations is required. After each successful incorporation event, a fluorescent signal is measured and then nulled by photobleaching. This method lends itself to massive parallelism. This technique permits observations of single molecule fluorescence by a conventional microscope equipped with total internal reflection illumination, which reduces background fluorescence. The surface of a quartz slide is chemically treated to specifically anchor DNA templates while preventing nonspecific binding of free nucleotides and a plastic flow cell is attached to the surface to exchange solutions. DNA template oligonucleotides are hybridized to a fluorescently labeled primer and bound to the surface via streptavidin and biotin with a surface density low enough to resolve single molecules. The primed templates are detected through their fluorescent tags, their locations are recorded for future reference, and the tags are photobleached. Labeled nucleotide triphosphates and DNA polymerase enzyme are then washed in and out of the flow cell while the known locations of the DNA templates are monitored for the appearance of fluorescence. The technique uses a combination of evanescent wave microscopy and single-pair fluorescence resonance energy transfer (spFRET) to reject unwanted noise. The donor fluorophore excites acceptors only within the Forster radius, thus effectively creating an extremely high-resolution near-field source. Because the Forster radius of this fluorophore pair is 5 nm, the spatial resolution of this method exceeds the diffraction limit by a factor of 50 and conventional near-field microscopy by an order of magnitude.

Another method of determining the identity of genomic DNA from the present samples is termed direct linear analysis (DLA), and is described in Chan et al. "DNA Mapping Using Microfluidic Stretching and Single-Molecule Detection of Fluorescent Site-Specific Tags," Genome Research 14:1137-1146 (2004). In this method, a microfluidic device is used for stretching DNA molecules in elongational flow that is coupled to a multicolor detection system capable of single fluorophore sensitivity. Double-stranded DNA molecules are tagged at sequence-specific motif sites with fluorescent bisPNA (Peptide Nucleic Acid) tags. The DNA molecules are then stretched in the microfluidic device and driven in a flow stream past confocal fluorescence detectors. DLA can provide the spatial locations of multiple specific sequence motifs along individual DNA molecules, and thousands of individual molecules can be analyzed per minute.

High throughput sequencing can involve sequencing- by-synthesis, sequencing-by-ligation, and ultra deep sequencing.

Sequence-by-synthesis can be initiated using sequencing primers complementary to the sequencing element on the nucleic acid tags. The method involves detecting the identity of each nucleotide immediately after (substantially real-time) or upon (real-time) the incorporation of a labeled nucleotide or nucleotide analog into a growing strand of a complementary nucleic acid sequence in a polymerase reaction. After the successful incorporation of a label nucleotide, a signal is measured and then nulled by methods known in the art. Examples of sequence-by-synthesis methods are described in U.S. Application Publication Nos. 2003/ 0044781, 2006/0024711, 2006/0024678 and 2005/0100932. Examples of labels that can be used to label nucleotide or nucleotide analogs for sequencing-by-synthesis include, but are not limited to, chromophores, fluorescent moieties, enzymes, antigens, heavy metal, magnetic probes, dyes, phosphorescent groups, radioactive materials, chemiluminescent moieties, scattering or fluorescent nanoparticles, Raman signal generating moieties, and electrochemical detection moieties. Sequencing-by-synthesis can generate at least 1,000, at least 5,000, at least 10,000, at least 20,000, 30,000, at least 40,000, at least 50,000, at least 100,000 or at least 500,000 reads per hour. Such reads can have at least 50, at least 60, at least 70, at least 80, at least 90, at least 100, at least 120 or at least 150 bases per read.

Another sequencing method involves hybridizing the amplified regions to a primer complementary to the sequence element in an LST. This hybridization complex is incubated with a polymerase, ATP sulfurylase, luciferase, apyrase, and the substrates luciferin and adenosine 5' phosphosulfate. Next, deoxynucleotide triphosphates corresponding to the bases A, C, G, and T (U) are added sequentially. Each base incorporation is accompanied by release of pyrophosphate, converted to ATP by sulfurylase, which drives synthesis of oxyluciferin and the release of visible light. Since pyrophosphate release is equimolar with the number of incorporated bases, the light given off is proportional to the number of nucleotides adding in any one step. The process is repeated until the entire sequence is determined. Yet another sequencing method involves a fourcolor sequencing by ligation scheme (degenerate ligation), which involves hybridizing an anchor primer to one of four positions. Then an enzymatic ligation reaction of the anchor primer to a population of degenerate nonamers that are labeled with fluorescent dyes is performed. At any given cycle, the population of nonamers that is used is structure such that the identity of one of its positions is correlated with the identity of the fluorophore attached to that nonamer. To the extent that the ligase discriminates for complementarily at that queried position, the fluorescent signal allows the inference of the identity of the base. After performing the ligation and four-color imaging, the anchor primer: nonamer complexes are stripped and a new cycle begins. Methods to image sequence information after performing ligation are known in the art.

U.S. patent application Ser. No. 09/572,530 describes a zero-mode waveguide used for sequencing nucleic acid molecules. This method involves providing a complex of a nucleic acid polymerizing enzyme and a target nucleic acid molecule oriented with respect to each other in a position suitable to add a nucleotide analog at an active site complementary to the target nucleic acid. A plurality of types of nucleotide analogs are provided proximate to the active site, where each type of nucleotide analog is complementary to a different nucleotide in the target nucleic acid, leaving the added nucleotide analog ready for subsequent addition of nucleotide analogs. The nucleotide analog added at the active site as a result of the polymerizing step is identified. The steps of providing a plurality of nucleotide analogs, polymerizing, and identifying are repeated so that the sequence of the target nucleic acid is determined. The zero-mode waveguide is used to carry out the step of identifying the nucleotide analog added to the target nucleic acid.

In some embodiments, high throughput sequencing involves the use of ultra-deep sequencing, such as described in Marguiles et al., Nature 437 (7057): 376-80 (2005). Briefly, the amplicons are diluted and mixed with beads such that each bead captures a single molecule of the amplified material. The DNA molecule on each bead is then amplified to generate millions of copies of the sequence which all remain bound to the bead. Such amplification can occur by PCR. Each bead can be placed in a separate well, which can be a (optionally addressable) picolitre-sized well. In some embodiments, each bead is captured within a droplet of a PCR-reaction-mixturein- oil-emulsion and PCR amplification occurs within each droplet. The amplification on the bead results in each bead carrying at least one million, at least 5 million, or at least 10 million copies of the original amplicon coupled to it. Finally, the beads are placed into a highly parallel sequencing by synthesis machine which generates over 400,000 reads (-100 by per read) in a single 4 hour run. Other methods for ultra-deep sequencing that can be used are described in Hong, S, et al. Nat. Biotechnol. 22(4): 435-9 (2004); Bennett, B. et al. Pharmacogenomics 6(4):373- 82 (2005); Shendure, P. et al. Science 309 (5741):1728-32 (2005).

### Gene Replacement Therapeutic Agents

In embodiments of the disclosure, gene replacement therapeutic agents can comprise one or more of a stem cell or a vector such as an adenovirus, a herpes simplex virus (HSV), a baculovirus, an adeno-associated virus (AAV), a recombinant adeno-associated virus (rAAV), a retrovirus, and a lentivirus. An rAAV can comprise an rAAV genome comprising one or more AAV inverted terminal repeats (ITRs) flanking a polynucleotide encoding one or more gene replacement therapeutic agents.

The term vector refers to any agent, such as a plasmid, phage, transposon, cosmid, chromosome, liposome, DNA-viral conjugates, RNA/DNA oligonucleotides, virus, bacteria, etc., which is capable of transferring gene sequences into cells. Thus, the term includes cloning and expression vehicles, as well as viral and non-viral vectors. A vector may be targeted to specific cells by linking a target molecule to the vector. A targeting molecule is any agent that is specific for a cell or tissue type of interest, including for example, a ligand, antibody, sugar, receptor, or other binding molecule. The invention is also intended to include such other forms of vectors which serve equivalent functions.

The rAAV genomes of the invention lack AAV rep and cap DNA. AAV DNA in the rAAV genomes may be from any AAV serotype for which a recombinant virus can be derived including, but not limited to, AAV serotypes AAV-1, AAV-2, AAV-3, AAV-4, AAV-5, AAV-6, AAV-7, AAV-8, AAV-9, AAV-10 and AAV-11. In some embodiments, the AAV DNA is derived from AAV serotype AAV-6. The nucleotide sequences of the genomes of the AAV serotypes are known in the art. For example, the complete genome of AAV-1 is provided in GenBank Accession No. NC_002077; the complete genome of AAV-2 is provided in GenBank Accession No. NC_001401; the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_OO 1862; at least portions of AAV-7 and AAV-8 genomes are provided in GenBank Accession Nos. AX753246 and AX753249, respectively. An AAV vector refers to vectors derived from an adeno-associated virus serotype, including human AAV-1, AAV-2, AAV-3, AAV-4, AAV-5, AAV-6, avian AAV, ovian AAV, etc., and to vectors derived from more than one AAV serotype (hybrid AAV vectors). For example, a hybrid AAV vector may contain DNA sequences derived from both AAV-1 and AAV-2.

A recombinant AAV vector plasmid refers to one type of recombinant AAV vector wherein the vector comprises a plasmid. As with AAV vectors in general, 5' and 3' ITRs flank the selected heterologous nucleotide sequence. AAV vectors can also include transcription sequences such as polyadenylation sites, as well as selectable markers or reporter genes, enhancer sequences, and other control elements which allow for the induction of transcription. Such control elements are described more fully below. In addition, an AAV vector can be stably introduced into a cell line or cell lines for the purpose of viral particle production. Such a cell line is usually termed as AAV packaging cell line.

As used herein, the term recombinant AAV, recombinant AAV particle or recombinant AAV virion is defined as an infectious, replication-defective virus composed of an AAV protein shell encapsidating (i.e., surrounding with a protein coat) a heterologous nucleotide sequence, which in turn is flanked 5' and 3' by AAV ITRs. In this regard, single-stranded AAV nucleic acid molecules (either the sense/coding strand or the antisense/anticoding strand as those terms are generally defined) can be packaged into an AAV virion; both the sense and the antisense strands are equally infectious. When the recombinant AAV DNA is equal to or smaller than 50% of the full length viral genome (about 5,000 nucleotides), it can also be packaged as double-stranded hairpin-like DNA into AAV virion. Such virion is also fully infectious.

A number of techniques for constructing recombinant AAV are known in the art. See, e.g., U.S. Pat. No. 5,173,414, Lebkowski et al. Mol Cell Biol8, 3988-3996 [1988]; Carter B J, Current Opinion in Biotechnology 3, 533-539 [1992]; Muzyczka N, cited supra; and Zhou et al.J. Exp. Med. 179, 1867-1875 [1994]; Xiao et al. J. Virol. 72,2224-32 [1998].

A vector can include a control element or regulatory element, terms that refer collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell. A control element may be cis acting or may be responsive to trans acting factors. Depending upon the nature of the regulation, promoters may be constitutive or regulated. Examples of promoters are SP6, T4, T7, SV40 early promoter, cytomegalovirus (CMV) promoter, mouse mammary tumor virus (MMTV) steroidinducible promoter, Moloney murine leukemia virus (MMLV) promoter, phosphoglycerate kinase (PGK) promoter, muscle creatine kinase (MCK) promoter, myosin promoter, α-actin promoter and the like. Alternatively, the modified versions of the above promoters and even the synthetic muscle promoters (Li et al. Nat Biotechnol 17, 241-245, [1999]) may be included. Finally, the promoter may be an endogenous AAV promoter or AAV inverted terminal repeat (ITR).

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlinetal., 1983, Gene, 23:65-73) or by direct, blunt-end ligation (Senapathy & Carter, 1984, J. BioI. Chern., 259: 4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); McLaughlin et aI., J. Virol., 62:1963 (1988); and Lebkowski et al., 1988 Mol. Cell. Biol., 7:349 (1988). Samulski et al. (1989, J. Virol., 63:3822-3828); U.S. Pat. No. 5,173,414; WO 95/13365 and corresponding U.S. Pat. No. 5,658,776; WO 95/13392; WO 96/17947; PCT/US98/18600; Samulski et al. (1989, J. Virol., 63:3822-3828); U.S. Pat. No. 5,173,414; WO 95/13365 and corresponding U.S. Pat. No. 5,658,776; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. Gene Therapy 4:609-615; Clark et al. (1996) Gene Therapy 3:1124-1132; U.S. Pat. No. 5,786,211; U.S. Pat. No. 5,871, 982; and U.S. Pat. No. 6,258,595.

For the purposes of the invention, suitable host cells for producing rAAV virions include microorganisms, yeast cells, insect cells, and mammalian cells, that can be, or have been, used as recipients of a heterologous DNA molecule. The term includes the progeny of the original cell which has been transfected. Thus, a host cell as used herein generally refers to a cell which has been transfected with an exogenous DNA sequence. Cells from the stable human cell line, 293 (readily available through, e.g., the American Type Culture Collection under Accession Number ATCC CRL1573) are preferred in the practice of the present invention. Particularly, the human cell line 293 is a human embryonic kidney cell line that has been transformed with adenovirus type-5 DNA fragments (Graham et al (1977) J. Gen. Virol. 36:59), and expresses the adenoviral E1a and E1b genes (Aiello et al (1979) Virology 94:460). The 293 cell line is readily transfected, and provides a particularly convenient platform in which to produce rAAV virions.

AAV rep coding region refers to the art-recognized region of the AAV genome which encodes the replication proteins Rep 78, Rep 68, Rep 52 and Rep 40. These Rep expression products have been shown to possess many functions, including recognition, binding and nicking of the AAV origin of DNA replication, DNA helicase activity and modulation of transcription from AAV (or other heterologous) promoters. The Rep expression products are collectively required for replicating the AAV genome. For a description of the AAV rep coding region, see, e.g., Muzyczka, N. (1992) Current Topics in Microbiol. and Immunol. 158:97-129; and Kotin, R. M. (1994) Human Gene Therapy 5:793-801. Suitable homologues of the AAV rep coding region include the human herpesvirus 6 (HHV-6) rep gene which is also known to mediate AAV-2 DNA replication (Thomson et al. (1994) Virology 204:304-311).

By AAV cap coding region is meant the art-recognized region of the AAV genome which encodes the capsid proteins VP1, VP2, and VP3, or functional homologues thereof. These Cap expression products supply the packaging functions which are collectively required for packaging the viral genome. For a description of the AAV cap coding region, see, e.g., Muzyczka, N. and Kotin, R. M. (supra). AAV helper functions are introduced into the host cell by transfecting the host cell with an AAV helper construct either prior to, or concurrently with, the transfection of the AAV expression vector. AAV helper constructs are thus used to provide at least transient expression of AAV rep and/or cap genes to complement missing AAV functions that are necessary for productive AAV infection. AAV helper constructs lack AAV ITRs and can neither replicate nor package themselves. These constructs can be in the form of a plasmid, phage, transposon, cosmid, virus, or virion. A number of AAV helper constructs have been described, such as the commonly used plasmids pAAV/Ad and p1M29+45 which encode both Rep and Cap expression products. See, e.g., Samulski et al. (1989) J. Virol. 63:3822-3828; and McCarty et al. (1991) J. Virol. 65:2936-2945. A number of other vectors have been described which encode Rep and/or Cap expression products. See, e.g., U.S. Pat. No. 5,139,941. Both AAV expression vectors and AAV helper constructs can be constructed to contain one or more optional selectable markers. Suitable markers include genes which confer antibiotic resistance or sensitivity to, impart color to, or change the antigenic characteristics of those cells which have been transfected with a nucleic acid construct containing the selectable marker when the cells are grown in an appropriate selective medium. Several selectable marker genes that are useful in the practice of the invention include the hygromycin B resistance gene (encoding Aminoglycoside phosphotranferase (APH)) that allows selection in mammalian cells by conferring resistance to G418 (available from Sigma, St. Louis, Mo.). Other suitable markers are known to those of skill in the art.

Host cells containing the above-described AAV expression vectors must be rendered capable of providing AAV helper functions in order to replicate and encapsidate the nucleotide sequences flanked by the AAV ITRs to produce rAAV virions. AAV helper functions are generally AAV derived coding sequences which can be expressed to provide AAV gene products that, in turn, function in trans for productive AAV replication. AAV helper functions are used herein to complement necessary AAV functions that are missing from the AAV expression vectors. Thus, AAV helper functions include one, or both of the major AAV ORFs, namely the rep and cap coding regions, or functional homologues thereof.

The host cell (or packaging cell) must also be rendered capable of providing non AAV derived functions, or accessory functions, in order to produce rAAV virions. Such methods are known in the art and disclosed in U.S. Pat. No.5,858,351.

Titers of rAAV to be administered in methods of the invention will vary depending, for example, on the particular rAAV, the mode of administration, the treatment goal, the individual, and the cell type(s) being targeted, and may be determined by methods standard in the art. Titers of rAAV may range from about 1x106, about 1x107, about 1x108, about 1x109, about 1x1010 about 1x1011, about 1x1012, about 1x1013 to about 1x1014 or more DNase resistant particles (DRP) per ml. Dosages may also be expressed in units of viral genomes (vg).

The rAAV may be purified by methods standard in the art such as by column chromatography or cesium chloride gradients. Methods for purifying rAAV vectors from helper virus are known in the art and include methods disclosed in, for example, Clark et al., Hum. Gene Ther., 10(6): 1031-1039 (1999); Schenpp and Clark, Methods Mol. Med., 69427-443 (2002); U.S. Pat. No. 6,566,118 and WO 98/09657.

In another embodiment, the disclosure contemplates delivery of compositions comprising rAAV of the present invention. These compositions may be used to enhance muscle and/or improve muscle function. In some embodiments, compositions of the disclosure comprise a rAAV encoding a myostatin inhibitor of interest. In other embodiments, compositions of the present invention may include two or more rAAV encoding different myostatin inhibitors of interest. In still further embodiments, compositions comprise an rAAV encoding one or more dystrophin proteins.

In some embodiments, a gene replacement therapeutic agent comprises one or more stem cells. Stem cells are pluripotent or multipotent cells that can differentiate into multiple cell types. Stem cells also include cells that can transdifferentiate into at least one other cell type. A precursor cell or progenitor cell can be any cell in a specific differentiation pathway that is capable of differentiating into a more mature cell. A differentiated cell is a cell which is not capable of differentiating into a more mature cell under normal physiological conditions. As such, the term precursor cell population refers to a group of cells capable of developing into a more mature cell. A precursor cell population can comprise cells that are totipotent, cells that are pluripotent and cells that are stem cell lineage restricted (i.e., cells capable of developing into less than all hematopoietic lineages, or into, for example, only cells of erythroid lineage). A stem cell can be a pluripotent stem cell or a totipotent stem cell. As used herein, the term totipotent cell refers to a cell capable of developing into all lineages of cells. Similarly, the term totipotent population of cells refers to a composition of cells capable of developing into all lineages of cells. Also as used herein, the term pluripotent cell refers to a cell capable of developing into a variety (albeit not all) lineages and are at least able to develop into all hematopoietic lineages (e.g., lymphoid, erythroid, and thrombocytic lineages). For example, a pluripotent cell can differ from a totipotent cell by having the ability to develop into all cell lineages except endothelial cells. A pluripotent population of cells refers to a composition of cells capable of developing into less than all lineages of cells but at least into all hematopoietic lineages. As such, a totipotent cell or composition of cells is less developed than a pluripotent cell or compositions of cells. As used herein, the terms develop, differentiate and mature all refer to the progression of a cell from the stage of having the potential to differentiate into at least two different cellular lineages to becoming a specialized cell. Such terms can be used interchangeably for the purposes of the present application.

Stem cells are typically classified into several types: embryonic stem cells (ESCs) found in blastocysts, adult stem cells found in post-embryonic tissues and induced pluripotent stem cells (iPSCs) which have been de-differentiated from the adult type into an embryonic-type state. Adult stem cells are extracted from fetal and adult tissue and, for the most part, are limited in the cell types into which they can differentiate. Although they have limited differentiating capacity as compared to ESC, the adult stem cells can function stably and have been shown to differentiate across some tissue types. Pittenger et al. (1999) Science 284(2): 143-147 and Huard et al. 2004) Curr. Opin. Biotechnol. 15(5):419-23.

Stem cells or precursor cells include but are not limited to, e.g., peripheral blood stem cells (PBSC), stem cells isolated from bone marrow (bone marrow cells; BMCs); stem cells isolated from adipose tissue; mesenchymal stem cells (MSCs), stem cells isolated from umbilical cord blood, menstral fluid, cardiac derived cells, embryonic stem cells, CD30+ cells, CD34+ cells, CD34" cells, CD9+ cells, CD29+ cells, CD44+ cells, CD45+ cells, CD49+ cells, CD54+ cells, CD56+ cells, CD59+ cells, CD71+ cells, CD90+ cells, e.g., CD90.1+ or CD90.2+ cells, CD105+ cells, CD133+ cells, CD135+ (flt-3+) cells, CD140a+ cells, CDCP1+ cells, CD146+(m"c-18+) cells, ABCG2+ cells, CD 144+ cells, fetal liver kinase 1+ cells, Stro-l+ cells, CD117+ (c-kit+) cells, nestin+ cells, PSA-NCAm+ cells, CD30+ cells, p75 neurotophin+ cells, CD106+ cells, CD120a+ cells, CD124+ cells, CD166+ cells, stem cell factor+ (SCF+) cells, Sca-1+ cells, SH2+ cells, SH3+ cells, HLA, e.g., HLA-ABC cells, bone morphogenic protein protein+ (BMP) cells, e.g., BMP2+ and BMP4+ cells, Gap43+ cells, glial fibrillary acidic protein"1" (GFAP+) cells, myelin basic protein+ (MBP+) cells, 04+ cells, O1+ cells, synaptophysin+ cells, alkaline phosphatase+ cells, cripto+ (TDGF-1+) cells, podocalyxin+ cells, sulfated proteoglycan+ cells, e.g., silylated keratin sulfate proteoglycan+ cells, stage-specific embryonic antigen+ (e.g., SSEA-1, -3 and -4) cells, TRA-1-60+ cells, TRA-1-81+ cells, osteocalcin+ cells, matrix gla protein+ cells, osteopontin"1" cells, Thyl+ cells, collagen type II+ cells, collagen type IV+ cells, fatty acid transporter+ cells, and beta-integrin+ cells.

The present invention provides for the successful transfer of a selected gene to a muscle cell or tissue using recombinant AAV virions. The method allows for the direct, in vivo injection of recombinant AAV virions into muscle tissue, e.g., by intramuscular injection, as well as for the in vitro transduction of muscle cells which can subsequently be introduced into a subject for treatment. Differentiated muscle cells and tissue provide a desirable target for gene therapy since they are readily accessible and non-dividing. However, the present invention also finds use with undifferentiated muscle cells, such as myoblasts, which can be transduced in vitro, and subsequently introduced into a subject. Intramuscular injection can be accomplished using a needle array device.

### Replacement Genes

In embodiments of the present disclosure, a gene replacement therapeutic agent serves to provide a replacement gene, thereby treating a disease or disorder in which the underlying cause is a defect in the corresponding gene in a subject and/or in which replacement of the gene would be beneficial to the subject. One or more genes may be replaced using methods of the invention.

In some embodiments, the replacement gene encodes a biologically functional micro-dystrophin. The gene encoding micro-dystrophin may be less than 5 kb. Importantly, a micro-dystrophin can protect muscle from dystrophic pathology and symptoms.

A gene encoding a micro-dystrophin can be referred to as a dystrophin minigene, a term which encompasses dystrophin constructs created by extensive deletions in the central rod domain plus extensive deletion in the C-terminal domain of the human dystrophin DNA. In addition, dystrophin minigenes may contain a modified N-terminal domain in which DNA sequences surrounding the original protein translation initiation codon ATG are modified. The modified sequences enhance the mini-dystrophin protein synthesis. Alternatively, the dystrophin minigene may be a hybrid gene in which some of the domains are substituted with homologous domains from utrophin or spectrin genes (Tinsley et al, Nature 360, 591-593 [1992]; Koenig et al. Cell 53, 219-216 [1988]). In particular, utrophin is highly homologous to dystrophin in both structure and functions, so that their major domains should be interchangeable (Tinsley et al, Nature. 384, 349-353 [1996]; Deconinck et al, Nat Med. 3, 1216-21 [1997]; Rafael et al Nat Genet. 19, 79-82 [1998];). For example, the N-terminal and/or the C-terminal domains of dystrophin may be substituted with the utrophin counterparts in the dystrophin minigenes. Similarly, the central rod domain may consist of rod repeats from utrophin or spectrin genes. The dystrophin minigenes are smaller than the 5 kb packaging limit of AAV viral vectors. Furthermore, it is also plausible to construct a minigene of utrophin in a similar fashion as of the dystrophin minigene described in this invention. Because some DMD patients completely lack the dystrophin protein, the dystrophin minigene product may be a neo-antigen. Substitution of dystrophin domains with those of utrophin may lower immune responses.

A dystrophin minigene can comprise the N-terminus sequence of the dystrophin gene, the C-terminal cysteine-rich (CR) domain of the dystrophin gene, at least hinges HI and H4 of dystrophin gene, and at least four rod repeats. The rod repeats may be chosen from the rod repeats of dystrophin, utrophin or spectrin genes, preferably from the 24 rod repeats of dystrophin gene, and most preferably from the group consisting of rod repeats R1, R2, R3, R22, R23 and R24 of dystrophin. gene. The N-terminus of the dystryphin minigene may be modified to improve expression efficiency without affecting the functionality of the gene product. For example, the original sequence surrounding the translation initiation ATG codon of the dystrophin gene may be substituted by the Kozak sequence to increase the efficiency of protein synthesis. In one embodiment of the current invention, the three nucleotides upstream of the coding sequence may be changed from "AAA" to "CCA" and the fourth nucleotide in the coding sequence may be changes from "C" to "G". In addition, a portion or the entire N-terminus may be substituted by its counterpart of the utrophin gene. Similarly, the CR domain of the dystrophin minigene can also be substituted by its counterpart of the utrophin gene.

In some embodiments, a replacement gene encodes an additional one or more copies of a myostatin inhibitor, or an enhanced version of a myostatin inhibitor. A myostatin inhibitor can include wild-type or mutant versions of native myostatin inhibitors, non-limiting examples of which include the myostatin propeptide, follistatin, FLRG and GASP-1.

Myostatin inhibitors of the disclosure may be peptides or polypeptides. The proteins may inhibit myostatin by binding myostatin [McPherron et al., Nature, 387(6628): 83-90 (1997)] or by binding the myostatin receptor activin IIb [McPherron et al., Nat. Genet., 22(3): 260-264 (1999)]. Examples of proteins that inhibit myostatin by binding to myostatin are myostatin propeptide, follistatin [Shimasaki et al., u.s. Pat. No. 5,041,538], other follistatin-like proteins (U.S. Pat. Nos. 5,942,420; 6,410,232; 6,537,966; and 6,953,662), FLRG ([Hill et al., J. Biol. Chem., 277(43): 40735-40741 (2002)] and GASP-1 ([Hill et al., Mol Endocrinol, 17: 1144-1154 (2003)]. Proteins that are myostatin inhibitors according to the invention may be protein fragments or may be chimeric (i.e., fusion) proteins.

Other suitable replacement genes of the disclosure include, for example, sarcoglycans for replacement in sarcoglycan deficiency and treatment of Amyotrophic lateral sclerosis (ALS) patients with IGF-1 or mutant SODI interference strategies.

Expression of a replacement gene may be controlled by a number of regulatory elements, including but not limited to, AAV inverted terminal repeat (ITR), retrovirus long terminal repeat (LTR), cytomegalovirus (CMV) immediate early promoter and/or enhancer, CMV enhancer and chicken β-actin promoter, α-actin promoter, myosin promoter, muscle-specific creatine kinase (MCK) promoter and/or enhancer, and the like. Alternatively, the modified versions of the above promoters and the synthetic muscle promoters may also be used.

Control elements of the disclosure include promoter regions, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control elements need always be present so long as the selected coding sequence is capable of being replicated, transcribed and translated in an appropriate host cell.

The term promoter region is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence.

Operably linked refers to an arrangement of elements wherein the components so described are configured so as to perform their usual function. Thus, control elements operably linked to a coding sequence are capable of effecting the expression of the coding sequence. The control elements need not be contiguous with the coding sequence, so long as they function to direct the expression thereof. Thus, for example, intervening untranslated yet transcribed sequences can be present between a promoter sequence and the coding sequence and the promoter sequence can still be considered operably linked to the coding sequence.

Control elements, such as muscle-specific and inducible promoters, enhancers and the like, can be used in methods of the present disclosure. Such control elements include, but are not limited to, those derived from the actin and myosin gene families, such as from the myoD gene family (Weintraub et aL (1991) Science 251:761-766); the myocyte-specific enhancer binding factor MEF-2 (Cserjesi and Olson (1991) Mol. Cell Biol. 11:4854-4862); control elements derived from the human skeletal actin gene (Muscat et aL (1987) Mol. Cell Biol. 7:4089-4099) and the cardiac actin gene; muscle creatine kinase sequence elements (Johnson et aL (1989) Mol. Cell Biol. 9:3393-3399) and the murine creatine kinase enhancer (mCK) element; control elements derived from the skeletal fast-twitch troponin C gene, the slow-twitch cardiac troponin C gene and the slow-twitch troponin I gene; hypoxia-inducible nuclear factors (Semenza et aL (1991) Proc. Natl. Acad. Sci. USA 88:5680-5684; Semenza et al. J. Biol. Chem. 269:23757-23763); steroid-inducible elements and promoters, such as the glucocorticoid response element (GRE) (Mader and White (1993) Proc. Natl. Acad. Sci. USA 90:5603-5607); the fusion consensus element for RU486 induction; and elements that provide for tetracycline regulated gene expression (Dhawan et aL (1995) Somat. Cell. Mol. Genet. 21:233-240; Shockett et al (1995) Proc. Natl. Acad. Sci. USA 92:6522-6526.

In some embodiments, a polynucleotide encoding one or more effectors of muscle function is operatively linked to transcriptional control DNA (e.g., promoter DNA) and polyadenylation signal sequence DNA that are functional in target cells to form a gene cassette. The gene cassette may also include intron sequences to facilitate processing of the RNA transcript when expressed in mammalian cells. Alternatively, the polynucleotide in the rAAV genome be effector of muscle function RNA or may encode RNA of an effector of muscle function. The RNAs may be antisense RNAS, ribozymes, small interfering RNAs (RNAi) or aptamers that effect muscle function by inhibiting expression of a negative regulator of muscle function (e.g. myostatin or its receptor activin IIb). Other modes of inhibiting expression include, but are not limited to, antisense RNA directed to translation initiation sites and RNA that binds to and prevents DNA unwinding and transcription. As yet another example, commercial providers such as Ambion Inc. (Austin, Tex.), Darmacon Inc. (Lafayette, Colo.), InvivoGen (San Diego, Calif.), and Molecular Research Laboratories, LLC (Herndon, Va.) generate custom siRNA molecules. In addition, commercially kits are available to produce custom siRNA molecules, such as SILENCERTM siRNA Construction Kit (Ambion Inc., Austin, Tex.) opsiRNA System (InvivoGen, San Diego, Calf.).

### Diseases and Disorders

Methods of the present disclosure are useful in treating muscle diseases or disorders such as muscle wasting diseases. The term muscle wasting disease refers broadly to conditions that result in reduced muscle function.

Muscular wasting diseases can generally be divided into three categories: (1) muscular dystrophies; (2) inflammatory myopathies; and (3) muscle atrophies. Muscular dystrophies for treatment with methods of the present disclosure include, for example, Duchenne muscular dystrophy (DMD), Becker muscular dystrophy, and Limb Girdle muscular dystrophy. Muscular dystrophies are hereditary, progressive, and each type causes a characteristic, selective pattern of muscle weakness. Other muscular dystrophies include facioscapulhumeral muscular dystrophy, congenital muscular dystrophy, oculopharyngeal muscular dystrophy, distal muscular dystrophy and Emery-Dreifuss muscular dystrophy.

Duchenne muscular dystrophy and Becker muscular dystrophy are similar in that these dystrophies share similar patterns of muscle weakness and disability and are inherited in the same way. Typically, subjects in need of treatment for Duchenne or Becker muscular dystrophies have trouble walking and eventually become wheelchair dependent. Generally, an arbitrary means of distinguishing between Duchenne muscular dystrophy and Becker muscular dystrophy depends on whether the affected subject can still walk at 16 years of age. Subjects with Duchenne muscular dystrophy are generally wheelchair bound by their teenage years. More specifically, a muscle biopsy of a subject affected with Duchenne muscular dystrophy will show more disabling change as compared to a subject affected with Becker muscular dystrophy.

Duchenne muscular dystrophy and Becker muscular dystrophy are due to defects or mutations of the same gene, which is directed to enabling muscle fibers to make dystrophin. The dystrophin gene encodes a large 427 kD protein that functions in linking the extracellular matrix to the muscle fiber cytoskeleton. The amino terminus on dystrophin binds to filamentous actin in contact with the contractile apparatus of skeletal muscle, while a cysteine-rich domain near the carboxyl terminus binds to dystroglycan proteins localized to the fiber membrane in connection with other membrane proteins that constitute the dystrophin glycoprotein complex (DGC). The absence of dystrophin expression causes a concomitant decrease in DGC members. It is now believed that loss of dystrophin and the resulting DGC complex compromises the integrity of skeletal muscle membranes, which undergo damage after repeated cycles of contractile activity. Membrane damage is further thought to cause creatine kinase release, stimulate the influx of calcium, and induce the recruitment of immune T cells, macrophages, and mast cells, culminating in muscle fiber necrosis. The regenerative capacity of these cells become exhausted in Duchenne muscular dystrophy patients, thus giving way to accumulated fibrosis and fatty deposits that exacerbates the muscle wasting process.

Limb Girdle muscular dystrophies include at least ten different inherited disorders that can further be classified into two categories, autosomal-dominant (LGMD 1) and autosomal-recessive (LGMD 2) syndromes. The symptoms of most Limb Girdle muscular dystrophies typically begin with pelvic muscle weakness starting in childhood to young adulthood. Later, there is an onset of shoulder weakness with progression to significant loss of mobility or wheelchair dependence over the next 20-30 years.

The defective gene causing most autosomal-dominant type Limb Girdle muscular dystrophies has not yet been discovered, but the diseases have been linked to mutations in various chromosomes. For example, LGMD 1A type dystrophy has been linked to chromosome 5. Additionally, LGMD 1 B type dystrophy has been linked to chromosome 1. Other chromosomes that have been linked to the autosomal-dominant type Limb Girdle muscular dystrophies include chromosomes 3 and 7. Several of the autosomal recessive type Limb Girdle muscular dystrophies are due to mutations in the dystrophin-associated glycoproteins (i.e., sarcoglycans).

Another embodiment encompasses using the method of the present disclosure to treat an inflammatory myopathy. Inflammatory myopathies are diseases or abnormal conditions of the striated skeletal muscles. The cause of most inflammatory myopathies is unknown. Typically, inflammatory myopathies are believed to result from an autoimmune reaction, whereby the body's own immune system attacks the muscle cells. Examples of inflammatory myopathies for treatment can include polymyositis and dermatomyositis.

Symptoms of polymyositis include muscle inflammation and muscle tenderness. The onset of symptoms may be acute, but the condition usually progresses slowly and, if left untreated, may compromise the subject's ability to walk.

Subjects in need of treatment for dermatomyositis have similar symptoms as with polymyositis, but additionally show signs of a distinctive skin rash. Specifically, a violet-colored or dusky red rash breaks out over the subject's face, eyelids, and areas around their nails, knuckles, elbows, knees, chest, and back. Dermatomyositis typically occurs in adult subjects in their late 40s to early 60s or in children between the ages of 5 and 15.

In some embodiments, the method of the present disclosure can be utilized to treat muscle atrophy. Muscle atrophy can be the result of a disorder or condition such as cancer cachexia, AIDS cachexia, or cardiac cachexia. Cachexia is generally associated with the massive loss (up to 30% of total body weight) of both adipose tissue and skeletal muscle mass that may occur as a side effect of many diseases such as cancer, AIDS, and chronic heart failure. The loss of adipose tissue and skeletal muscle mass can lead to anorexia, early satiety, fatigue, generalized muscle weakness, decreased muscle function, and progressive muscle wasting.

Muscle wasting disease in a subject can be the result of the subject having a muscular dystrophy; muscle atrophy; X-linked spinal-bulbar muscular atrophy (SBMA), cachexia; malnutrition, tuberculosis, leprosy, diabetes, renal disease, chronic obstructive pulmonary disease (COPD), cancer, end stage renal failure, burns, diabetes, congestive heart failure, sarcopenia, emphysema, osteomalacia, or cardiomyopathy.

In another embodiment, the muscle wasting disease is due to infection with enterovirus, Epstein-Barr virus, herpes zoster, HIV, trypanosomes, influenze, coxsackie, rickettsia, trichinella, schistosoma or mycobacteria.

Sarcopenia is a debilitating disease that afflicts the elderly and chronically ill patients and is characterized by loss of muscle mass and function, which may be treated, etc., by the methods of this invention. In addition, other circumstances and conditions are linked to, and can cause muscle wasting disorders. For example, studies have shown that in severe cases of chronic lower back pain, there is paraspinal muscle wasting.

Further non-limiting examples of diseases or disorders contemplated for treatment with methods of the disclosure include neurodegenerative diseases/disorders in which muscle is adversely affected (for example, Amyotrophic Lateral Sclerosis multiple sclerosis and spinal muscular atrophy), sarcopenia, cachexia, obesity, Type II diabetes, Pompe disease and lysosomal storage disorders.

### Target Tissues for Gene Therapy

In some embodiments, the disclosure exemplifies methods of introducing gene replacement therapeutic agents to muscle tissue (used interchangeably with the term muscle) or muscle cells, terms which refer to a cell or group of cells derived from muscle, including but not limited to cells and tissue derived from skeletal muscle; smooth muscle, e.g., from the digestive tract, urinary bladder and blood vessels; and cardiac muscle. The term muscle cell captures muscle cells both in vitro and in vivo. Thus, for example, an isolated cardiomyocyte would constitute a muscle cell for purposes of the present invention, as would a muscle cell as it exists in muscle tissue present in a subject in vivo. Also encompassed are muscle cells and muscle tissue derived from both differentiated and nondifferentiated muscle cells, such as myocytes such as myotubes, myoblasts, both dividing and differentiated, cardiomyocytes and cardiomyoblasts.

Tissues in general are well known to the medical arts and may include any cohesive, spatially discrete non-fluid defined anatomic compartment that is substantially the product of multicellular, intercellular, tissue and/or organ architecture, such as a three-dimensionally defined compartment that may comprise or derive its structural integrity from associated connective tissue and may be separated from other body areas by a thin membrane (e.g., meningeal membrane, pericardial membrane, pleural membrane, mucosal membrane, basement membrane, omentum, organ-encapsulating membrane, or the like). Non-limiting exemplary tissues may include brain, liver, lung, kidney, prostate, ovary, spleen, lymph node (including tonsil), thyroid, pancreas, heart, skeletal muscle, intestine, larynx, esophagus and stomach. Anatomical locations, morphological properties, histological characterization, and invasive and/or non-invasive access to these and other solid tissues are all well known to those familiar with the relevant arts. In some embodiments, the tissue is normal. In some embodiments, the tissue is, or is suspected of being, cancerous, inflamed, infected, atrophied, numb, in seizure, or coagulated. In some embodiments, the tissue is, or is suspected of being, affected by a muscle disease or disorder. In some embodiments, the tissue is affected by a muscle disease or disorder.

### Subjects for Gene Therapy

In certain embodiments, the subject is one of a preclinical model and a human subject. The subject can be a mammal, referring to any member of the class Mammalia including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

In some embodiments, the subject is a canine preclinical model such as a wild type dog or cxmd dog. Effective penetration and delivery of AAV vectors into skeletal muscles of dogs is achieved using an immunosuppressive regimen that is effective in permitting prolonged transgene expression in both WT and cxmd dogs following individual intramuscular AAV injection.

In some embodiments, the subject is a preclinical animal model. In some embodiments, the subject is one of a mouse model or rat model. A preclinical model may be an animal model that is the recipient of a xenograft or xenotransplantation, terms that are used interchangeably to refer to the transplantation of living cells, tissues or organs from one species to another. In some preferred cases, the preclinical model is the recipient of one or more cancer cells that develops into a tumor. The recipient preclinical model may be an immunocompromised animal, such as a SCID mouse or nude mouse. An athymic nude mouse is a laboratory mouse from a strain with a genetic mutation that causes a deteriorated or absent thymus, resulting in an inhibited immune system due to a greatly reduced number of T cells. An immunocompromised state in a preclinical model may be the result of genetic abnormalities, or it may be the result of drug treatments to suppress immune system function. Immunosuppressive drugs or immunosuppressive agents are drugs that inhibit or prevent activity of the immune system. Non-limiting examples of immunosuppressive drugs include glucocorticoids; cytostatics; alkylating agents; antimetabolites including folic acid analogues, such as methotrexate, and purine analogues such as azathioprine and mercaptopurine; azathioprine and mercaptopurine; cytotoxic antibiotics, including dactinomycin, anthracyclines, mitomycin C, bleomycin, and mithramycin; polyclonal and monoclonal antibodies targeting elements of the immune system; and drugs acting on immunophilins, including cyclosporin, tacrolimus, voclosporin and other calcineurin inhibitors, and sirolimus; interferons, opioids, TNF-binding proteins, mycophenolate, and fingolimod.

Certain other embodiments contemplate a non-human subject or biological source, for example a non-human primate such as a macaque, chimpanzee, gorilla, vervet, orangutan, baboon or other non-human primate, including such non-human subjects that may be known to the art as preclinical models, including preclinical models for solid tumors and/or other cancers. Certain other embodiments contemplate a non-human subject that is a mammal, for example, a mouse, dog, rat, rabbit, pig, sheep, horse, bovine, goat, gerbil, hamster, guinea pig or other mammal; many such mammals may be subjects that are known to the art as preclinical models for certain diseases or disorders, including solid tumors and/or other cancers (e.g., Talmadge et al., 2007 Am. J. Pathol. 170:793; Kerbel, 2003 Canc. Biol. Therap. 2(4 Suppl 1):S134; Man et al., 2007 Canc. Met. Rev. 26:737; Cespedes et al., 2006 Clin. TransL Oncol. 8:318). The range of embodiments is not intended to be so limited, however, such that there are also contemplated other embodiments in which the subject or biological source may be a non-mammalian vertebrate, for example, another higher vertebrate, or an avian, amphibian or reptilian species, or another subject or biological source. A transgenic animal is a non-human animal in which one or more of the cells of the animal includes a nucleic acid that is non-endogenous (i.e., heterologous) and is present as an extrachromosomal element in a portion of its cell or stably integrated into its germ line DNA (i.e., in the genomic sequence of most or all of its cells). In certain embodiments of the present invention, the tissue of a transgenic animal may be targeted. In some embodiments, the solid tissue is a xenograft produced by introducing one or more cells of one organism (e.g. cultured human cancer cells or primary human cancer cells) into a nonhuman model organism.

The method of the invention is suitable for administering gene replacement therapeutic agents to a variety of tissues; thus the method has medical and veterinary uses. In some embodiments, the animal is a pet, a companion, a guardian, a working animal, a breeding animal, a service animal, a racing animal, a farm animal, a herded animal, or a laboratory animal.

The subject or biological source can be a human or non-human animal, a transgenic or cloned or tissue-engineered (including through the use of stem cells) organism, a primary cell culture or culture adapted cell line including but not limited to genetically engineered cell lines that can contain chromosomally integrated or episomal recombinant nucleic acid sequences, immortalized or immortalizable cell lines, somatic cell hybrid cell lines, differentiated or differentiatable cell lines, transformed cell lines and the like. In some embodiments of the invention, the subject or biological source can be suspected of having or being at risk for having a malignant condition or muscle disease or disorder, and in some embodiments of the invention the subject or biological source can be known to be free of a risk or presence of such disease.

### Formulations

A fluid agent or a gene replacement therapeutic agent can comprise a pharmaceutically acceptable carrier, examples of which are well known in the pharmaceutical art, and are described, for example, in Remingtons Pharmaceutical Sciences. Mack Publishing Co. (A.R. Gennaro edit. 1985). For example, sterile saline and phosphate-buffered saline at physiological pH can be used. Preservatives, stabilizers, dyes and other ancillary agents can be provided in the pharmaceutical composition. For example, sodium benzoate, sorbic acid and esters of p-hydroxybenzoic acid can be added as preservatives. In addition, antioxidants and suspending agents can be used. Pharmaceutically acceptable salt refers to salts of drug compounds derived from the combination of such compounds and an organic or inorganic acid (acid addition salts) or an organic or inorganic base (base addition salts). The agents, including drugs, contemplated for use herein can be used in either the free base or salt forms, with both forms being considered as being within the scope of the certain present invention embodiments.

The agent can be in any form which allows for it to be administered to a subject. According to some embodiments the agent will be in liquid form and the route of administration will comprise administration to a tissue as described herein. The term parenteral as used herein includes, but is not limited to, transcutaneous or subcutaneous injections, and intramuscular, intramedullar and intrastemal techniques.

A liquid pharmaceutical composition as used herein, whether in the form of a solution, suspension or other like form, can include one or more of the following adjuvants: sterile diluents such as water for injection, saline solution, physiological saline, Ringer's solution, saline solution (e.g., normal saline, or isotonic, hypotonic or hypertonic sodium chloride), fixed oils such as synthetic mono or digylcerides which can serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. In some embodiments, physiological saline is the adjuvant. An injectable pharmaceutical composition can be sterile. It can also be desirable to include other components in the preparation, such as delivery vehicles including but not limited to aluminum salts, water-in-oil emulsions, biodegradable oil vehicles, oil-in-water emulsions, biodegradable microcapsules, hydrogels, and liposomes.

While any suitable carrier known to those of ordinary skill in the art can be employed in the pharmaceutical compositions of this invention, the type of carrier will vary depending on the mode of administration and whether a conventional sustained drug release is also desired. For parenteral administration, such as supplemental injection of drug, the carrier can comprise water, saline, alcohol, a fat, a wax or a buffer. Biodegradable microspheres (e.g., polylactic galactide) can also be employed as carders for the pharmaceutical compositions of this invention. Suitable biodegradable microspheres are disclosed, for example, in U.S. Patent Nos. 4,897,268 and 5,075,109. In some embodiments, the microsphere be larger than approximately 25 microns, while other embodiments are not so limited and contemplate other dimensions.

Pharmaceutical compositions can also contain diluents such as buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. In some embodiments, an agent (e.g., a therapeutic drug or a candidate drug) is formulated as a lyophilizate using appropriate excipient solutions (e.g., sucrose) as diluents.

In some embodiments, a pharmaceutical composition further comprises a hydrogel. The hydrogel is effective to slow the rate of diffusion or dispersion of a pharmaceutical formulation through a solid tissue. In some embodiments, a pharmaceutical composition containing the hydrogel disperses through a solid tissue to a lesser degree than does an analogous pharmaceutical composition lacking the hydrogel. In some embodiments, a pharmaceutical composition containing the hydrogel disperses through a solid tissue more slowly than does an analogous pharmaceutical composition lacking the hydrogel.

The hydrogel can be present in an amount from about 1% to about 99% of a pharmaceutical composition. In some embodiments, the hydrogel is present in an amount of about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.5%, about 6%, about 6.5%, about 7%, about 7.5%, about 8%, about 8.5%, about 9%, about 9.5%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% of a pharmaceutical composition.

Exemplary porous materials suitable for biological use are described in U.S. Pat. No. 4,014,335, which is incorporated herein by reference in its entirety. These materials include, but are not limited to, cross-linked polyvinyl alcohol, polyolefins or polyvinyl chmorides or cross-linked gelatins; regenerated, insoluble, non-erodible cellulose, acylated cellulose, esterified celluloses, cellulose acetate propionate, cellulose acetate butyrate, cellulose acetate phthalate, cellulose acetate diethyl-aminoacetate; polyurethanes, polycarbonates, and microporous polymers formed by co-precipitation of a polycation and a polyanion modified insoluble collagen.

In some embodiments, the hydrogel comprises collagen. Non-limiting examples of sources of collagen include Engelbreth-Holm-Swarm murine sarcoma basement membrane, bovine achilles tendon, bovine nasal septum, bovine tracheal cartilage, calf skin, chicken sternal cartilage, human lung , human placenta, kangaroo tail, mouse sternum, and rat tail tendon. Collagen can comprise more than 0.1%, 0.2%, 0.5%, 0.7%, 1%, 1.2%, 1.4%, 1.6%, 1.8%, 2%, or 5% of the hydrogel. Recombinant collagen can be used. Several sources of collagen are described in US Patent Application No. US20070254041. Collagen material that is insoluble in water can be used, and can be derived from natural tissue sources (e.g. xenogenic, allogenic, or autogenic relative to the recipient human or other patient) or recombinantly prepared. Collagens can be subclassified into several different types depending upon their amino acid sequence, carbohydrate content and the presence or absence of disulfide crosslinks. Types I and III collagen are two of the most common subtypes of collagen. Type I collagen is present in skin, tendon and bone, whereas Type III collagen is found primarily in skin. The collagen used in compositions of the invention can be obtained from skin, bone, tendon, or cartilage and purified by methods well known in the art and industry. Alternatively, the collagen can be purchased from commercial sources. Type I bovine collagen is preferred for use in the invention.

The collagen can be atelopeptide collagen and/or telopeptide collagen. Still further, either or both of non-fibrillar and fibrillar collagen can be used. Non-fibrillar collagen is collagen that has been solubilized and has not been reconstituted into its native fibrillar form.

Suitable collagen products are available commercially, including for example from Kensey Nash Corporation (Exton, Pa.), which manufactures a fibrous collagen known as semed F, from bovine hides. Collagen materials derived from bovine hide are also manufactured by Integra Life Science Holding Corporation (Plainsboro, N.J.). Naturally-derived or recombinant human collagen materials are also suitable for use in the invention. Illustratively, recombinant human collagen products are available from Fibrogen, Inc. (San Francisco, Calif.). The solid particulate collagen incorporated into the inventive compositions can be in the form of intact or reconstituted fibers, or randomly-shaped particles, for example.

Collagen can be dissolved in water to form an aqueous solution at room temperature, but undergoes polymerization to form a gel at 37 degrees. Miyata notes in US Pat. No. 4,164,559 that the chemistry, molecular structure and biochemical properties of collagen have been well established (Annual Review of Biophysics and Bioengineering, Vol. 3, p. 231-253, 1974). Collagen is a major protein of connective tissue such as cornea, skin, etc., and can be solubilized and purified by the treatment with proteolytic enzymes (other than collagenase) such as pepsin. Solubilized collagen is telopeptides-poor, relatively inexpensive, not antigenic and useful as a biomedical material. Enzyme solubilized native collagen is soluble in acidic pH but polymerizes to form a gel at physiologic pH and at 37 degrees.

In other embodiments, the hydrogel comprises polyethylene glycol (PEG), in various formulations known in the art. Non-limiting examples of polymers that can be present in PEG hydrogels include polylactic acid (PLA), poly(lactic-co-glycolic acid) (PLGA), and polycaprolactone (PCL). Some examples of these copolymers include PLA-PEG-PLA, PLGA-PEG-PLA, and mPEG-b+PCL(1200)-b-PEG(6000)-b-PCL(1200) copolymer. PLGA is a copolymer which is used in a host of Food and Drug Administration (FDA) approved therapeutic devices, owing to its biodegradability and biocompatibility. PLGA is synthesized by means of random ring-opening co-polymerization of two different monomers, the cyclic dimers (1,4-dioxane-2,5-diones) of glycolic acid and lactic acid. Depending on the ratio of lactide to glycolide used for the polymerization, different forms of PLGA can be obtained: these are usually identified in regard to the monomers' ratio used (e.g. PLGA 75:25 identifies a copolymer whose composition is 75% lactic acid and 25% glycolic acid). All PLGAs are amorphous rather than crystalline and show a glass transition temperature in the range of 40-60 degrees. There is very minimal systemic toxicity associated with using PLGA for drug delivery or biomaterial applications. PLA is a biodegradable, thermoplastic, aliphatic polyester derived from renewable resources, such as corn starch, tapioca products, or sugarcanes. PCL is a biodegradable polyester with a low melting point of around 60°C and a glass transition temperature of about -60°C. PCL is prepared by ring opening polymerization of e-caprolactone using a catalyst such as stannous octanoate. PCL is an FDA-approved material that is used in the human body, and undergoes slow degradation upon implantation.

### Dosing and Administration

A drug-delivery device comprising hollow and/or porous tubes of the invention is useful for methods of administering therapeutic or candidate therapeutic agents to a subject by depositing one or more porous tubes packed with one or more therapeutic agents in the a tissue of the subject. A tube can be partially- or fully-submerged in the tissue. Deposition of tubes into the tissue can be facilitated by inserting the tube into the tissue via a needle. The tube can be further deposited into the tissue by depression of a plunger associated with the needle.

After deposition of the tube into the tissue, the tube can be broken, cut, sliced, disjoined, or separated to remove the top end of the tube form the bottom end of the tube. The bottom end remains deposited in the tissue, whereas the top end is removed from the tissue. In some embodiments, the bottom end of the tube contains a therapeutic agent and the top end of the tube does not contain a therapeutic agent. In some embodiments, both the bottom end and the top end of the tube contain a therapeutic agent.

Once the tube has been deposited into the tissue, the contents of the tube (for example, a pharmaceutical composition or a therapeutic agent) can diffuse from the tube into the tissue. The process is illustrated in Figure 1. The rate of diffusion can be influenced by the porosity of the tube. The contents can diffuse through the tube material into the tissue over a period of about a minute to about a month; about an hour to about a week; about 12 hours to about 72 hours; or about 24 hours to about 48 hours.

If the contents of the tube contained a dye, the dye can be imaged within the tissue to monitor the disbursement or activity of a therapeutic agent. A dye can be chosen to report, for example, by fluorescence, the activation or inactivation of a biological function upon imaging. This process allows an experimenter to make a direct assessment of the affect of a therapeutic agent on a physiological system of interest.

Generally, rAAV virions may be introduced into a muscle cell using either in vivo or in vitro transduction techniques known in the art. If transduced in vitro, the desired recipient muscle cell will be removed from the subject, transduced with rAAV virions and reintroduced into the subject. Alternatively, syngeneic or xenogeneic muscle cells can be used where those cells will not generate an inappropriate immune response in the subject.

Suitable methods for the delivery and introduction of transduced cells into a subject have been described. For example, cells can be transduced in vitro by combining recombinant AAV virions with muscle cells e.g., in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using e.g., a needle array device.

For in vivo delivery, the rAAV virions can be formulated into pharmaceutical compositions and will generally be administered parenterally, e.g., by intramuscular injection directly into skeletal or cardiac muscle. Pharmaceutical compositions will comprise sufficient genetic material to produce a therapeutically effective amount of the protein of interest, i.e., an amount sufficient to reduce or ameliorate symptoms of the disease state in question or an amount sufficient to confer the desired benefit. The pharmaceutical compositions will also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, liquids such as water, saline, glycerol and ethanol.

Appropriate doses will depend on the mammal being treated, age and general condition of the subject to be treated, the severity of the condition being treated, the particular therapeutic protein in question, its mode of administration, among other factors. An appropriate effective amount can be readily determined by one of skill in the art. Thus, a therapeutically effective amount will fall in a relatively broad range that can be determined through clinical trials. For example, for in vivo injection, i.e., injection directly to skeletal or cardiac muscle, a therapeutically effective dose will be on the order of about 106, 107, 108, 109, 1010, 1011, 1012, 1013, 1014, or 1015 of the rAAV virions. For in vitro transduction, an effective amount of rAAV virions to be delivered to muscle cells will be on the order of 108, 109, 1010, 1011, 1012, or 1013 of the rAAV virions. The amount of transduced cells in the pharmaceutical compositions will be from about 104, 105, 106, 107, 108, 109, 1010, or 1010 muscle cells. When the transduced cells are introduced to vascular smooth muscle, a lower dose may be appropriate. Other effective dosages can be readily established by one of ordinary skill in the art through routine trials establishing dose response curves.

Dosage treatment may be a single dose schedule or a multiple dose schedule. Moreover, the subject may be administered as many doses as appropriate. One of skill in the art can readily determine an appropriate number of doses.

### Methods for Gene Therapy

Animal model, anesthesia, and immunosuppression: Wild type research dogs can be subjects of the present disclosure. Needle arrays can be used in conjunction with local anesthesia (e.g., with lidocaine) or general anesthesia. General anesthesia can involve Butorphanol, Diazepam/ Ketamine, Propofol and Isoflurane and animals can be monitored by methods known in the art. A dog can be immunosuppressed with cyclosporine (CSP), a calcineurin inhibitor given 2 days before vector injection and extended through week 6 at 7.5 mg/kg orally twice daily and mycophenolate mofetil (MMF), an antimetabolite administered at a dose of 5 mg/kg s.q. 2 times daily from day 0 through experiment completion.

Needle Manufacturing: 30 mm long needles with 5 mm porous region starting 0.5 mm from the needle tip can be employed in embodiments of the disclsoure. The needles can be composed of surgical grade stainless steel tubing that has an external diameter of 0.46 mm and an internal diameter of 0.25 mm, the pores can be 0.15 mm in diameter and spaced 0.25 mm apart. The pores can be established by electrical discharge machining (EDM). The stainless steel tubing can be rotary welded to seal the tip and the tip can be ground to a pencil-tip point that has a maximum flange angle of 48 degrees with a tip that does not vary from center by more than 0.06 mm. The custom made porous needles can be internally quality controlled (QC) by visual inspection under microscopy for size, length, tip geometry, pore size and pore distribution per standard operating procedure.

Array Head Design and Manufacturing: Needle array heads for dog muscle injection can be made to be compatible with needles, tubing, and pumps used for mouse model oncology studies. Designs can minimize the number of connections in the circuit and minimize abrupt change in internal diameter along the fluid path to minimize air bubble formation and turbulence in fluid. The hub of the array head can be made from medical grade polysulfone polymer will have an ovoid shape of roughly 3 x 9 cm and 1-1.5 cm height for ease of handling. The needles can be fitted and held in an axial opening through the hub and then can be fixed using medical grade adhesive. Final QC, packaging, and sterilization can be performed on all array heads.

Syringe Pump: A pump such as the Harvard Apparatus PHD Ultra syringe pump can be used to deliver accurate volumes against back pressure of less than about 1000 psi, with a flow rate range of about 0.0001 µl/hr to 221 ml/hour. 500 µl syringes can be used to deliver between about 1.5 nl/min to about 1.6 ml/min. Polyvinyl chloride (PVC) tubing that has a diameter of about 0.25 mm and wall thickness of about 1.6 mm is suitable for delivery of fluids at the desired rate. The tubing material can be selected for solvent resistance and suitability for FDA approval and human clinical use.

Infusion concentration, volume, and rate: Needles of the disclosure can deliver 5 x 1011 vector genome in 25, 50, 75, 100, 150, 200 or 250 µl volumes of Hank's buffered salt solution over about 1, 2, 3, 4, 5, 6, or 10 minutes or in less than about 30 seconds. Each muscle can receive a single porous needle injection. Non-absorbable sutures can be placed at sites of injection as markers.

Muscle biopsies and tissue analysis: Muscle biopsies can be obtained at various time points to assess, e.g. Factor IX expression. Muscle tissues can be embedded in O.C.T. medium (Tissue-Tek, Hatfield, PA), and consecutive cryostat sections can be made for histology evaluation using hematoxylin and eosin-phloxine (H&E) staining; for detection of canine factor IX expression, in which rabbit anti-canine Factor IX (Affinity Biologicals, Ontario, Canada) can be used as primary antibody. Muscle biopsies taken from buffer-injected sites can serve as negative controls. Muscle fibers expressing cFIX can be counted within 1cm2 areas and the percentage of the cFIX positive muscle fibers can be calculated and compared among different delivery regimens.

### Data Acquisition and Analysis

In some embodiments it is contemplated that the target region in a solid tissue can be imaged using known techniques to evaluate the effects of the agents. The imaging can be by any suitable process or method, including, for example, radiographic imaging, magnetic resonance imaging, positron emission tomogoraphy, biophotonic imaging, etc. In some embodiments, the target region can be imaged repeatedly before, during, and after the delivery process.

Upon imaging, the level of the reporting signal can be quantified by methods known to one of skill in the art. Observation and/or quantification of the reporting signal can be used to make informed research and health care decisions regarding the use and efficacy of a therapeutic agent. Non-limiting examples of decisions that can be made on such observations include fluid volume quality control, positional tracking, and drug biodistribution. Such experiments can be performed on a lower mammal, for example, a mouse, to provide reporting signals that can be used to make informed predictions regarding the activity of a potential therapeutic agent in a human. Animal studies of this type can be used to avoid the inherent uncertainty and inaccuracies that arise by conducting drug efficacy studies in cells in controlled environments instead of in the native environment.

Quantification of fluorescence signals can be accomplished by any method known in the art. Fluorescence signals can be compared with a standard or a control to determine up-regulation or down-regulation of a biological pathway. Such observations can be used to make predictions regarding the therapeutic value of drug candidates.

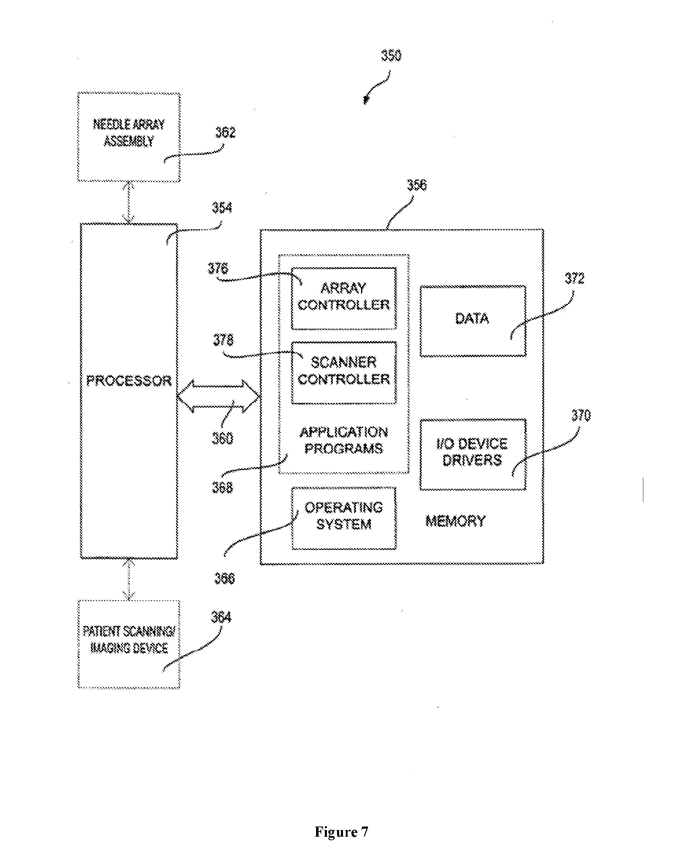

According to the embodiment of Figure 7, a data processing system 350 is used to carry out or direct operations, and includes a processor 354 and a memory 356. The processor 354 communicates with the memory 356 via an address/data bus 360 and also communicates with a needle array assembly 362 and a subject scanning device 364. The subject scanning device 364 is used, according to an embodiment, to assist in placing the needles of the needle array assembly 362 in a subject *in vivo* and for non-invasive analysis of target tissue regions using imaging techniques, such as radiographic imaging or nuclear medical assays. The processor 354 can be a commercially available or custom microprocessor, microcontroller, signal processor or the like. The memory 356 can include any memory devices and/or storage media containing the software and data used to implement the functionality circuits and modules.

The memory 356 can any of include several categories of software and data used in the data processing system, such as, for example, an operating system 366, application programs 368; input/output device drivers 370; and data 372. The application programs 368 are illustrative of the programs that implement the various features of the circuits and modules according to some embodiments, and the data 372 represents the static and dynamic data used by the application programs 368, the operating system 366, the input/output device drivers 370 and other software programs that can reside in the memory 356.

According to various embodiments, the data processing system 350 can include several modules, including an array controller 376, a scanner controller 378 and the like. The modules can be configured as a single module or additional modules otherwise configured to implement the operations described herein. For example, the array controller 376 can be configured to control the needle array assembly 100 of Figure 2, by controlling the actuators 116, and consequently, the release of therapeutic agents from the reservoirs 114 via the needles 112. The scanner controller 378 can be configured to control the subject scanning device 364.

In some embodiments, detection in a solid tissue of an altered physiologic state subsequent to introducing an agent or a plurality of agents includes detecting a degree of permeation of the agent(s) through the solid tissue, detecting a degree of absorption of the agent(s) in the tissue, detecting a physicochemical effect of the agent(s) on the tissue, and/or detecting a pharmacological effect of the agent(s) on the tissue. Assays, including fluorescence assays, of drug permeation or penetration in solid tissues are known in the art and have been described *(e.g.,* Kerr et al., 1987 *Canc. Chemother. Pharmacol*. 19:1 and references cited therein; Nederman et al., 1981 *In Vitro* 17:290; Durand, 1981 *Canc. Res.* 41:3495; Durand, 1989 *JNCI* 81:146; Tunggal et al., 1999 *Clin. Canc. Res.* 5:1583) and can be configured further according to the present disclosure, for instance, through the detection in histological serial sections of a detectable label that has been co-administered to the solid tissue, prior to excision and sectioning, with an agent of interest.

In such embodiments, permeation or penetration refers to the area of retention of an agent in the solid tissue in the immediate vicinity of the needle from which the agent was introduced exclusive of perfusion (entry into and dispersion via any blood vessel), and can include retention of the agent in extracellular space or extracellular matrix or in association with a cell membrane or intracellularly. Permeation can be distinct from a physicochemical effect, which refers to microscopically detectable mechanical disruption of tissue that results from the needle insertion or fluid injection itself, or from non-biological mechanical or chemical tissue disruption caused by the agent (*e.g.*, damage to cell membranes or disintegration of cell-cell junctions). Pharmacological effects include statistically significant alterations of a cell or tissue physiological state that are detectable as consequences of the molecular mechanism of action of the agent, for example, cytoskeletal reorganization, extension or withdrawal of cellular processes, or evidence of biological signal transduction as can be detected using any of a number of known cytological, biochemical, molecular biological or other read-outs. Comparison of serial sections can permit distinguishing the nature of the effect that is detected histologically.

In some preferred embodiments of the present disclosure, a porous needle array is used to deposit a plurality of compositions comprising a hydrogel and a plurality of candidate therapeutic agents along parallel axes within a tissue. In some cases, the composition further comprises a dye that may be useful in indicating, for example, position, permeation, status of a signaling pathway, or delivery into cells. In some preferred embodiments, the tissue is a tumor. A tumor may be excised following delivery of a plurality of compositions and sectioned, and the individual sections may be imaged using brightfield or fluorescence microscopy to determine, for example, the relative efficacies of the plurality of candidate therapeutic agents.

### EXAMPLES

### Example 1: Spatially Restricted Delivery of Dye at Multiple Tumor Depths

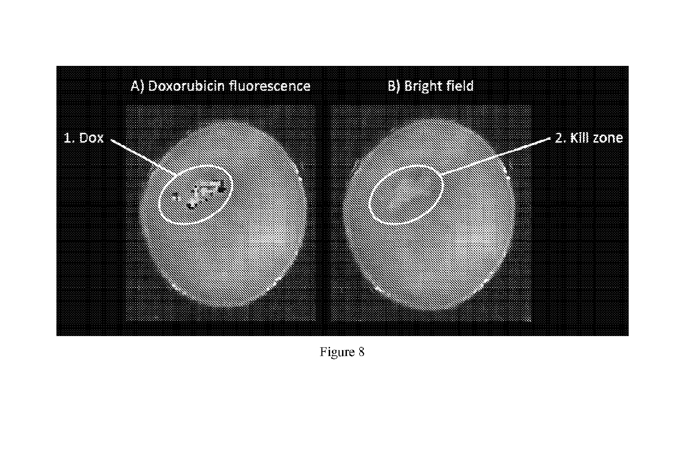

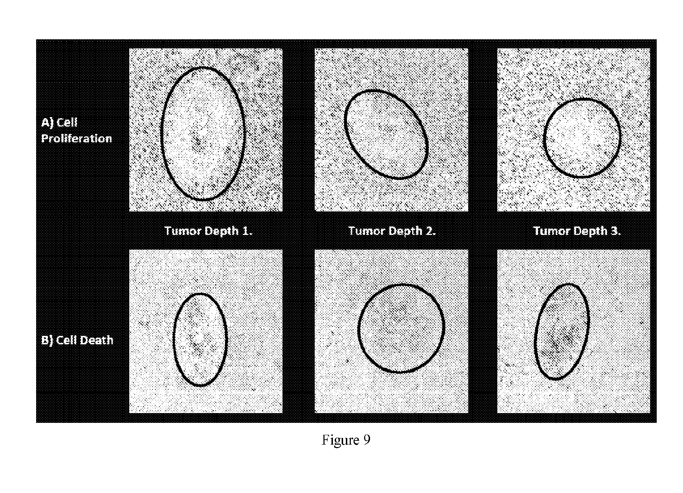

Doxorubicin was delivered to a lymphoma tumor using a porous tube of the method. The tumor was then excised and sectioned. Figure 8 shows a slice of the tumor, imaged using fluorescence and brightfield microscopy. These images show that doxorubicin fluorescence overlaps with the region of dead cells discernible in the brightfield image. Regions of doxorubicin fluorescence and cell death are localized to a zone within the tumor slice, reflecting spatially constrained doxorubicin delivery. Figure 9 shows three tumor cross-sectional slices from different depths, and demonstrates that the localized delivery depicted in Figure 8 extends to various tumor depths. Cell death was observed in a localized area across the three tumor depths shown.

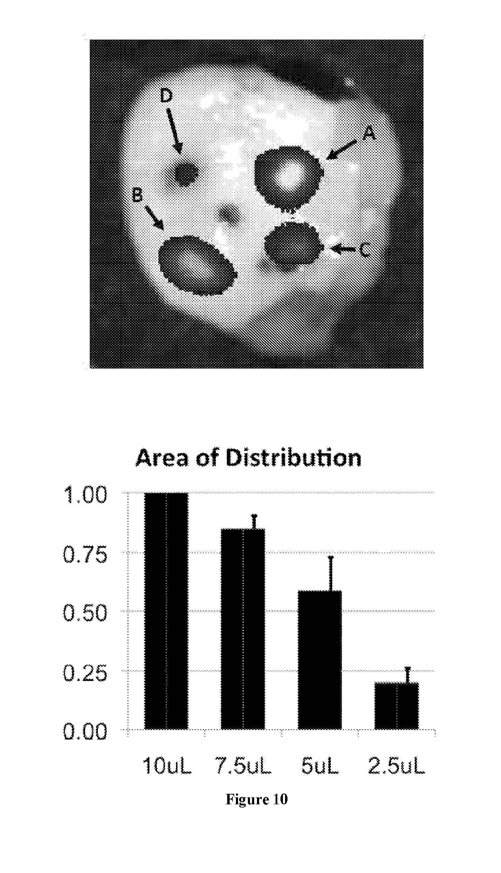

Spatially restricted delivery was tested by injecting four different volumes of a fluorescent dye using a needle array. The dye was injected along four parallel axes within a tumor. Figure 10 illustrates fluorescent microscopy of these injections, and the resulting spatially-restricted distribution of fluorescent dye (top panel). The injections were A) 10 µL; B) 7.5 µL; C) 5 µL; and D) 2.5 µL. The graph in the bottom panel depicts the relative areas of distribution, averaged over 15 sections from different tumor depths, for each injection.

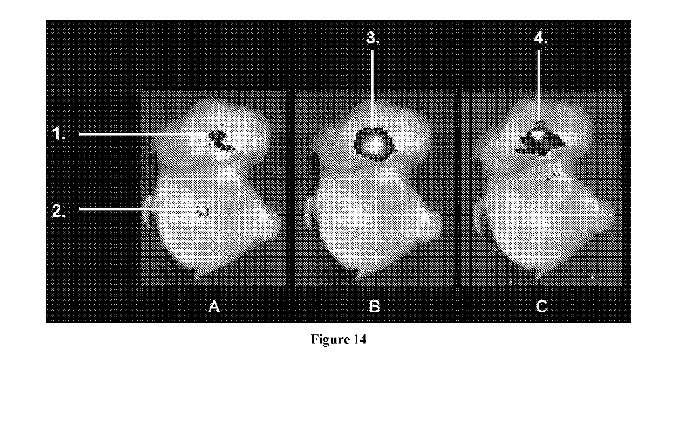

Figure 14 depicts the use of various indicator dyes according to the method, to monitor spatially restricted delivery of compounds and resulting localized effects on regions of a tumor. A mouse tumor was injected with 1. doxorubicin; and 2. a control. Panel A illustrates excitation at 640 nm and emission at 800 mm of the dye. Panel B illustrates the doxorubicin signal (3.) observed after excitation at 500 mm and emission at 600 nm of doxorubicin. Panel C illustrates an apoptosis signal (4.) observed after excitation at 640 nm and emission at 720 nm of the dye. The results show that apoptosis overlaps with the region that received doxorubicin, but not control, demonstrating spatially restricted drug delivery and tumor cell killing.

### Example 2: Comparison of in vivo and in vitro analyses

Sonic hedgehog (Shh) antagonists were tested in vitro and in vivo, and the results were compared.

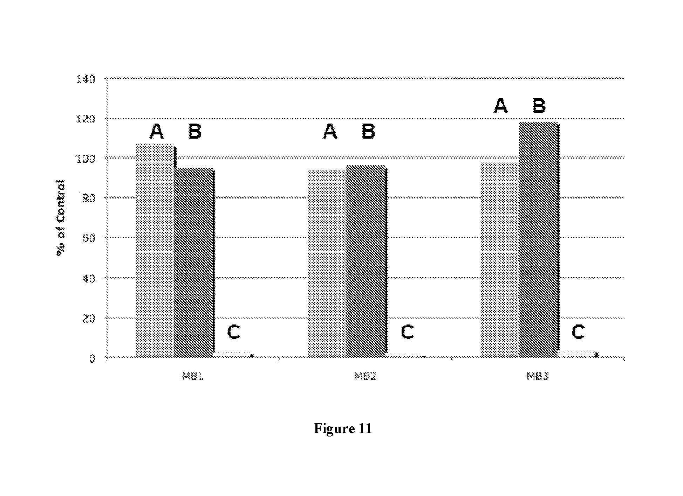

Figure 11 illustrates an in vitro response to hedgehog pathway antagonism in a human medulloblastoma sample. Medulloblastoma cells were taken from three patients and cultured in vitro. Samples from the three patients, MB1-MB3, were tested for the effect of Shh antagonists, which showed no response compared to positive control in this study. Bars A) depicts injection of 1 µM of SHH antagonist; Bars B) injection of 5 µM SHH antagonist; and Bars C) injection of a positive control.

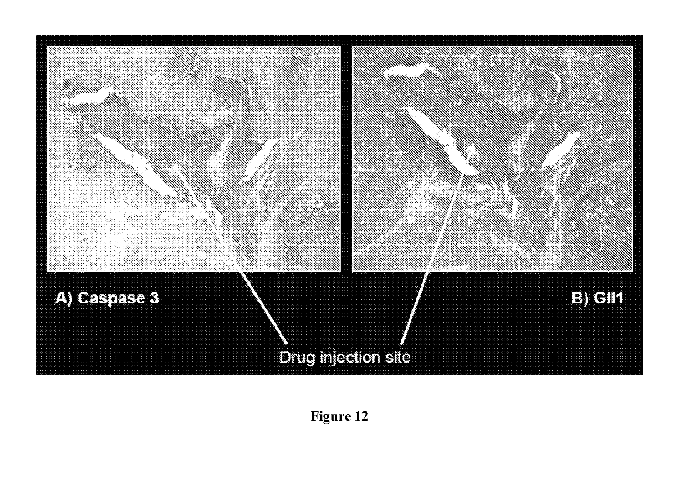

In contrast to the results of the in vitro experiment shown in Figure 11, Figure 12 illustrates the response of Shh antagonist injection to a tumor in vivo. Shh antagonists were injected in the tumor in a spatially-restricted fashion, using the method of the invention, and visualized by fluorescent microscopy. Figure 12 shows brightfield microscopy of localized positive signal for A) caspase 3; and B) Gli1, illustrating spatially restricted tumor kill in both cases.

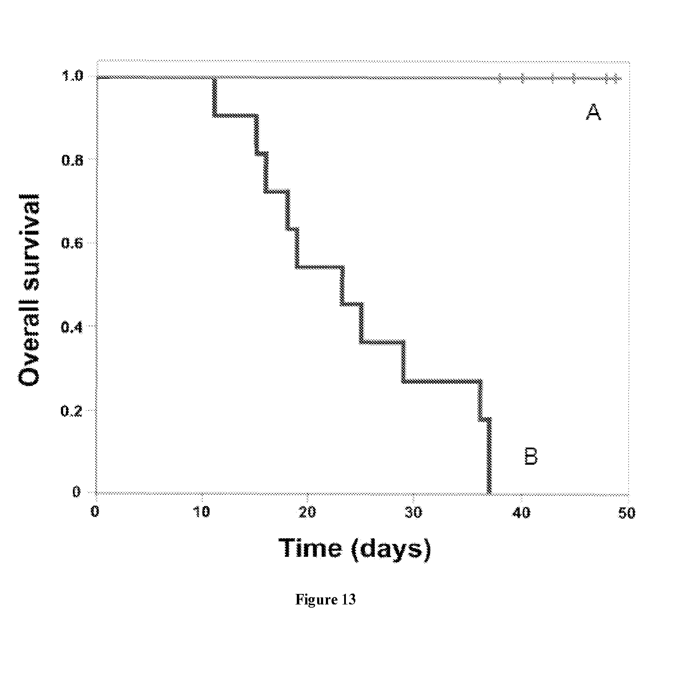

The results of the in vivo experiment depicted in Figure 12 predicted that Shh antagonists would have a positive effect on a mouse model of cancer. Intracranial medulloblastoma model (conditional Patched1 null) mice used in this experiment develop medulloblastoma with an early onset and 100% penetrance. Accordingly, mice were injected daily with 20 mg/kg of either A) vehicle plus IPI-926 (n=12); or B) vehicle only (n=11). The mice were monitored for 50 days for survival, and the results depicted in Figure 13. The experiment showed that mice given IPI-926 drug had significantly increased survival compared to the control mice. The results of this experiment demonstrate that Shh antagonism does effect cancer progression in vivo, as predicted by the in vivo experiment of Figure 12, but not by the in vitro experiment of Figure 11.

### Example 3: Spatially-restricted Delivery of Nucleic Acids

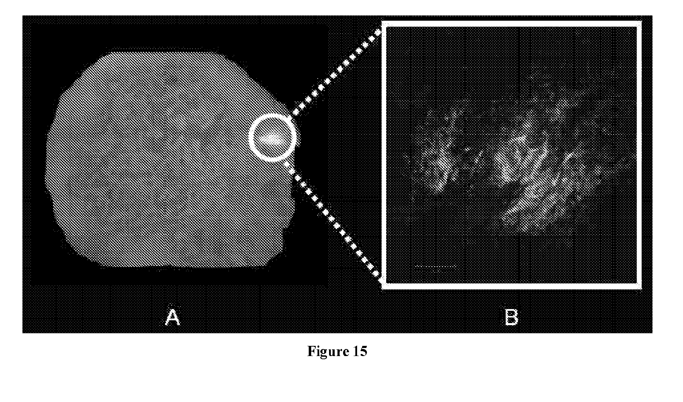

Spatially-restricted delivery of nucleic acid molecules was tested using the present method. A HT29 colon tumor xenograft was injected with lentivirus bearing a promoter driving GFP expression. Figure 15 illustrates fluorescent microscopy of a whole tumor slice following spatially-restricted injection of GFP-expressing lentivirus. Panel A shows that GFP expression was localized to the region of injection. Panel B shows magnification of the virus infusion zone.

The method was then applied for spatially restricted RNA interference (RNAi). A small hairpin RNAi (shRNA) construct within a lentivirus was locally delivered to a mouse tumor. The shRNA was directed against KIF11, an essential gene for tumor cell mitosis. A control construct with no knockdown ability was also used. As an additional control, GFP virus alone was injected. These three constructs were injected at three different locations within the tumor, and localized effects were observed. In all three cases, the apoptosis reporter near-infrared tagged annexin 5 (Visen) was co-injected together with the constructs. Following injection, the tumor was excised, sectioned, and visualized by fluorescent microscopy to observe the apoptosis reporter.

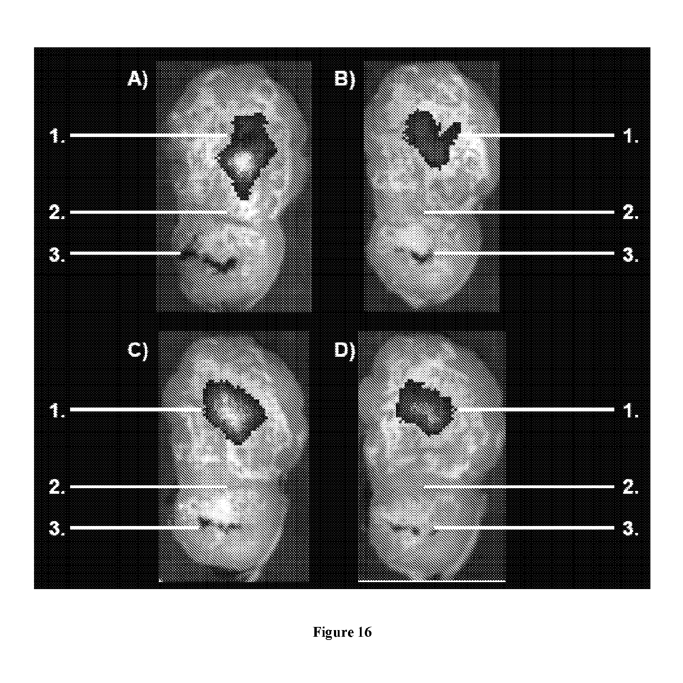

Figure 16 depicts the results of this analysis, showing 1. KIF11 shRNA with an apoptosis reporter; 2. Control virus with an apoptosis reporter; and 3. GFP virus with an apoptosis reporter. Scans were taken at four tumor depths: 500 microns; 1,000 microns; 1,500 microns; and 2,000 microns. Only the region that received KIF11 shRNA shows a positive readout for apoptosis, indicating that tumor cell killing correlated with knockdown of KIF11, and was spatially restricted to the region that received the KIF 11 shRNA construct.

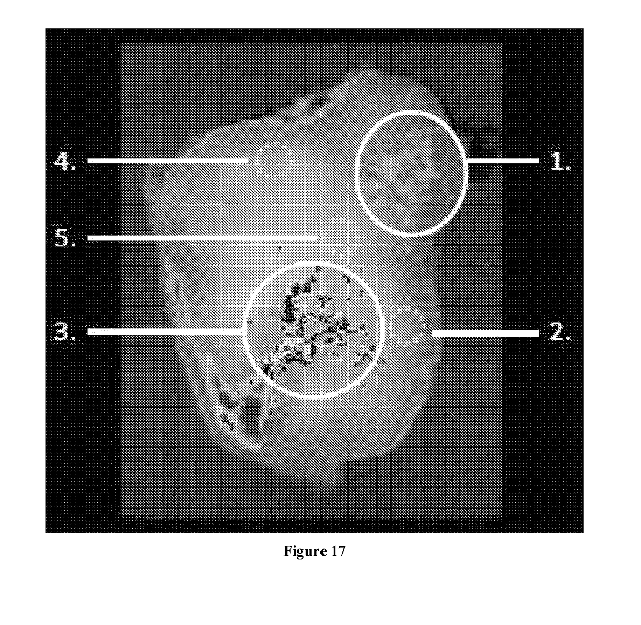

Spatially restricted tumor cell killing is also shown in Figure 17. A mouse tumor was injected with 1. KIF11 shRNA; 2. Control; 3. KIF11 shRNA and an apoptosis dye (near infrared-tagged annexin 5); 4. Control; and 5. Control and the apoptosis dye. The tumor was excised, sectioned, and visualized by fluorescent microscopy. The results show that spatially restricted delivery of KIF11 shRNA, but not control, results in a signal observable using an apoptosis dye. No background signal was observed when KIF11 or control constructs were administered without the apoptosis dye.

### Example 4: Assess Tumor Model/Porous Needle Array Compatibility

All potential cell lines can be grown as xenografted tumors in athymic nude mice and assessed for compatibility with injection by the needle array device. Breast, lung, and colon tumor lines known to harbor either activation mutations of the EGF receptor (e.g. HCC827 lung carcinoma), RAS (e.g. A549 lung carcinoma, HCT-116 colon carcinoma), or PI3K (HCT-116 colon carcinoma, MCF7 breast carcinoma, T47D breast carcinoma), or deactivating mutations of the tumor suppressor PTEN (MDA-MB-468 breast carcinoma, EVSA-T breast carcinoma) can be employed. Cell lines that can be used include A2058 melanoma (braf, pten), LnCAP prostate carcinoma (kras, pten), H2122 lung carcinoma (kras, sty11), and MCF7 breast carcinoma (pik3ca, cdkn2a). To best approximate the tissue environment from which the tumor cells are derived, tumor models can be orthotopically transplanted when possible.

Tumor characteristics such as growth rate, size, shape, symmetry, solidity, and accessibility all influence the ease with which agents can be delivered to tumor subportions through the needle array head. To date, most xenografted tumors (>80%) have been found to be compatible with injection. Some tumors, however, harbor characteristics (e.g. large intratumor cysts) that preclude arrayed injection. In order to avoid potential waste of time and resources spent on generating reporter lines that will ultimately not be usable in the context of our platform, each tumor type can be grown as a xenografted tumor until it reaches a size compatible with injection by the porous needle array device (∼1.0 - 1.5 mm³). The tumor can then be injected at five geometrically defined positions with a near infrared tracking dye. Twenty four hours later the tumor can be resected, formalin fixed, and sectioned using a vibratome. Two hundred micron thick sections can then be analyzed on an IVIS Imaging System (Caliper LifeSciences, Inc.) to assess the biodistribution of the injected tracking dye. If tumor take rate exceeds 50% and spatially restricted distribution of the dye (< 400 cell layers perpendicular to the injection column) is clearly observed at all five injected positions, then the tumor line is deemed compatible with our platform and can be used to generate cancer pathway specific reporter models.

### Example 5: Generation of Reporter Cell Lines

Tumor cell lines can be transduced with an optimized gene trap vector and subjected to customized selection protocols to yield robust cancer pathway specific reporter cells. The employed vector contains a promoterless trapping cassette consisting of splice acceptor and splice donor sequences that flank genes encoding Gaussia luciferase (reporter) and Neo (selectable marker). When integrated into a gene, expression of the Gaussia luciferase reporter is regulated by endogenous promoters, enhancers, and insulators. Accordingly, reporter activity accurately indexes expression of the endogenous gene. Gaussia luciferase is ideally suited for in vivo tumor platforms. It is very bright (100 - 1000x > firefly luciferase) and ATP-independent. A humanized, membrane-anchored, and extracellularly displayed Gaussia luciferase has been engineered (Xactagen, LLC). The engineered luciferase retains high flash activity and is stabilized for glow applications as well. Membrane-anchored Gaussia luciferase has been shown to outperform the naturally secreted Gaussia luciferase for in vivo imaging, permitting real-time visualization of pathway inhibitory events in live animals upon introduction of the luciferase substrate coelenterazine. Cell surface expression of this form of luciferase also facilitates in vitro selection strategies. Trapping events that occur within genes that are highly upregulated by pathway inhibition can result in luciferase positive cells, which can be isolated by methods such as flow assisted flow sorting (FACS).

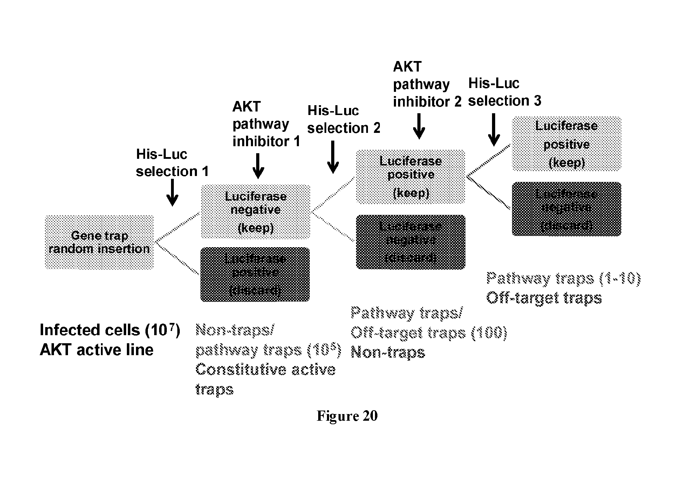

The strategy for selection-based generation of reporter cell lines is illustrated in Figure 20. The gene trap vector can be introduced to large populations of AKT active cells as packaged lentiviral particles. Cells can be subjected to luciferase-based selection. The first round eliminates traps that constitutively express luciferase. Next, cells can be exposed to an AKT inhibitor and subjected to a second round of selection. This round can select cells that express luciferase in response to the inhibitor and eliminate cells that do not harbor traps in functioning gene. A final round of selection with a structurally unrelated AKT inhibitor selects traps that are pathway-specific and eliminates traps that respond to a molecule-specific activity unrelated to the AKT pathway.

Sub-lethal concentrations of Gefitinib (Iressa), MK-2206, and PD325901, can be used to select EGF, PI3K-AKT, and Ras pathway inhibitor responsive reporters, respectively. Cells can be removed from drug postselection to minimize potential growth inhibitory effects on traps of interest. The selection strategy also takes into account that the inducible gene traps are responding to off-target activity of the agent used in the selection process, unrelated to inhibition of the oncogenic pathway of interest. Counter-screening of the original pooled population of positive inducible reporter cells with a second, structurally unrelated pathway inhibitor can remove these off-target gene traps. Cells that retain inducible expression of luciferase in response to the second inhibitor can be cloned and expanded for further analysis.

### Example 6: Screening of Reporter Tissues and Comparison of in vivo and ex vivo Analyses

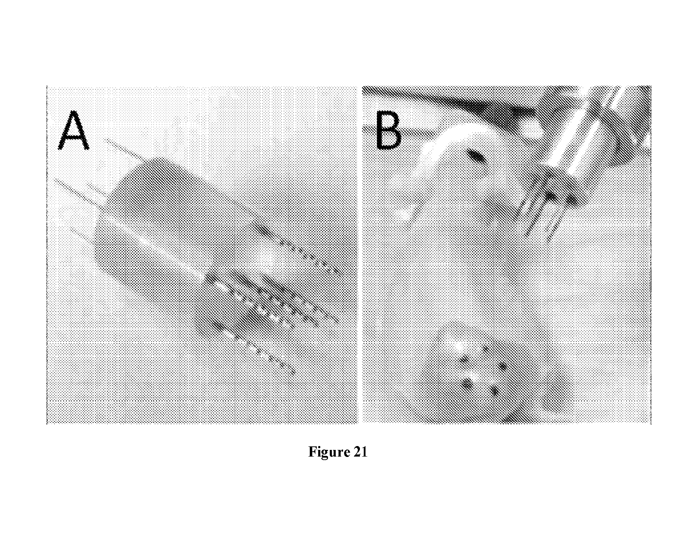

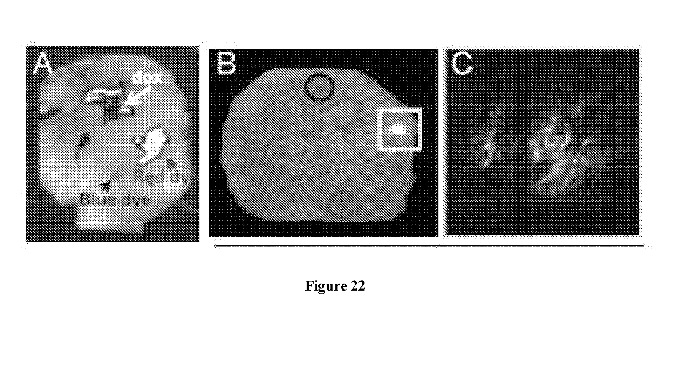

Orthotopic xenografts can be established in Nu/Nu (nude) athymic mice using a reporter line selected based on robust signal intensity in response to pathway inhibition. The tumors can be permitted to grow until they are approximately 1.5 - 2 cm in diameter. A positive control pathway inhibitor (e.g. an AKT, MEK, or EGFR inhibitor) can be loaded into one of the five needles of the porous needle array device. As additional positive controls, RNAi agents (primarily lentiviral shRNA vectors), validated for efficient silencing of AKT, MEK, and the EGFR can also be employed. Pathway inert reagents can be loaded as negative controls. Test compounds can be loaded into the remaining needles. Spatially-constrained injection of dyes using a porous needle array is shown in Figure 21. Both chemical compounds and lentiviral vectors have been successfuly delivered to spatially restricted portions of tumors using the porous needle array device, as shown in Figure 22.

The system is loaded until the samples are extruded from the porous needles. The needle head can be injected into the tumor of an anesthetized mouse and 7 microliters of drug plus near-infrared tracking dye, can be injected over the course of 5 minutes to prevent reflux along the needle track. One hour following tumor injection, mice can be injected with coelenterazine (7.7 mg/kg), and tumors can be examined immediately, and every hour for the next 6 hours for light emission resulting from induced luciferase expression using an IVIS. Light emission can be quantified as photons/minute using Living Image software. At the end of the 6 hour period, the tumor can be resected and reanalyzed using the IVIS to assess the difference in signal intensity, if any, between in vivo and ex vivo readings. The experiment can be performed on multiple xenografted mice.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein can be employed in practicing the invention. It is intended that the following claims define the scope of the invention and that methods and structures within the scope of these claims and their equivalents be covered thereby.

### Example 7: Porous needle mediated injection of GSK3 inhibitors induces b-catenin driven luciferase activity in tumors

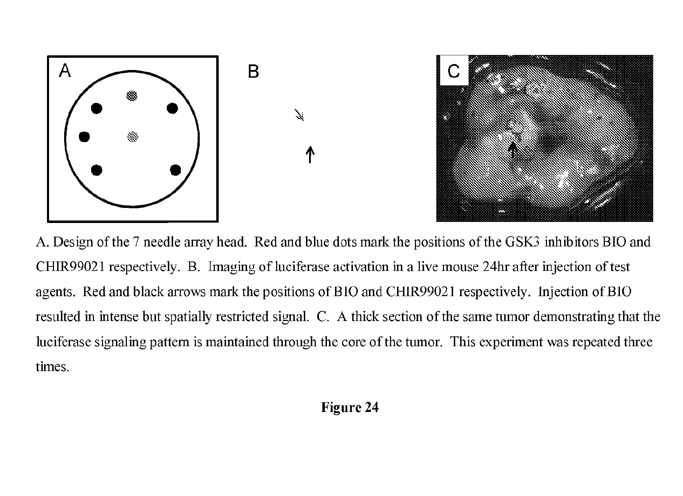

In this example, we used a well-characterized b-catenin reporter that expresses luciferase in response to WNT pathway activation (Figure 24). Tumor cells harboring this reporter were grown as xenograft tumors on the flanks of nude mice. In the absence of WNT stimulation, or exposure to drugs that inhibit negative regulators of b-catenin (such as GSK3b), the activity state of b-catenin in these tumors would be very low. We therefore used this model to test whether we could detect focal activation of the b-catenin reporter in tumors that were injected with two distinct GSK3 inhibitors. Using a 7-porous needle array the two GSK3 inhibitiors were in parallel and compared to injection of five pathway inert test agents. Following injection, animals were returned to their cages for 24 h prior to intraperitoneal injection with luciferin. Imaging of live animals on a Xenogen IVIS revealed two distinct regions of luciferase activity that corresponded to the precise locations of the GSK3 inhibitors (Figure 24). No signal above background was observed at the other five injection sites. Thick sections of the tumor expose to luciferin also demonstrated spatially restricted regions of lucifease activity induced by GSK3 inhibition. The ability to achieve discreet non-overlapping areas of luciferase signal is consistent with the potential to scale up our in vivo multi-comparison analysis to hundreds of samples for comprehensive screen hit follow up in animal models of cancer. We are confident that comparable results will be achieved with gene trap-derived pathway reporters.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### STATEMENTS OF THE INVENTION

1. An administration device comprising
   a) an array of needles positioned for delivery of a plurality of fluid agents along parallel axes within a tissue; and
   b) one or more fluid agents and a hydrogel, wherein the agents are formulated to disperse through the tissue to a lesser degree than the same fluid agents lacking the hydrogel upon delivery to the tissue.
2. The administration device of paragraph 1, further comprising an actuator associated with the array of needles, and the fluid agents are dispensed via the actuator.
3. The administration device of paragraph 1, wherein the actuator comprises a plunger.
4. The administration device of paragraph 1, wherein the actuator comprises a pump.
5. The administration device of paragraph 1, wherein the array of needles comprises about 1 to about 1,000 needles.
6. The administration device of paragraph 1, wherein the array of needles comprises about 2 to about 1,000 needles.
7. The administration device of paragraph 1, wherein the array of needles comprises about 5 to about 1,000 needles.
8. The administration device of paragraph 1, wherein the fluid agents comprise at least one indicator particle.
9. The administration device of paragraph 8, wherein the indicator particle is selected from the group consisting of a metallic particle, a fluorescent dye, a quantum dot, a quantum barcode, a radiographic contrast agent, a magnetic resonance imaging contrast agent, a positive control, and a negative control.
10. The administration device of paragraph 8, wherein the indicator particle comprises a dye.
11. The administration device of paragraph 10, wherein the dye is fluorescent.
12. The administration device of paragraph 1, wherein the tissue is from an animal.
13. The administration device of paragraph 1, wherein the tissue is from a human.
14. The administration device of paragraph 1, wherein the fluid agents comprise an anti-cancer agent, an anti-inflammatory agent, an anti-infective agent, a regenerative agent, a relaxing agent, an apoptosis-inhibiting agent, an apoptosis-inducing agent, an anti-coagulatory agent, a dermatological agent, a growth-stimulating agent, a vasodilating agent, a vasorestricting agent, an analgesic agent, an anti-allergic agent, or a combination thereof.
15. The administration device of paragraph 1, wherein the fluid agents comprise an anti-cancer agent.
16. The administration device of paragraph 1, wherein the tissue is, or is suspected of being, cancerous, inflamed, infected, atrophied, numb, in seizure, or coagulated.
17. The administration device of paragraph 1, wherein the tissue is, or is suspected of being, cancerous.
18. The administration device of paragraph 1, wherein the tissue is a tumor.
19. The administration device of paragraph 1, wherein the tumor is selected from a benign tumor and a malignant tumor.
20. The administration device of paragraph 1, wherein said hydrogel comprises a binary mixture of at least one polymer with two different molecular weight ranges.
21. The administration device of paragraph 1, wherein said hydrogel has a tensile strength of at least 20 gf/cm² in the dry state.
22. The administration device of paragraph 1, wherein said hydrogel has a tensile strength of 20-120 gf/cm² in the dry state.
23. The administration device of paragraph 1, wherein said hydrogel has a tensile strength of at least 5 gf/cm² in the hydrate state.
24. The administration device of paragraph 1, wherein said hydrogel has a tensile strength of 5-15 gf/cm² in the hydrate state.
25. The administration device of paragraph 1, wherein said hydrogel comprises collagen.
26. The administration device of paragraph 1, wherein said hydrogel comprises polyethylene glycol (PEG).
27. The administration device of paragraph 26, wherein said PEG has a molecular weight of at least 500.
28. The administration device of paragraph 27, wherein said PEG has a molecular weight of 4,000-8,000.
29. The administration device of paragraph 27, wherein said PEG has a molecular weight of 8,000-45,000.
30. The administration device of paragraph 1, wherein said hydrogel comprises poly(lactic-co-glycolic acid).
31. The administration device of paragraph 1, wherein said hydrogel comprise polycaprolactone.
32. The administration device of paragraph 20, wherein said at least one polymer comprises PEG.
33. The administration device of paragraph 20, wherein the two different molecular ranges comprise 500-2,000 and 4,000-8,000.
34. The administration device of paragraph 20, wherein the two different molecular ranges comprise 2,000-4,000 and 8,000-20,000.
35. The administration device of paragraph 20, wherein the two different molecular ranges comprise 2,000-8,000 and 10,000-45,000.
36. The administration device of paragraph 20, wherein the two different molecular ranges comprise 4,000-8000 and 10,000-45,000.
37. The administration device of paragraph 1, wherein the fluid agents and the hydrogel are delivered along parallel axes within the tissue.
38. The administration device of paragraph 1, wherein the diffusion rates of the fluid agents are controlled by the pore size of the hydrogel.
39. The administration device of paragraph 38, wherein the pore size is a range between about 50 nm and 500 µm.
40. The administration device of paragraph 1, further comprising one or more reservoirs each in fluid communication with a respective one of said needles.
41. The administration device of paragraph 1, further comprising two or more reservoirs each in fluid communication with a respective one of said needles.
42. The administration device of paragraph 1, further comprising five or more reservoirs each in fluid communication with a respective one of said needles.
43. The administration device of paragraph 41 or 42, wherein none of said reservoirs comprises the same fluid agent as the agent in any other reservoirs.
44. The administration device of paragraph 41 or 42, wherein at least two of said reservoirs comprise the same fluid agent.
45. The administration device of paragraph 44, wherein the concentrations of the same fluid agent in different reservoirs are different.
46. The administration device of paragraphs 40-42, wherein at least one of said reservoirs comprises at least two fluid agents.
47. A method of delivering a candidate agent into a tissue of an animal, the method comprising:
   a) depositing at least one pharmaceutical composition comprising a hydrogel into the tissue via an administration device comprising a needle; and
   b) dispensing the pharmaceutical composition into the tissue, wherein the pharmaceutical composition comprises one or more candidate agents and wherein the pharmaceutical composition disperses through the tissue to a lesser degree than does the same pharmaceutical composition lacking the hydrogel.
48. A method of delivering a candidate agent into a tissue of an animal, the method comprising:
   a) loading an administration device comprising a needle with one or more drug implants comprising one or more candidate agents; and
   b) inserting said one or more drug implants into the tissue using said administration device.
49. The method of paragraph 47 or 48, wherein the administration device comprises 2 or more needles.
50. The method of paragraph 47 or 48, wherein the administration device comprises 5 or more needles.
51. The method of paragraph 47 or 48, wherein the administration device comprises an actuator.
52. The method of paragraph 51, wherein the actuator comprises a plunger.
53. The method of paragraph 51, wherein the actuator comprises a pump.
54. The method of paragraph 47 or 48, wherein the administration device comprises 2 or more reservoirs each in communication with a respective one of said needle.
55. The method of paragraph 47 or 48, wherein the administration device comprises 5 or more reservoirs each in communication with a respective one of said needle.
56. The method of paragraph 47 or 48, wherein the administration device comprises 10 or more reservoirs each in communication with a respective one of said needle.
57. The method of paragraphs 54-56, wherein none of said reservoirs comprises the same candidate agent as the agent in any other reservoirs.
58. The method of paragraphs 54-56, wherein at least two of said reservoirs comprise a same candidate agent.
59. The method of paragraph 58, wherein the concentrations of the same fluid agent in different reservoirs are different.
60. The method of paragraphs 54-56, wherein at least one of said reservoirs comprises two or more candidate agents.
61. The method of paragraph 47 or 48, comprising 2 or more candidate agents.
62. The method of paragraph 47 or 48, comprising 5 or more candidate agents.
63. The method of paragraph 47 or 48, wherein the candidate agent is dispensed in an approximately column-shaped region coaxial with respect to the axis of delivery.
64. The method of paragraph 61 or 62, wherein said candidate agents are dispensed along parallel axes within the tissue.
65. The method of paragraph 47 or 48, wherein the candidate agent is delivered at or below systematically detectable concentration.
66. The method of paragraph 47 or 48, wherein the candidate agent comprises at least one indicator particle.
67. The method of paragraph 66, wherein the indicator particle is selected from the group consisting of a metallic particle, a fluorescent dye, a quantum dot, a quantum barcode, a radiographic contrast agent, a magnetic resonance imaging contrast agent, a positive control, and a negative control.
68. The method of paragraph 67, wherein the indicator particle is a fluorescent dye.
69. The method of paragraph 68, further comprising the step of imaging the fluorescent dye within the tissue, thereby monitoring the disbursement of the candidate agent.
70. The method of paragraph 47 or 48, wherein the animal is a human.
71. The method of paragraph 47 or 48, wherein the animal is a mouse, rat, or dog.
72. The method of paragraph 47 or 48, wherein said one ore more candidate agents comprise an anti-cancer agent, an anti-inflammatory agent, an anti-infective agent, a regenerative agent, a relaxing agent, an apoptosis-inhibiting agent, an apoptosis-inducing agent, an anti-coagulatory agent, a dermatological agent, a growth-stimulating agent, a vasodilating agent, a vasorestricting agent, a gene modulating agent, an analgesic agent, an anti-allergic agent, an RNAi molecule, or a combination thereof.
73. The method of paragraph 47 or 48, wherein said one or more candidate agents comprise an anti-cancer agent.
74. The method of paragraph 47 or 48, wherein the tissue is, or is suspected of being, cancerous.
75. The method of paragraph 47 or 48, wherein the tissue is a tumor.
76. The method of paragraph 75, wherein the tumor is selected from a benign tumor and a malignant tumor.
77. The method of paragraph 47, wherein the hydrogel comprises collagen.
78. The method of paragraph 47, wherein the hydrogel comprises polyethylene glycol (PEG).
79. The method of paragraph 47, wherein the hydrogel comprises poly(lactic-co-glycolic acid).
80. The method of paragraph 47, wherein the hydrogel comprises polycaprolactone.
81. The method of paragraph 47, wherein said pharmaceutical composition further comprises a dye.
82. The method of paragraph 81, wherein the dye allows for the detection of activation and deactivation of a biological function.
83. The method of paragraph 82, wherein the biological function is a pathway, an activity of an enzyme, an expression of a gene, drug sensitivity, drug resistance, transcription of the gene, translation of an RNA molecule associated with the gene, or peptide synthesis.
84. The method of paragraph 82, wherein the biological function is associated with cancer, degenerative disease, inflammation, metabolism, apoptosis, or immune response.
85. The method of paragraph 82, wherein the biological function is associated with cancer.
86. The method of paragraph 82, wherein the biological function is an apoptotic pathway, and said dye reports apoptosis.
87. The method of paragraph 82, wherein said detection is performed by measuring fluorescence.
88. The method of paragraph 82, further comprising the step of imaging the dye, thereby detecting the activation or deactivation of the biological function.
89. The method of paragraph 88, further comprising the step of predicting a potential therapeutic value of the therapeutic agents based on the results of the imaging.
90. The method of paragraph 88, further comprising the step of quantifying the activation or deactivation of the biological function.
91. The method of paragraph 48, wherein each of said one or more drug implants comprises an inert biodegradable binder that permits sustained or timed release of a drug within the tissue.
92. The method of paragraph 48, wherein the drug implants comprise at least one candidate agent in solid form.
93. The method of paragraph 48, wherein the candidate agent is released over a period of time between one minute and six weeks.
94. The method of paragraph 48, wherein each of the one or more drug implants has a diameter of less than about 0.1, 0.2, 0.3, 0.4, 0.5, 0.6, 0.7, 0.8, 0.9, or 1 mm.
95. The method of paragraph 48, wherein each of the needles is configured to receive one or more of said drug implants.
96. The method of paragraph 48, wherein each of the needles is configured to receive two or more of said drug implants.
97. The method of paragraph 48, wherein each of the needles is configured to receive five or more of said drug implants.
98. The method of paragraph 48, comprising two or more drug implants.
99. The method of paragraph 48, comprising five or more drug implants.
100. The method of paragraph 47 or 48, further comprising evaluating the effect of said one or more candidate agents on the tissue.
101. The method of paragraph 100, wherein the evaluation comprises excising at least one portion of the tissue after introducing said one or more candidate agents.
102. The method of paragraph 100, wherein the evaluation comprises analysis of at least one pre-excised portion of the tissue after introducing said one or more candidate agents.
103. The method of paragraph 101 or 102, wherein said excising or pre-excising is performed between one day and six weeks after introducing said one or more candidate agents.
104. The method of paragraph 100, wherein the evaluation comprises imaging the tissue.
105. The method of paragraph 104, wherein said imaging comprises radiographic imaging, magnetic resonance imaging, positron emission tomogoraphy, and biophotonic imaging.
106. The method of paragraph 104, wherein said imaging occurs before, during, or after introduction of said one or more candidate agents.
107. The method of paragraph 100, wherein said evaluating comprises detecting an altered physiological state.
108. The method of paragraph 100, further comprising one of (i) selecting at least one of said agents based on said evaluation; (ii) deselecting at least one of said agents based on said evaluation; and (iii) prioritizing at least two of said agents based on said evaluating.
109. A method of evaluating a plurality of candidate agents using a reporter tissue, comprising the steps of:
   a. producing a reporter cell that generates a detectable signal upon modulation of a pathway;
   b. constructing a reporter tissue from said reporter cell; and
   c. assessing the effects of said candidate agents on said reporter cell within said reporter tissue by delivering said candidate agents to regions within said reporter tissue with a needle array device, and detecting said detectable signal within said reporter cell.
110. The method of paragraph 109, wherein said needle array device comprises about 2 to about 100 needles.
111. The method of paragraph 109, wherein said needle array device comprises 5 to about 1000 needles.
112. The method of paragraph 109, wherein said reporter cell is a cancer cell.
113. The method of paragraph 109, wherein said reporter tissue comprises a xenografted tumor.
114. The method of paragraph 109, wherein said modulation comprises activation.
115. The method of paragraph 109, wherein said modulation comprises inhibition.
116. The method of paragraph 109, wherein said candidate agents comprise an agent that is selected from the group consisting of a gene therapy agent, a chemotherapy agent, a small molecule, an antibody, a protein, a small interfering RNA, an antisense RNA, a ribozyme, a detectable label, a therapeutic protein, a therapeutic cell, a cytokine, an antibody-drug conjugate, an antibody, a polypeptide, a peptidomimetic, and a microRNA.
117. The method of paragraph 109, wherein said candidate agents comprises an anti-cancer agent.
118. The method of paragraph 109, wherein each of said candidate agents is within a hydrogel.
119. The method of paragraph 109, wherein said reporter cell comprises a gene trap exhibiting luciferase-based activity.
120. The method of paragraph 109, wherein said reporter cell comprises a reporter gene associated with an endogenous promoter.
121. The method of paragraph 109, wherein the reporter comprises luciferase- and GFP-based reporters, the pathways comprises AKT pathway, and the endogenous promoter comprises phosphatase and tensin homolog gene.
122. The method of paragraph 109, wherein said detectable signal comprises fluorescence.
123. The method of paragraph 122, wherein said fluorescence is generated by a fluorescent protein which is selected from the group consisting of GFP, BFP, CFP, YFP, EGFP, EYFP, Venus, Citrine, phiYFP, copGFP CGFP, ECFP, Cerulean, CyPet, T-Sapphire, Emerald, YPet, AcGFP1, AmCyan, AsRed2, dsRed, dsRed2, dsRed-Express, EBFP, HcRed, ZsGreen, ZsYellow, J-Red, TurboGFP, Kusabira Orange, Midoriishi Cyan, mOrange, DsRed-monomer, mStrawberry, mRFP1, tdTomato, mCherry, mPlum, and mRaspberry.
124. The method of paragraph 123, wherein said fluorescent protein is GFP.
125. The method of paragraph 109, wherein said detectable signal comprises the detectable product from the reaction of an enzyme.
126. The method of paragraph 125, wherein said enzyme is selected from the group consisting of: peroxidase, alkaline phosphatase, galactosidase, luciferase, and lactamase.
127. The method of paragraph 125, wherein said enzyme is luciferase.
128. The method of paragraph 109, wherein the assessing comprises imaging said reporter tissue following delivery to observe said detectable signal.
129. The method of paragraph 128, further comprising the step of excising the reporter tissue prior to the imaging.
130. The method of paragraph 129, further comprising the step of sectioning the excised reporter tissue prior to imaging.
131. The method of paragraph 109, further comprising the step of producing a reporter cell line from the reporter cell line of step (a) that generates a second detectable signal upon modulation of said signaling pathway.
132. The method of paragraph 131, wherein the first detectable signal indicates activation of the signaling pathway, and the second detectable signal indicates inhibition of the signaling pathway.
133. The method of paragraph 131, wherein the first detectable signal indicates inhibition of the signaling pathway, and the second detectable signal indicates activation of the signaling pathway.
134. The method of paragraph 132 or 133, further comprising the step of isolating cells that previously generated said second detectable signal in response to said candidate agents, but now generates said first detectable signal in response to said candidate agents.
135. The method of paragraph 109, further comprising the step of isolating cells that previously generated said detectable signal in response to said candidate agents, but no longer generate said detectable signal.
136. The method of paragraph 134 or 135, further comprising the step of sequencing at least one nucleic acid of the isolated cells to determine the genetic basis of the change in response to said candidate agents.
137. The method of paragraph 136, wherein said sequencing comprises high-throughput sequencing.
138. The method of paragraph 136, wherein said nucleic acid comprises a genomic DNA.
139. The method of paragraph 136, wherein said nucleic acid comprises a messenger RNA.
140. A method for determining resistance to a cancer treatment, comprising the steps of:
   a. administering a plurality of cancer treatments to a reporter tissue that generates a detectable signal upon modulation of a pathway;
   b. isolating a population of cells within said reporter tissue that exhibit said detectable signal in response to one or more of said plurality of cancer treatments; and
   c. sequencing at least one nucleic acid from the population of cells, thereby determining resistance to the selected cancer treatment.
141. A method for determining resistance to a cancer treatment, comprising the steps of:
   a. administering a plurality of cancer treatments to a reporter tissue that generates a detectable signal upon modulation of a pathway;
   b. isolating a population of cells within said reporter tissue that exhibit said detectable signal in response to one or more of said plurality of cancer treatments; and
   c. assessing the number of copies of genes, thereby determining resistance to the selected cancer treatment.
142. The method of paragraph 140 or 141, wherein said administering a plurality of cancer treatments comprises injecting with a needle array.
143. The method of paragraph 140 or 141, wherein said modulation comprises activation.
144. The method of paragraph 140 or 141, wherein said modulation comprises inhibition.
145. The method of paragraph 140 or 141, wherein said isolating comprises cell sorting.
146. The method of paragraph 140, wherein said sequencing comprises high-throughput sequencing.
147. The method of paragraph 140, wherein said nucleic acid comprises a genomic DNA.
148. The method of paragraph 140, wherein said nucleic acid comprises a messenger RNA.
149. A method of local delivery of a therapy for the treatment of a muscle disease or disorder of a subject, comprising administrating of a therapeutic agent in vivo simultaneously to a tissue of said subject using a needle array device comprising a plurality of needles.
150. A method of delivering a selected gene to a muscle tissue of a subject in vivo, said method comprising: (a) providing a virus which comprises a viral vector, said viral vector comprising said selected gene operably linked to control elements capable of directing the in vivo transcription and translation of said selected gene; and (b) introducing said virus into said muscle tissue in vivo using a needle array device comprising a plurality of needles.
151. The method of paragraph 149, wherein the therapy comprises a gene replacement therapy, a microRNA therapy, a RNAi therapy, an antibody therapy, and an aptamer therapy.
152. The method of paragraph 149, wherein the therapy is a gene replacement therapy.
153. The method of paragraph 149, wherein the muscle disease or disorder is selected from the group consisting of: Duchenne muscular dystrophy (DMD), Becker muscular dystrophy, Limb Girdle muscular dystrophy, facioscapulhumeral muscular dystrophy, congenital muscular dystrophy, oculopharyngeal muscular dystrophy, distal muscular dystrophy, Emery-Dreifuss muscular dystrophy, muscle atrophy, X-linked spinal-bulbar muscular atrophy (SBMA), cachexia, malnutrition, tuberculosis, leprosy, diabetes, renal disease, chronic obstructive pulmonary disease (COPD), cancer, end stage renal failure, burns, diabetes, congestive heart failure, sarcopenia, emphysema, osteomalacia, or cardiomyopathy.
154. The method of paragraph 149, wherein the muscle disease or disorder is a muscle wasting disease.
155. The method of paragraph 149, wherein the muscle disease or disorder is Duchenne Muscular Dystrophy.
156. The method of paragraph 149, wherein the muscle disease or disorder is sarcopenia.
157. The method of paragraph 149 or 150, wherein the needles are configured for delivery by intramuscular injection.
158. The method of paragraph 152 or 150, wherein the gene or gene replacement therapeutic agent is delivered into greater than 1%, 2% 5%, 10%, 20%, 30%, 40%, 50%, 60%, 70%, 80%, or 90% of large muscle fibers of a single limb.
159. The method of paragraph 149, wherein the tissue is brain, liver, lung, kidney, prostate, ovary, spleen, lymph, thyroid, pancreas, heart, muscle, intestine, larynx, esophagus or stomach tissue.
160. The method of paragraph 149, wherein the tissue is muscle tissue.
161. The method of paragraph 150 or 160, wherein the muscle tissue is skeletal muscle, smooth muscle or cardiac muscle.
162. The method of paragraph 149 or 150, wherein the subject is a nonhuman primate, mouse, dog, rat, rabbit, pig, sheep, horse, bovine, goat, gerbil, hamster, guinea pig or human.
163. The method of paragraph 149 or 150, wherein the subject is a dog.
164. The method of paragraph 149 or 150, wherein the subject is a human.
165. The method of paragraph 150 or 152, wherein the gene comprises dystrophin.
166. The method of paragraph 150 or 152, wherein the gene comprises a dystrophin minigene encoding a protein or the complement of the dystrophin minigene, wherein the protein comprises:
   (a) a N-terminal domain of a dystrophin protein or modified N-terminal domain of the dystrophin protein;
   (b) five rod repeats of the dystrophin protein;
   (c) an HI domain of a dystrophin gene and an H4 domain of the dystrophin protein; and
   (d) a cysteine-rich domain of the dystrophin protein, wherein said nucleotide sequence has fewer than 5,000 nucleotides.
167. The method of paragraph 150 or 152, wherein the gene encodes a myostatin inhibitor.
168. The method of paragraph 167, wherein the myostatin inhibitor is myostatin propeptide, follistatin, other follistatin-like proteins, FLRG or GASP-1.
169. The method of paragraph 150 or 152, wherein the gene is operably linked to one or more control elements.
170. The method of paragraph 169, wherein each of said one or more control elements is selected from the group consisting of: AAV inverted terminal repeat, retrovirus long terminal repeat, cytomegalovirus (CMV) immediate early promoter, CMV enhancer, β-actin promoter, α-actin promoter, myosin promoter, muscle-specific creatine kinase (MCK) promoter, and MCK enhancer.
171. The method of paragraph 170, wherein at least one control element is MCK promoter.
172. The method of paragraph 152, wherein the gene replacement therapeutic agent comprises an adenovirus, a herpes simplex virus (HSV), a baculovirus, an adeno-associated virus (AAV), a recombinant adeno-associated virus (rAAV), a retrovirus, or a lentivirus.
173. The method of paragraph 172, wherein the gene replacement therapeutic agent comprises AAV.
174. The method of paragraph 150, wherein said viral vector comprises a retrovirus.
175. The method of paragraph 150, wherein said viral vector comprises a lentivirus.
176. The method of paragraph 150, wherein said viral vector comprises a recombinant adeno-associated virus.
177. The method of paragraph 150 or 173, wherein the AAV is selected from the group consisting of: AAV-1, AAV-2, AAV-3, AAV-4, AAV-5, AAV-6, AAV-7, AAV-8, AAV-9, AAV-10 and AAV-11.
178. The method of paragraph 177, wherein the AAV is AAV-6.
179. The method of paragraph 152, wherein the gene replacement therapeutic agent comprises a stem cell.
180. The method of paragraph 179, wherein the stem cell is an embryonic stem cell, adult stem cell, or induced pluripotent stem cell.

## Claims

1. A non-therapeutic method of evaluating an agent using a reporter tissue, comprising:
(a) constructing a reporter tissue from a reporter cell, wherein said reporter cell generates one or more detectable signals upon modulation of a pathway, wherein said reporter tissue is pre-administered with an agent by a device comprising two or more needles; and
(b) assessing an effect of said agent on said reporter cell within said reporter tissue.

2. The method of claim 1, wherein said device comprises from about 6 to about 15 needles.

3. The method of claim 1, wherein said modulation is selected from the group consisting of: activation, and inhibition, or any combination thereof.

4. The method of claim 1, wherein said agent is selected from said group consisting of: a gene therapy agent, a chemotherapy agent, a small molecule, an antibody, a protein, a small interfering RNA, an antisense RNA, a ribozyme, a detectable label, a therapeutic protein, a therapeutic cell, a cytokine, an antibody-drug conjugate, an antibody, a polypeptide, a peptidomimetic, a hydrogel-comprising agent, and a microRNA, or any combination thereof.

5. The method of claim 1, wherein said agent comprise an anti-cancer agent.

6. The method of claim 1, wherein said agent comprises a hydrogel.

7. The method of claim 1, wherein said reporter cell comprises a cancer cell.

8. The method of claim 1, wherein said reporter tissue comprises a tumor.

9. The method of claim 8, wherein said tumor comprises a xenografted tumor.

10. The method of claim 1, wherein said assessing comprises detecting said detectable signal within said reporter cell.

11. The method of claim 1, wherein said assessing comprises imaging said reporter tissue.

12. The method of claim 1, wherein said assessing comprises isolating said reporter cell.

13. The method of claim 12, wherein said method further comprises sequencing at least one nucleic acid of an isolated cell.

14. The method of claim 13, wherein said sequencing determines a genetic basis of a change in response to said agent.
